# EUROPEAN PATENT APPLICATION

(11) **EP 3 875 468 A1**
(43) Date of publication of application: **08.09.2021**
(21) Application number: 19878882.0
(22) Date of filing: 28.10.2019
(51) Int. Cl.: C07K 14/00

(54) **METHOD FOR PRODUCING FERRITIN CONTAINING ORGANIC COMPOUND ENCLOSED THEREIN**

(30) Priority: 29.10.2018 JP 2018203246
(71) Applicant: AJINOMOTO CO., INC., Chuo-ku Tokyo 104-8315 (JP)
(72) Inventor: INOUE, Ippei, Kawasaki-shi, Kanagawa 210-8681 (JP); NAKAHARA, Yuichi, Kawasaki-shi, Kanagawa 210-8681 (JP)
(74) Representative: Mewburn Ellis LLP
(86) International application number: PCT/JP2019/042116
(87) International publication number: WO 2020/090708

(57) **Abstract**

The present invention provides an alternative method for producing an organic compound-encapsulating ferritin. More specifically, the present invention provides a method for producing the organic compound-encapsulating ferritin. The method comprises:
(1) mixing an organic compound with ferritin in a buffer to obtain a mixture of the organic compound and ferritin; and
(2) incubating the mixture in the buffer,
wherein the buffer has a pH of 3 or more and less than 13, and
the buffer has a pH of 3 or more and less than 7 when the organic compound has a pKa less than 7, and has a pH of 6 or more and less than 13 when the organic compound has a pKa of 7 or more.

### [Sequence Listing]

SEQUENCE LISTING

<110>  Ajinomoto Co., Inc.

<120>  Method of producing an organic compound-encapsulating ferritin

<130>  AM-R011828

<150>  JP2018-203246
<151>  2018-10-29

<160>  235

<170>  PatentIn version 3.5

<210>  1
<211>  183
<212>  PRT
<213>  Homo sapiens

<400>  1

Met Thr Thr Ala Ser Thr Ser Gln Val Arg Gln Asn Tyr His Gln Asp
1               5                   10                  15

Ser Glu Ala Ala Ile Asn Arg Gln Ile Asn Leu Glu Leu Tyr Ala Ser
            20                  25                  30

Tyr Val Tyr Leu Ser Met Ser Tyr Tyr Phe Asp Arg Asp Asp Val Ala
        35                  40                  45

Leu Lys Asn Phe Ala Lys Tyr Phe Leu His Gln Ser His Glu Glu Arg
    50                  55                  60

Glu His Ala Glu Lys Leu Met Lys Leu Gln Asn Gln Arg Gly Gly Arg
65                  70                  75                  80

Ile Phe Leu Gln Asp Ile Lys Lys Pro Asp Cys Asp Asp Trp Glu Ser
                85                  90                  95

Gly Leu Asn Ala Met Glu Cys Ala Leu His Leu Glu Lys Asn Val Asn
            100                 105                 110

Gln Ser Leu Leu Glu Leu His Lys Leu Ala Thr Asp Lys Asn Asp Pro
        115                 120                 125

His Leu Cys Asp Phe Ile Glu Thr His Tyr Leu Asn Glu Gln Val Lys
    130                 135                 140

Ala Ile Lys Glu Leu Gly Asp His Val Thr Asn Leu Arg Lys Met Gly
145                 150                 155                 160

Ala Pro Glu Ser Gly Leu Ala Glu Tyr Leu Phe Asp Lys His Thr Leu

165                 170                 175

Gly Asp Ser Asp Asn Glu Ser
            180

<210>  2
<211>  175
<212>  PRT
<213>  Homo sapiens

<400>  2

<210>  3
<211>  36
<212>  DNA
<213>  Artificial Sequence

<220>
<223>  Primer

<400>  3
gaaggagata tacatatgac gaccgcgtcc acctcg                                36


<210>  4
<211>  36
<212>  DNA
<213>  Artificial Sequence

<220>
<223>  Primer

<400>  4
ctcgaattcg gatccttagc tttcattatc actgtc                                36


<210>  5
<211>  33
<212>  DNA
<213>  Artificial Sequence

<220>
<223>  Primer

<400>  5
tttcatatgt atatctcctt cttaaagtta aac                                   33


<210>  6
<211>  26
<212>  DNA
<213>  Artificial Sequence

<220>
<223>  Primer

<400>  6
tttggatccg aattcgagct ccgtcg                                           26


<210>  7
<211>  6
<212>  PRT
<213>  Artificial Sequence

<220>
<223>  Titanium-binding peptide

<400>  7

Arg Lys Leu Pro Asp Ala
1               5


<210>  8
<211>  36
<212>  DNA
<213>  Artificial Sequence

<220>

<223>  Primer

<400>  8
gaaggagata tacatatgac gaccgcgtcc acctcg                                36


<210>  9
<211>  36
<212>  DNA
<213>  Artificial Sequence

<220>
<223>  Primer

<400>  9
ctcgaattcg gatccttagc tttcattatc actgtc                                36


<210>  10
<211>  33
<212>  DNA
<213>  Artificial Sequence

<220>
<223>  Primer

<400>  10
tttcatatgt atatctcctt cttaaagtta aac                                   33


<210>  11
<211>  26
<212>  DNA
<213>  Artificial Sequence

<220>
<223>  Primer

<400>  11
tttggatccg aattcgagct ccgtcg                                           26


<210>  12
<211>  26
<212>  DNA
<213>  Artificial Sequence

<220>
<223>  Primer

<400>  12
tttggatccg aattcgagct ccgtcg                                           26


<210>  13
<211>  31
<212>  DNA
<213>  Artificial Sequence

<220>
<223>  Primer

<400>  13

tttggatcct taacagcttt cattatcact g                                    31

<210>  14
<211>  6
<212>  PRT
<213>  Artificial Sequence

<220>
<223>  Cancer-recognizing peptide

<400>  14

Met Ala Arg Ser Gly Leu
1               5

<210>  15
<211>  42
<212>  DNA
<213>  Artificial Sequence

<220>
<223>  Primer

<400>  15
tttcatatgg cacgtagtgg tttaacgacc gcgtccacct cg                        42

<210>  16
<211>  33
<212>  DNA
<213>  Artificial Sequence

<220>
<223>  Primer

<400>  16
tttcatatgt atatctcctt cttaaagtta aac                                  33

<210>  17
<211>  6
<212>  PRT
<213>  Artificial Sequence

<220>
<223>  RNA-recognizing peptide

<400>  17

Thr Arg Gln Ala Arg Arg
1               5

<210>  18
<211>  46
<212>  DNA
<213>  Artificial Sequence

<220>
<223>  Primer

<400>  18
tttggatcct tatctgcgtg cttgacgtgt gctttcatta tcactg                    46


<210>  19
<211>  3
<212>  PRT
<213>  Artificial Sequence

<220>
<223>  Peptide capable of binding to a biological organic molecule

<400>  19

Arg Gly Asp
1


<210>  20
<211>  11
<212>  PRT
<213>  Artificial Sequence

<220>
<223>  Peptide capable of binding to a biological organic molecule

<400>  20

Ala Cys Asp Cys Arg Gly Asp Cys Phe Cys Gly
1               5                   10


<210>  21
<211>  9
<212>  PRT
<213>  Artificial Sequence

<220>
<223>  Peptide capable of binding to a biological organic molecule

<400>  21

Cys Asp Cys Arg Gly Asp Cys Phe Cys
1               5


<210>  22
<211>  5
<212>  PRT
<213>  Artificial Sequence

<220>
<223>  Peptide capable of binding to a biological organic molecule

<400>  22

Gly Arg Gly Asp Ser
1               5


<210>  23
<211>  193
<212>  PRT

<213>  Artificial Sequence

<220>
<223>  Fusion protein of human ferritin H chain and tumor-binding
       peptide inserted into a flexible linker portion between 2nd and
       3rd alpha-helices of human ferritin H chain from N-terminus, and
       a cysteine residue at its C-terminus

<400>  23

Met Thr Thr Ala Ser Thr Ser Gln Val Arg Gln Asn Tyr His Gln Asp
1               5                   10                  15

Ser Glu Ala Ala Ile Asn Arg Gln Ile Asn Leu Glu Leu Tyr Ala Ser
            20                  25                  30

Tyr Val Tyr Leu Ser Met Ser Tyr Tyr Phe Asp Arg Asp Asp Val Ala
        35                  40                  45

Leu Lys Asn Phe Ala Lys Tyr Phe Leu His Gln Ser His Glu Glu Arg
    50                  55                  60

Glu His Ala Glu Lys Leu Met Lys Leu Gln Asn Gln Arg Gly Gly Arg
65                  70                  75                  80

Ile Phe Leu Gln Asp Ile Lys Ala Ser Asp Arg Gly Asp Phe Ser Gly
                85                  90                  95

Lys Pro Asp Cys Asp Asp Trp Glu Ser Gly Leu Asn Ala Met Glu Cys
            100                 105                 110

Ala Leu His Leu Glu Lys Asn Val Asn Gln Ser Leu Leu Glu Leu His
        115                 120                 125

Lys Leu Ala Thr Asp Lys Asn Asp Pro His Leu Cys Asp Phe Ile Glu
    130                 135                 140

Thr His Tyr Leu Asn Glu Gln Val Lys Ala Ile Lys Glu Leu Gly Asp
145                 150                 155                 160

His Val Thr Asn Leu Arg Lys Met Gly Ala Pro Glu Ser Gly Leu Ala
                165                 170                 175

Glu Tyr Leu Phe Asp Lys His Thr Leu Gly Asp Ser Asp Asn Glu Ser
            180                 185                 190

Cys

<210>  24

<211>  5
<212>  PRT
<213>  Artificial Sequence

<220>
<223>  Peptide capable of binding to a biological organic molecule

<400>  24

Glu Ile Leu Asp Val
1               5


<210>  25
<211>  4
<212>  PRT
<213>  Artificial Sequence

<220>
<223>  Peptide capable of binding to a biological organic molecule

<400>  25

Arg Glu Asp Val
1


<210>  26
<211>  12
<212>  PRT
<213>  Artificial Sequence

<220>
<223>  Peptide capable of binding to a biological organic molecule

<400>  26

Ser Tyr Ser Met Glu His Phe Arg Trp Gly Lys Pro
1               5                   10


<210>  27
<211>  7
<212>  PRT
<213>  Artificial Sequence

<220>
<223>  Peptide capable of binding to a biological organic molecule

<400>  27

Val Asn Thr Ala Asn Ser Thr
1               5


<210>  28
<211>  12
<212>  PRT
<213>  Artificial Sequence

<220>
<223>  Peptide capable of binding to a biological organic molecule

<400>  28

Asp His Leu Ala Ser Leu Trp Trp Gly Thr Glu Leu
1               5                   10


<210>  29
<211>  12
<212>  PRT
<213>  Artificial Sequence

<220>
<223>  Peptide capable of binding to a biological organic molecule

<400>  29

Asn Tyr Ser Lys Pro Thr Asp Arg Gln Tyr His Phe
1               5                   10


<210>  30
<211>  12
<212>  PRT
<213>  Artificial Sequence

<220>
<223>  Peptide capable of binding to a biological organic molecule

<400>  30

Ile Pro Leu Pro Pro Pro Ser Arg Pro Phe Phe Lys
1               5                   10


<210>  31
<211>  12
<212>  PRT
<213>  Artificial Sequence

<220>
<223>  Peptide capable of binding to a biological organic molecule

<400>  31

Leu Met Asn Pro Asn Asn His Pro Arg Thr Pro Arg
1               5                   10


<210>  32
<211>  9
<212>  PRT
<213>  Artificial Sequence

<220>
<223>  Peptide capable of binding to a biological organic molecule

<400>  32

Cys His His Asn Leu Thr His Ala Cys
1               5


<210>  33

<211>  9
<212>  PRT
<213>  Artificial Sequence

<220>
<223>  Peptide capable of binding to a biological organic molecule

<400>  33

Cys Leu His His Tyr His Gly Ser Cys
1               5


<210>  34
<211>  9
<212>  PRT
<213>  Artificial Sequence

<220>
<223>  Peptide capable of binding to a biological organic molecule

<400>  34

Cys His His Ala Leu Thr His Ala Cys
1               5


<210>  35
<211>  12
<212>  PRT
<213>  Artificial Sequence

<220>
<223>  Peptide capable of binding to a biological organic molecule

<400>  35

Ser Pro Arg Pro Arg His Thr Leu Arg Leu Ser Leu
1               5                   10


<210>  36
<211>  12
<212>  PRT
<213>  Artificial Sequence

<220>
<223>  Peptide capable of binding to a biological organic molecule

<400>  36

Thr Met Gly Phe Thr Ala Pro Arg Phe Pro His Tyr
1               5                   10


<210>  37
<211>  16
<212>  PRT
<213>  Artificial Sequence

<220>
<223>  Peptide capable of binding to a biological organic molecule

<400>  37

Asn Gly Tyr Glu Ile Glu Trp Tyr Ser Trp Val Thr His Gly Met Tyr
1               5                   10                  15


<210>  38
<211>  12
<212>  PRT
<213>  Artificial Sequence

<220>
<223>  Peptide capable of binding to a biological organic molecule

<400>  38

Phe Arg Ser Phe Glu Ser Cys Leu Ala Lys Ser His
1               5                   10


<210>  39
<211>  12
<212>  PRT
<213>  Artificial Sequence

<220>
<223>  Peptide capable of binding to a biological organic molecule

<400>  39

Tyr His Trp Tyr Gly Tyr Thr Pro Gln Asn Val Ile
1               5                   10


<210>  40
<211>  12
<212>  PRT
<213>  Artificial Sequence

<220>
<223>  Peptide capable of binding to a biological organic molecule

<400>  40

Gln His Tyr Asn Ile Val Asn Thr Gln Ser Arg Val
1               5                   10


<210>  41
<211>  7
<212>  PRT
<213>  Artificial Sequence

<220>
<223>  Peptide capable of binding to a biological organic molecule

<400>  41

Gln Arg His Lys Pro Arg Glu
1               5


<210>  42

<211>  7
<212>  PRT
<213>  Artificial Sequence

<220>
<223>  Peptide capable of binding to a biological organic molecule

<400>  42

His Ser Gln Ala Ala Val Pro
1               5


<210>  43
<211>  7
<212>  PRT
<213>  Artificial Sequence

<220>
<223>  Peptide capable of binding to a biological organic molecule

<400>  43

Ala Gly Asn Trp Thr Pro Ile
1               5


<210>  44
<211>  7
<212>  PRT
<213>  Artificial Sequence

<220>
<223>  Peptide capable of binding to a biological organic molecule

<400>  44

Pro Leu Leu Gln Ala Thr Leu
1               5


<210>  45
<211>  7
<212>  PRT
<213>  Artificial Sequence

<220>
<223>  Peptide capable of binding to a biological organic molecule

<400>  45

Leu Ser Leu Ile Thr Arg Leu
1               5


<210>  46
<211>  6
<212>  PRT
<213>  Artificial Sequence

<220>
<223>  Peptide capable of binding to a biological organic molecule

<400>  46

Cys Arg Gly Asp Cys Leu
1               5



<210>  47
<211>  9
<212>  PRT
<213>  Artificial Sequence

<220>
<223>  Peptide capable of binding to a biological organic molecule

<400>  47

Cys Arg Arg Glu Thr Ala Trp Ala Cys
1               5



<210>  48
<211>  12
<212>  PRT
<213>  Artificial Sequence

<220>
<223>  Peptide capable of binding to a biological organic molecule

<400>  48

Arg Thr Asp Leu Asp Ser Leu Arg Thr Tyr Thr Leu
1               5                   10



<210>  49
<211>  10
<212>  PRT
<213>  Artificial Sequence

<220>
<223>  Peptide capable of binding to a biological organic molecule

<400>  49

Cys Thr Thr His Trp Gly Phe Thr Leu Cys
1               5                   10



<210>  50
<211>  7
<212>  PRT
<213>  Artificial Sequence

<220>
<223>  Peptide capable of binding to a biological organic molecule

<400>  50

Ala Pro Ser Pro Met Ile Trp
1               5



<210>  51

<211>  7
<212>  PRT
<213>  Artificial Sequence

<220>
<223>  Peptide capable of binding to a biological organic molecule

<400>  51

Leu Gln Asn Ala Pro Arg Ser
1               5


<210>  52
<211>  12
<212>  PRT
<213>  Artificial Sequence

<220>
<223>  Peptide capable of binding to a biological organic molecule

<400>  52

Ser Trp Thr Leu Tyr Thr Pro Ser Gly Gln Ser Lys
1               5                   10


<210>  53
<211>  12
<212>  PRT
<213>  Artificial Sequence

<220>
<223>  Peptide capable of binding to a biological organic molecule

<400>  53

Ser Trp Glu Leu Tyr Tyr Pro Leu Arg Ala Asn Leu
1               5                   10


<210>  54
<211>  12
<212>  PRT
<213>  Artificial Sequence

<220>
<223>  Peptide capable of binding to a biological organic molecule

<400>  54

Trp Gln Pro Asp Thr Ala His His Trp Ala Thr Leu
1               5                   10


<210>  55
<211>  9
<212>  PRT
<213>  Artificial Sequence

<220>
<223>  Peptide capable of binding to a biological organic molecule

<400>  55

Cys Ser Asp Ser Trp His Tyr Trp Cys
1               5


<210>  56
<211>  12
<212>  PRT
<213>  Artificial Sequence

<220>
<223>  Peptide capable of binding to a biological organic molecule

<400>  56

Trp His Trp Leu Pro Asn Leu Arg His Tyr Ala Ser
1               5                   10


<210>  57
<211>  12
<212>  PRT
<213>  Artificial Sequence

<220>
<223>  Peptide capable of binding to a biological organic molecule

<400>  57

Trp His Thr Glu Ile Leu Lys Ser Tyr Pro His Glu
1               5                   10


<210>  58
<211>  12
<212>  PRT
<213>  Artificial Sequence

<220>
<223>  Peptide capable of binding to a biological organic molecule

<400>  58

Leu Pro Ala Phe Phe Val Thr Asn Gln Thr Gln Asp
1               5                   10


<210>  59
<211>  12
<212>  PRT
<213>  Artificial Sequence

<220>
<223>  Peptide capable of binding to a biological organic molecule

<400>  59

Tyr Asn Thr Asn His Val Pro Leu Ser Pro Lys Tyr
1               5                   10


<210>  60

<211>  12
<212>  PRT
<213>  Artificial Sequence

<220>
<223>  Peptide capable of binding to a biological organic molecule

<400>  60

Tyr Ser Ala Tyr Pro Asp Ser Val Pro Met Met Ser
1               5                   10


<210>  61
<211>  12
<212>  PRT
<213>  Artificial Sequence

<220>
<223>  Peptide capable of binding to a biological organic molecule

<400>  61

Thr Asn Tyr Leu Phe Ser Pro Asn Gly Pro Ile Ala
1               5                   10


<210>  62
<211>  9
<212>  PRT
<213>  Artificial Sequence

<220>
<223>  Peptide capable of binding to a biological organic molecule

<400>  62

Cys Leu Ser Tyr Tyr Pro Ser Tyr Cys
1               5


<210>  63
<211>  14
<212>  PRT
<213>  Artificial Sequence

<220>
<223>  Peptide capable of binding to a biological organic molecule

<400>  63

Cys Val Gly Val Leu Pro Ser Gln Asp Ala Ile Gly Ile Cys
1               5                   10


<210>  64
<211>  14
<212>  PRT
<213>  Artificial Sequence

<220>
<223>  Peptide capable of binding to a biological organic molecule

<400>  64

Cys Glu Trp Lys Phe Asp Pro Gly Leu Gly Gln Ala Arg Cys
1               5                   10


<210>  65
<211>  14
<212>  PRT
<213>  Artificial Sequence

<220>
<223>  Peptide capable of binding to a biological organic molecule

<400>  65

Cys Asp Tyr Met Thr Asp Gly Arg Ala Ala Ser Lys Ile Cys
1               5                   10


<210>  66
<211>  6
<212>  PRT
<213>  Artificial Sequence

<220>
<223>  Peptide capable of binding to a biological organic molecule

<400>  66

Lys Cys Cys Tyr Ser Leu
1               5


<210>  67
<211>  6
<212>  PRT
<213>  Artificial Sequence

<220>
<223>  Peptide capable of binding to a biological organic molecule

<400>  67

Met Ala Arg Ala Lys Glu
1               5


<210>  68
<211>  6
<212>  PRT
<213>  Artificial Sequence

<220>
<223>  Peptide capable of binding to a biological organic molecule

<400>  68

Met Ser Arg Thr Met Ser
1               5


<210>  69

<211>  20
<212>  PRT
<213>  Artificial Sequence

<220>
<223>  Peptide capable of binding to a biological organic molecule

<400>  69

Trp Thr Gly Trp Cys Leu Asn Pro Glu Glu Ser Thr Trp Gly Phe Cys
1               5                   10                  15

Thr Gly Ser Phe
            20

<210>  70
<211>  12
<212>  PRT
<213>  Artificial Sequence

<220>
<223>  Peptide capable of binding to a biological organic molecule

<400>  70

Met Cys Gly Val Cys Leu Ser Ala Gln Arg Trp Thr
1               5                   10

<210>  71
<211>  12
<212>  PRT
<213>  Artificial Sequence

<220>
<223>  Peptide capable of binding to a biological organic molecule

<400>  71

Ser Gly Leu Trp Trp Leu Gly Val Asp Ile Leu Gly
1               5                   10

<210>  72
<211>  16
<212>  PRT
<213>  Artificial Sequence

<220>
<223>  Peptide capable of binding to a biological organic molecule

<400>  72

Asn Pro Gly Thr Cys Lys Asp Lys Trp Ile Glu Cys Leu Leu Asn Gly
1               5                   10                  15

<210>  73
<211>  16
<212>  PRT
<213>  Artificial Sequence

<220>
<223>  Peptide capable of binding to a biological organic molecule

<400>  73

Ala Asn Thr Pro Cys Gly Pro Tyr Thr His Asp Cys Pro Val Lys Arg
1               5                   10                  15


<210>  74
<211>  15
<212>  PRT
<213>  Artificial Sequence

<220>
<223>  Peptide capable of binding to a biological organic molecule

<400>  74

Ile Val Trp His Arg Trp Tyr Ala Trp Ser Pro Ala Ser Arg Ile
1               5                   10                  15


<210>  75
<211>  10
<212>  PRT
<213>  Artificial Sequence

<220>
<223>  Peptide capable of binding to a biological organic molecule

<400>  75

Cys Gly Leu Ile Ile Gln Lys Asn Glu Cys
1               5                   10


<210>  76
<211>  7
<212>  PRT
<213>  Artificial Sequence

<220>
<223>  Peptide capable of binding to a biological organic molecule

<400>  76

Met Gln Leu Pro Leu Ala Thr
1               5


<210>  77
<211>  15
<212>  PRT
<213>  Artificial Sequence

<220>
<223>  Peptide capable of binding to a biological organic molecule

<400>  77

Cys Arg Ala Leu Leu Arg Gly Ala Pro Phe His Leu Ala Glu Cys

1               5                   10                  15

<210>  78
<211>  7
<212>  PRT
<213>  Artificial Sequence

<220>
<223>  Peptide capable of binding to a biological organic molecule

<400>  78

Ile Glu Leu Leu Gln Ala Arg
1               5

<210>  79
<211>  7
<212>  PRT
<213>  Artificial Sequence

<220>
<223>  Peptide capable of binding to a biological organic molecule

<400>  79

Thr Leu Thr Tyr Thr Trp Ser
1               5

<210>  80
<211>  13
<212>  PRT
<213>  Artificial Sequence

<220>
<223>  Peptide capable of binding to a biological organic molecule

<400>  80

Cys Val Ala Tyr Cys Ile Glu His His Cys Trp Thr Cys
1               5                   10

<210>  81
<211>  7
<212>  PRT
<213>  Artificial Sequence

<220>
<223>  Peptide capable of binding to a biological organic molecule

<400>  81

Thr His Glu Asn Trp Pro Ala
1               5

<210>  82
<211>  12
<212>  PRT
<213>  Artificial Sequence

<220>
<223>  Peptide capable of binding to a biological organic molecule

<400>  82

Trp His Pro Trp Ser Tyr Leu Trp Thr Gln Gln Ala
1               5                   10


<210>  83
<211>  7
<212>  PRT
<213>  Artificial Sequence

<220>
<223>  Peptide capable of binding to a biological organic molecule

<400>  83

Val Leu Trp Leu Lys Asn Arg
1               5


<210>  84
<211>  9
<212>  PRT
<213>  Artificial Sequence

<220>
<223>  Peptide capable of binding to a biological organic molecule

<400>  84

Cys Thr Val Arg Thr Ser Ala Asp Cys
1               5


<210>  85
<211>  12
<212>  PRT
<213>  Artificial Sequence

<220>
<223>  Peptide capable of binding to a biological organic molecule

<400>  85

Ala Ala Ala Pro Leu Ala Gln Pro His Met Trp Ala
1               5                   10


<210>  86
<211>  7
<212>  PRT
<213>  Artificial Sequence

<220>
<223>  Peptide capable of binding to a biological organic molecule

<400>  86

Ser His Ser Leu Leu Ser Ser

1               5


<210>  87
<211>  12
<212>  PRT
<213>  Artificial Sequence

<220>
<223>  Peptide capable of binding to a biological organic molecule

<400>  87

Ala Leu Trp Pro Pro Asn Leu His Ala Trp Val Pro
1               5                   10


<210>  88
<211>  7
<212>  PRT
<213>  Artificial Sequence

<220>
<223>  Peptide capable of binding to a biological organic molecule

<400>  88

Leu Thr Val Ser Pro Trp Tyr
1               5


<210>  89
<211>  12
<212>  PRT
<213>  Artificial Sequence

<220>
<223>  Peptide capable of binding to a biological organic molecule

<400>  89

Ser Ser Met Asp Ile Val Leu Arg Ala Pro Leu Met
1               5                   10


<210>  90
<211>  12
<212>  PRT
<213>  Artificial Sequence

<220>
<223>  Peptide capable of binding to a biological organic molecule

<400>  90

Phe Pro Met Phe Asn His Trp Glu Gln Trp Pro Pro
1               5                   10


<210>  91
<211>  7
<212>  PRT
<213>  Artificial Sequence

<220>
<223>  Peptide capable of binding to a biological organic molecule

<400>  91

Ser Tyr Pro Ile Pro Asp Thr
1               5


<210>  92
<211>  7
<212>  PRT
<213>  Artificial Sequence

<220>
<223>  Peptide capable of binding to a biological organic molecule

<400>  92

His Thr Ser Asp Gln Thr Asn
1               5


<210>  93
<211>  9
<212>  PRT
<213>  Artificial Sequence

<220>
<223>  Peptide capable of binding to a biological organic molecule

<400>  93

Cys Leu Phe Met Arg Leu Ala Trp Cys
1               5


<210>  94
<211>  8
<212>  PRT
<213>  Artificial Sequence

<220>
<223>  Peptide capable of binding to a biological organic molecule

<400>  94

Asp Met Pro Gly Thr Val Leu Pro
1               5


<210>  95
<211>  9
<212>  PRT
<213>  Artificial Sequence

<220>
<223>  Peptide capable of binding to a biological organic molecule

<400>  95

Asp Trp Arg Gly Asp Ser Met Asp Ser

1               5


<210>  96
<211>  8
<212>  PRT
<213>  Artificial Sequence

<220>
<223>  Peptide capable of binding to a biological organic molecule

<400>  96

Val Pro Thr Asp Thr Asp Tyr Ser
1               5


<210>  97
<211>  9
<212>  PRT
<213>  Artificial Sequence

<220>
<223>  Peptide capable of binding to a biological organic molecule

<400>  97

Val Glu Glu Gly Gly Tyr Ile Ala Ala
1               5


<210>  98
<211>  12
<212>  PRT
<213>  Artificial Sequence

<220>
<223>  Peptide capable of binding to a biological organic molecule

<400>  98

Val Thr Trp Thr Pro Gln Ala Trp Phe Gln Trp Val
1               5                   10


<210>  99
<211>  7
<212>  PRT
<213>  Artificial Sequence

<220>
<223>  Peptide capable of binding to a biological organic molecule

<400>  99

Ala Gln Tyr Leu Asn Pro Ser
1               5


<210>  100
<211>  9
<212>  PRT
<213>  Artificial Sequence

<220>
<223>  Peptide capable of binding to a biological organic molecule

<400>  100

Cys Ser Ser Arg Thr Met His His Cys
1               5


<210>  101
<211>  9
<212>  PRT
<213>  Artificial Sequence

<220>
<223>  Peptide capable of binding to a biological organic molecule

<400>  101

Cys Pro Leu Asp Ile Asp Phe Tyr Cys
1               5


<210>  102
<211>  9
<212>  PRT
<213>  Artificial Sequence

<220>
<223>  Peptide capable of binding to a biological organic molecule

<400>  102

Cys Pro Ile Glu Asp Arg Pro Met Cys
1               5


<210>  103
<211>  18
<212>  PRT
<213>  Artificial Sequence

<220>
<223>  Peptide capable of binding to a biological organic molecule

<400>  103

Arg Gly Asp Leu Ala Thr Leu Arg Gln Leu Ala Gln Glu Asp Gly Val
1               5                   10                  15


Val Gly



<210>  104
<211>  12
<212>  PRT
<213>  Artificial Sequence

<220>
<223>  Peptide capable of binding to a biological organic molecule

<400>  104

Ser Pro Arg Gly Asp Leu Ala Val Leu Gly His Lys
1               5                   10


<210>  105
<211>  13
<212>  PRT
<213>  Artificial Sequence

<220>
<223>  Peptide capable of binding to a biological organic molecule

<400>  105

Ser Pro Arg Gly Asp Leu Ala Val Leu Gly His Lys Tyr
1               5                   10


<210>  106
<211>  12
<212>  PRT
<213>  Artificial Sequence

<220>
<223>  Peptide capable of binding to a biological organic molecule

<400>  106

Cys Gln Gln Ser Asn Arg Gly Asp Arg Lys Arg Cys
1               5                   10


<210>  107
<211>  12
<212>  PRT
<213>  Artificial Sequence

<220>
<223>  Peptide capable of binding to a biological organic molecule

<400>  107

Cys Met Gly Asn Lys Cys Arg Ser Ala Lys Arg Pro
1               5                   10


<210>  108
<211>  9
<212>  PRT
<213>  Artificial Sequence

<220>
<223>  Peptide capable of binding to a biological organic molecule

<400>  108

Cys Gly Glu Met Gly Trp Val Arg Cys
1               5

<210>  109
<211>  12
<212>  PRT
<213>  Artificial Sequence

<220>
<223>  Peptide capable of binding to a biological organic molecule

<400>  109

Gly Phe Arg Phe Gly Ala Leu His Glu Tyr Asn Ser
1               5                   10


<210>  110
<211>  9
<212>  PRT
<213>  Artificial Sequence

<220>
<223>  Peptide capable of binding to a biological organic molecule

<400>  110

Cys Thr Leu Pro His Leu Lys Met Cys
1               5


<210>  111
<211>  12
<212>  PRT
<213>  Artificial Sequence

<220>
<223>  Peptide capable of binding to a biological organic molecule

<400>  111

Ala Ser Gly Ala Leu Ser Pro Ser Arg Leu Asp Thr
1               5                   10


<210>  112
<211>  12
<212>  PRT
<213>  Artificial Sequence

<220>
<223>  Peptide capable of binding to a biological organic molecule

<400>  112

Ser Trp Asp Ile Ala Trp Pro Pro Leu Lys Val Pro
1               5                   10


<210>  113
<211>  13
<212>  PRT
<213>  Artificial Sequence

<220>
<223>  Peptide capable of binding to a biological organic molecule

<400>  113

Cys Thr Val Ala Leu Pro Gly Gly Tyr Val Arg Val Cys
1               5                   10


<210>  114
<211>  12
<212>  PRT
<213>  Artificial Sequence

<220>
<223>  Peptide capable of binding to a biological organic molecule

<400>  114

Glu Thr Ala Pro Leu Ser Thr Met Leu Ser Pro Tyr
1               5                   10


<210>  115
<211>  6
<212>  PRT
<213>  Artificial Sequence

<220>
<223>  Peptide capable of binding to a biological organic molecule

<400>  115

Gly Ile Arg Leu Arg Gly
1               5


<210>  116
<211>  9
<212>  PRT
<213>  Artificial Sequence

<220>
<223>  Peptide capable of binding to a biological organic molecule

<400>  116

Cys Pro Gly Pro Glu Gly Ala Gly Cys
1               5


<210>  117
<211>  9
<212>  PRT
<213>  Artificial Sequence

<220>
<223>  Peptide capable of binding to a biological organic molecule

<400>  117

Cys Gly Arg Arg Ala Gly Gly Ser Cys
1               5

<210>  118
<211>  7
<212>  PRT
<213>  Artificial Sequence

<220>
<223>  Peptide capable of binding to a biological organic molecule

<400>  118

Cys Arg Gly Arg Arg Ser Thr
1               5


<210>  119
<211>  13
<212>  PRT
<213>  Artificial Sequence

<220>
<223>  Peptide capable of binding to a biological organic molecule

<400>  119

Cys Asn Gly Arg Cys Val Ser Gly Cys Ala Gly Arg Cys
1               5                   10


<210>  120
<211>  9
<212>  PRT
<213>  Artificial Sequence

<220>
<223>  Peptide capable of binding to a biological organic molecule

<400>  120

Cys Gly Asn Lys Arg Thr Arg Gly Cys
1               5


<210>  121
<211>  7
<212>  PRT
<213>  Artificial Sequence

<220>
<223>  Peptide capable of binding to a biological organic molecule

<400>  121

His Val Gly Gly Ser Ser Val
1               5


<210>  122
<211>  7
<212>  PRT
<213>  Artificial Sequence

<220>
<223>  Peptide capable of binding to a biological organic molecule

<400>  122

Arg Gly Asp Gly Ser Ser Val
1               5


<210>  123
<211>  7
<212>  PRT
<213>  Artificial Sequence

<220>
<223>  Peptide capable of binding to a biological organic molecule

<400>  123

Ser Trp Lys Leu Pro Pro Ser
1               5


<210>  124
<211>  10
<212>  PRT
<213>  Artificial Sequence

<220>
<223>  Peptide capable of binding to a biological organic molecule

<400>  124

Cys Arg Gly Asp Lys Arg Gly Pro Asp Cys
1               5                   10


<210>  125
<211>  7
<212>  PRT
<213>  Artificial Sequence

<220>
<223>  Peptide capable of binding to a biological organic molecule

<400>  125

Gly Gly Lys Arg Pro Ala Arg
1               5


<210>  126
<211>  7
<212>  PRT
<213>  Artificial Sequence

<220>
<223>  Peptide capable of binding to a biological organic molecule

<400>  126

Arg Ile Gly Arg Pro Leu Arg
1               5

<210>  127
<211>  9
<212>  PRT
<213>  Artificial Sequence

<220>
<223>  Peptide capable of binding to a biological organic molecule

<400>  127

Cys Gly Phe Tyr Trp Leu Arg Ser Cys
1               5


<210>  128
<211>  7
<212>  PRT
<213>  Artificial Sequence

<220>
<223>  Peptide capable of binding to a biological organic molecule

<400>  128

Arg Pro Ala Arg Pro Ala Arg
1               5


<210>  129
<211>  7
<212>  PRT
<213>  Artificial Sequence

<220>
<223>  Peptide capable of binding to a biological organic molecule

<400>  129

Thr Leu Thr Tyr Thr Trp Ser
1               5


<210>  130
<211>  7
<212>  PRT
<213>  Artificial Sequence

<220>
<223>  Peptide capable of binding to a biological organic molecule

<400>  130

Ser Ser Gln Pro Phe Trp Ser
1               5


<210>  131
<211>  18
<212>  PRT
<213>  Artificial Sequence

<220>
<223>  Peptide capable of binding to a biological organic molecule

<400>  131

Tyr Arg Cys Thr Leu Asn Ser Pro Phe Phe Trp Glu Asp Met Thr His
1               5                   10                  15

Glu Cys

<210>  132
<211>  7
<212>  PRT
<213>  Artificial Sequence

<220>
<223>  Peptide capable of binding to a biological organic molecule

<400>  132

Lys Thr Leu Leu Pro Thr Pro
1               5

<210>  133
<211>  12
<212>  PRT
<213>  Artificial Sequence

<220>
<223>  Peptide capable of binding to a biological organic molecule

<400>  133

Lys Glu Leu Cys Glu Leu Asp Ser Leu Leu Arg Ile
1               5                   10

<210>  134
<211>  12
<212>  PRT
<213>  Artificial Sequence

<220>
<223>  Peptide capable of binding to a biological organic molecule

<400>  134

Ile Arg Glu Leu Tyr Ser Tyr Asp Asp Asp Phe Gly
1               5                   10

<210>  135
<211>  5
<212>  PRT
<213>  Artificial Sequence

<220>
<223>  Peptide capable of binding to a biological organic molecule

<400>  135

Asn Val Val Arg Gln
1               5


<210>  136
<211>  13
<212>  PRT
<213>  Artificial Sequence

<220>
<223>  Peptide capable of binding to a biological organic molecule

<400>  136

Val Glu Cys Tyr Leu Ile Arg Asp Asn Leu Cys Ile Tyr
1               5                   10


<210>  137
<211>  9
<212>  PRT
<213>  Artificial Sequence

<220>
<223>  Peptide capable of binding to a biological organic molecule

<400>  137

Cys Gly Gly Arg Arg Leu Gly Gly Cys
1               5


<210>  138
<211>  13
<212>  PRT
<213>  Artificial Sequence

<220>
<223>  Peptide capable of binding to a biological organic molecule

<400>  138

Trp Phe Cys Ser Trp Tyr Gly Gly Asp Thr Cys Val Gln
1               5                   10


<210>  139
<211>  19
<212>  PRT
<213>  Artificial Sequence

<220>
<223>  Peptide capable of binding to a biological organic molecule

<400>  139

Asn Gln Gln Leu Ile Glu Glu Ile Ile Gln Ile Leu His Lys Ile Phe
1               5                   10                  15


Glu Ile Leu

<210>  140
<211>  9
<212>  PRT
<213>  Artificial Sequence

<220>
<223>  Peptide capable of binding to a biological organic molecule

<400>  140

Lys Met Val Ile Tyr Trp Lys Ala Gly
1               5


<210>  141
<211>  10
<212>  PRT
<213>  Artificial Sequence

<220>
<223>  Peptide capable of binding to a biological organic molecule

<400>  141

Leu Asn Ile Val Ser Val Asn Gly Arg His
1               5                   10


<210>  142
<211>  12
<212>  PRT
<213>  Artificial Sequence

<220>
<223>  Peptide capable of binding to a biological organic molecule

<400>  142

Gln Met Ala Arg Ile Pro Lys Arg Leu Ala Arg His
1               5                   10


<210>  143
<211>  7
<212>  PRT
<213>  Artificial Sequence

<220>
<223>  Peptide capable of binding to a biological organic molecule

<400>  143

Gln Asp Gly Arg Met Gly Phe
1               5


<210>  144
<211>  7
<212>  PRT
<213>  Artificial Sequence

<220>

<223>  Peptide capable of binding to a biological organic molecule

<400>  144

Thr Arg Gln Ala Arg Arg Asn
1               5

<210>  145
<211>  17
<212>  PRT
<213>  Artificial Sequence

<220>
<223>  Peptide capable of binding to a biological organic molecule

<400>  145

Thr Arg Gln Ala Arg Arg Asn Arg Arg Arg Arg Trp Arg Glu Arg Gln
1               5                   10                  15

Arg

<210>  146
<211>  13
<212>  PRT
<213>  Artificial Sequence

<220>
<223>  Peptide capable of binding to a biological organic molecule

<400>  146

Thr Arg Arg Gln Arg Thr Arg Arg Ala Arg Arg Asn Arg
1               5                   10

<210>  147
<211>  17
<212>  PRT
<213>  Artificial Sequence

<220>
<223>  Peptide capable of binding to a biological organic molecule

<400>  147

Asn Ala Lys Thr Arg Arg His Glu Arg Arg Arg Lys Leu Ala Ile Glu
1               5                   10                  15

Arg

<210>  148
<211>  21
<212>  PRT
<213>  Artificial Sequence

<220>
<223>  Peptide capable of binding to a biological organic molecule

<400>  148

Met Asp Ala Gln Thr Arg Arg Arg Glu Arg Arg Ala Glu Lys Gln Ala
1               5                   10                  15

Gln Trp Lys Ala Ala
            20

<210>  149
<211>  9
<212>  PRT
<213>  Artificial Sequence

<220>
<223>  Peptide capable of binding to a biological organic molecule

<400>  149

Arg Lys Lys Arg Arg Gln Arg Arg Arg
1               5

<210>  150
<211>  17
<212>  PRT
<213>  Artificial Sequence

<220>
<223>  Peptide capable of binding to a biological organic molecule

<400>  150

Thr Arg Gln Ala Arg Arg Asn Arg Arg Arg Arg Trp Arg Glu Arg Gln
1               5                   10                  15

Arg

<210>  151
<211>  16
<212>  PRT
<213>  Artificial Sequence

<220>
<223>  Peptide capable of binding to a biological organic molecule

<400>  151

Lys Arg Ala Arg Asn Thr Glu Ala Ala Arg Arg Ser Arg Ala Arg Lys
1               5                   10                  15

<210>  152
<211>  12
<212>  PRT
<213>  Artificial Sequence

<220>
<223>  Peptide capable of binding to a biological organic molecule

<400>  152

Asp Val Phe Tyr Pro Tyr Pro Tyr Ala Ser Gly Ser
1               5                   10


<210>  153
<211>  15
<212>  PRT
<213>  Artificial Sequence

<220>
<223>  Peptide capable of binding to a biological organic molecule

<400>  153

Arg Val Trp Tyr Pro Tyr Gly Ser Tyr Leu Thr Ala Ser Gly Ser
1               5                   10                  15


<210>  154
<211>  9
<212>  PRT
<213>  Artificial Sequence

<220>
<223>  Peptide capable of binding to a biological organic molecule

<400>  154

Asp Thr Trp Pro Asn Thr Glu Trp Ser
1               5


<210>  155
<211>  7
<212>  PRT
<213>  Artificial Sequence

<220>
<223>  Peptide capable of binding to a biological organic molecule

<400>  155

Asp Ser Tyr His Asn Ile Trp
1               5


<210>  156
<211>  11
<212>  PRT
<213>  Artificial Sequence

<220>
<223>  Peptide capable of binding to a biological organic molecule

<400>  156

Asp Thr Tyr Phe Gly Lys Ala Tyr Asn Pro Trp

1               5                   10


<210>  157
<211>  10
<212>  PRT
<213>  Artificial Sequence

<220>
<223>  Peptide capable of binding to a biological organic molecule

<400>  157

Asp Thr Ile Gly Ser Pro Val Asn Phe Trp
1               5                   10


<210>  158
<211>  12
<212>  PRT
<213>  Artificial Sequence

<220>
<223>  Peptide capable of binding to a biological organic molecule

<400>  158

Thr Tyr Cys Asn Pro Gly Trp Asp Pro Arg Asp Arg
1               5                   10


<210>  159
<211>  15
<212>  PRT
<213>  Artificial Sequence

<220>
<223>  Peptide capable of binding to a biological organic molecule

<400>  159

Thr Phe Tyr Asn Glu Glu Trp Asp Leu Val Ile Lys Asp Glu His
1               5                   10                  15


<210>  160
<211>  10
<212>  PRT
<213>  Artificial Sequence

<220>
<223>  Peptide capable of binding to a biological organic molecule

<400>  160

Met Thr Leu Ile Leu Glu Leu Val Val Ile
1               5                   10


<210>  161
<211>  10
<212>  PRT
<213>  Artificial Sequence

<220>
<223>  Peptide capable of binding to a biological organic molecule

<400>  161

Met Thr Ser Ile Leu Glu Arg Glu Gln Arg
1               5                   10


<210>  162
<211>  10
<212>  PRT
<213>  Artificial Sequence

<220>
<223>  Peptide capable of binding to a biological organic molecule

<400>  162

Met Thr Thr Ile Leu Gln Gln Arg Glu Ser
1               5                   10


<210>  163
<211>  23
<212>  PRT
<213>  Artificial Sequence

<220>
<223>  Peptide capable of binding to a biological organic molecule

<400>  163

Val Phe Gln Phe Leu Gly Lys Ile Ile His His Val Gly Asn Phe Val
1               5                   10                  15


His Gly Phe Ser His Val Phe
            20


<210>  164
<211>  25
<212>  PRT
<213>  Artificial Sequence

<220>
<223>  Peptide capable of binding to a biological organic molecule

<400>  164

Lys Lys Ala Val Lys Val Pro Lys Lys Glu Lys Ser Val Leu Gln Gly
1               5                   10                  15


Lys Leu Thr Arg Leu Ala Val Gln Ile
            20                  25


<210>  165
<211>  6
<212>  PRT

<213>  Artificial Sequence

<220>
<223>  Titaniumu-binding peptide

<400>  165

Arg Lys Leu Pro Asp Ala
1               5


<210>  166
<211>  19
<212>  PRT
<213>  Artificial Sequence

<220>
<223>  Peptide capable of binding to a metal material

<400>  166

Ser Ser Lys Lys Ser Gly Ser Tyr Ser Gly Ser Lys Gly Ser Lys Arg
1               5                   10                  15


Arg Ile Leu


<210>  167
<211>  12
<212>  PRT
<213>  Artificial Sequence

<220>
<223>  Peptide capable of binding to a metal material

<400>  167

Ala Tyr Pro Gln Lys Phe Asn Asn Asn Phe Met Ser
1               5                   10


<210>  168
<211>  12
<212>  PRT
<213>  Artificial Sequence

<220>
<223>  Peptide capable of binding to a metal material

<400>  168

Arg Lys Leu Pro Asp Ala Pro Gly Met His Thr Trp
1               5                   10


<210>  169
<211>  6
<212>  PRT
<213>  Artificial Sequence

<220>

<223>  Peptide capable of binding to a metal material

<400>  169

Arg Ala Leu Pro Asp Ala
1               5


<210>  170
<211>  14
<212>  PRT
<213>  Artificial Sequence

<220>
<223>  Gold-binding peptide

<400>  170

Met His Gly Lys Thr Gln Ala Thr Ser Gly Thr Ile Gln Ser
1               5                   10


<210>  171
<211>  12
<212>  PRT
<213>  Artificial Sequence

<220>
<223>  Peptide capable of binding to a metal material

<400>  171

Thr Gly Thr Ser Val Leu Ile Ala Thr Pro Tyr Val
1               5                   10


<210>  172
<211>  12
<212>  PRT
<213>  Artificial Sequence

<220>
<223>  Peptide capable of binding to a metal material

<400>  172

Thr Gly Thr Ser Val Leu Ile Ala Thr Pro Gly Val
1               5                   10


<210>  173
<211>  12
<212>  PRT
<213>  Artificial Sequence

<220>
<223>  Peptide capable of binding to a metal material

<400>  173

Leu Lys Ala His Leu Pro Pro Ser Arg Leu Pro Ser
1               5                   10

<210>  174
<211>  19
<212>  PRT
<213>  Artificial Sequence

<220>
<223>  Peptide capable of binding to a silicon material

<400>  174

Ser Ser Lys Lys Ser Gly Ser Tyr Ser Gly Ser Lys Gly Ser Lys Arg
1               5                   10                  15

Arg Ile Leu

<210>  175
<211>  12
<212>  PRT
<213>  Artificial Sequence

<220>
<223>  Peptide capable of binding to a silicon material

<400>  175

Met Ser Pro His Pro His Pro Arg His His His Thr
1               5                   10

<210>  176
<211>  12
<212>  PRT
<213>  Artificial Sequence

<220>
<223>  Peptide capable of binding to a silicon material

<400>  176

Thr Gly Arg Arg Arg Arg Leu Ser Cys Arg Leu Leu
1               5                   10

<210>  177
<211>  12
<212>  PRT
<213>  Artificial Sequence

<220>
<223>  Peptide capable of binding to a silicon material

<400>  177

Lys Pro Ser His His His His His Thr Gly Ala Asn
1               5                   10

<210>  178
<211>  12

<212>  PRT
<213>  Artificial Sequence

<220>
<223>  Peptide capable of binding to a carbon nanomaterial

<400>  178

Asp Tyr Phe Ser Ser Pro Tyr Tyr Glu Gln Leu Phe
1               5                   10


<210>  179
<211>  12
<212>  PRT
<213>  Artificial Sequence

<220>
<223>  Peptide capable of binding to a carbon nanomaterial

<400>  179

His Ser Ser Tyr Trp Tyr Ala Phe Asn Asn Lys Thr
1               5                   10


<210>  180
<211>  7
<212>  PRT
<213>  Artificial Sequence

<220>
<223>  Peptide capable of binding to a carbon nanomaterial

<400>  180

Tyr Asp Pro Phe His Ile Ile
1               5


<210>  181
<211>  7
<212>  PRT
<213>  Artificial Sequence

<220>
<223>  Peptide which can be degraded by protease

<400>  181

Gly Arg Arg Gly Lys Gly Gly
1               5


<210>  182
<211>  5
<212>  PRT
<213>  Artificial Sequence

<220>
<223>  Peptide which can be degraded by protease

<400>  182

Gly Pro Leu Gly Val
1               5

<210>  183
<211>  7
<212>  PRT
<213>  Artificial Sequence

<220>
<223>  Peptide which can be degraded by protease

<400>  183

Gly Pro Leu Gly Val Arg Gly
1               5

<210>  184
<211>  11
<212>  PRT
<213>  Artificial Sequence

<220>
<223>  Peptide which can be degraded by protease

<400>  184

Gly Gly Leu Val Pro Arg Gly Ser Gly Ala Ser
1               5                   10

<210>  185
<211>  6
<212>  PRT
<213>  Artificial Sequence

<220>
<223>  Peptide which can be degraded by protease

<400>  185

Tyr Glu Val Asp Gly Trp
1               5

<210>  186
<211>  6
<212>  PRT
<213>  Artificial Sequence

<220>
<223>  Peptide which can be degraded by protease

<400>  186

Leu Glu Val Asp Gly Trp
1               5

<210>  187
<211>  7

<212>  PRT
<213>  Artificial Sequence

<220>
<223>  Peptide which can be degraded by protease

<400>  187

Val Asp Gln Met Asp Gly Trp
1               5


<210>  188
<211>  7
<212>  PRT
<213>  Artificial Sequence

<220>
<223>  Peptide which can be degraded by protease

<400>  188

Val Asp Val Ala Asp Gly Trp
1               5


<210>  189
<211>  6
<212>  PRT
<213>  Artificial Sequence

<220>
<223>  Peptide which can be degraded by protease

<400>  189

Val Gln Val Asp Gly Trp
1               5


<210>  190
<211>  7
<212>  PRT
<213>  Artificial Sequence

<220>
<223>  Peptide which can be degraded by protease

<400>  190

Val Asp Gln Val Asp Gly Trp
1               5


<210>  191
<211>  11
<212>  PRT
<213>  Artificial Sequence

<220>
<223>  Peptide which can be degraded by protease

<400>  191

Glu Leu Ser Leu Ser Arg Leu Arg Asp Ser Ala
1               5                   10

<210>  192
<211>  8
<212>  PRT
<213>  Artificial Sequence

<220>
<223>  Peptide which can be degraded by protease

<400>  192

Glu Leu Ser Leu Ser Arg Leu Arg
1               5

<210>  193
<211>  8
<212>  PRT
<213>  Artificial Sequence

<220>
<223>  Peptide which can be degraded by protease

<400>  193

Asp Asn Tyr Thr Arg Leu Arg Lys
1               5

<210>  194
<211>  8
<212>  PRT
<213>  Artificial Sequence

<220>
<223>  Peptide which can be degraded by protease

<400>  194

Tyr Thr Arg Leu Arg Lys Gln Met
1               5

<210>  195
<211>  8
<212>  PRT
<213>  Artificial Sequence

<220>
<223>  Peptide which can be degraded by protease

<400>  195

Ala Pro Ser Gly Arg Val Ser Met
1               5

<210>  196
<211>  8

<212>  PRT
<213>  Artificial Sequence

<220>
<223>  Peptide which can be degraded by protease

<400>  196

Val Ser Met Ile Lys Asn Leu Gln
1               5


<210>  197
<211>  8
<212>  PRT
<213>  Artificial Sequence

<220>
<223>  Peptide which can be degraded by protease

<400>  197

Arg Ile Arg Pro Lys Leu Lys Trp
1               5


<210>  198
<211>  8
<212>  PRT
<213>  Artificial Sequence

<220>
<223>  Peptide which can be degraded by protease

<400>  198

Asn Phe Phe Trp Lys Thr Phe Thr
1               5


<210>  199
<211>  8
<212>  PRT
<213>  Artificial Sequence

<220>
<223>  Peptide which can be degraded by protease

<400>  199

Lys Met Tyr Pro Arg Gly Asn His
1               5


<210>  200
<211>  8
<212>  PRT
<213>  Artificial Sequence

<220>
<223>  Peptide which can be degraded by protease

<400>  200

Gln Thr Tyr Pro Arg Thr Asn Thr
1               5


<210>  201
<211>  8
<212>  PRT
<213>  Artificial Sequence

<220>
<223>  Peptide which can be degraded by protease

<400>  201

Gly Val Tyr Ala Arg Val Thr Ala
1               5


<210>  202
<211>  8
<212>  PRT
<213>  Artificial Sequence

<220>
<223>  Peptide which can be degraded by protease

<400>  202

Ser Gly Leu Ser Arg Ile Val Asn
1               5


<210>  203
<211>  5
<212>  PRT
<213>  Artificial Sequence

<220>
<223>  Peptide which can be degraded by protease

<400>  203

Asn Ser Arg Val Ala
1               5


<210>  204
<211>  5
<212>  PRT
<213>  Artificial Sequence

<220>
<223>  Peptide which can be degraded by protease

<400>  204

Gln Val Arg Leu Gly
1               5


<210>  205
<211>  6

<212>  PRT
<213>  Artificial Sequence

<220>
<223>  Peptide which can be degraded by protease

<400>  205

Met Lys Ser Arg Asn Leu
1               5


<210>  206
<211>  6
<212>  PRT
<213>  Artificial Sequence

<220>
<223>  Peptide which can be degraded by protease

<400>  206

Arg Cys Lys Pro Val Asn
1               5


<210>  207
<211>  6
<212>  PRT
<213>  Artificial Sequence

<220>
<223>  Peptide which can be degraded by protease

<400>  207

Ser Ser Lys Tyr Pro Asn
1               5


<210>  208
<211>  6
<212>  PRT
<213>  Artificial Sequence

<220>
<223>  Peptide which can be degraded by protease

<400>  208

Leu Val Pro Arg Gly Ser
1               5


<210>  209
<211>  6
<212>  PRT
<213>  Artificial Sequence

<220>
<223>  Stabilization peptide

<400>  209

Cys Cys Ala Leu Asn Asn
1               5


<210>  210
<211>  3
<212>  PRT
<213>  Artificial Sequence

<220>
<223>  Stabilization peptide

<400>  210

Pro Ala Ser
1


<210>  211
<211>  13
<212>  PRT
<213>  Artificial Sequence

<220>
<223>  Cell-penetrating peptide

<400>  211

Gly Arg Lys Lys Arg Arg Gln Arg Arg Arg Pro Pro Gln
1               5                   10


<210>  212
<211>  16
<212>  PRT
<213>  Artificial Sequence

<220>
<223>  Cell-penetrating peptide

<400>  212

Arg Gln Ile Lys Ile Trp Phe Gln Asn Arg Arg Met Lys Trp Lys Lys
1               5                   10                  15


<210>  213
<211>  13
<212>  PRT
<213>  Artificial Sequence

<220>
<223>  Cell-penetrating peptide

<400>  213

Cys Gly Tyr Gly Pro Lys Lys Lys Arg Lys Val Gly Gly
1               5                   10


<210>  214
<211>  8

<212>  PRT
<213>  Artificial Sequence

<220>
<223>  Cell-penetrating peptide

<400>  214

Arg Arg Arg Arg Arg Arg Arg Arg
1               5


<210>  215
<211>  8
<212>  PRT
<213>  Artificial Sequence

<220>
<223>  Cell-penetrating peptide

<400>  215

Lys Lys Lys Lys Lys Lys Lys Lys
1               5


<210>  216
<211>  15
<212>  PRT
<213>  Artificial Sequence

<220>
<223>  Cell-penetrating peptide

<400>  216

Gly Leu Ala Phe Leu Gly Phe Leu Gly Ala Ala Gly Ser Thr Met
1               5                   10                  15


<210>  217
<211>  12
<212>  PRT
<213>  Artificial Sequence

<220>
<223>  Cell-penetrating peptide

<400>  217

Gly Ala Trp Ser Gln Pro Lys Lys Lys Arg Lys Val
1               5                   10


<210>  218
<211>  18
<212>  PRT
<213>  Artificial Sequence

<220>
<223>  Cell-penetrating peptide

<400>  218

Leu Leu Ile Ile Leu Arg Arg Arg Ile Arg Lys Gln Ala His Ala His
1               5                   10                  15

Ser Lys

<210>  219
<211>  9
<212>  PRT
<213>  Artificial Sequence

<220>
<223>  Cell-penetrating peptide

<400>  219

Met Val Arg Arg Phe Leu Val Thr Leu
1               5

<210>  220
<211>  13
<212>  PRT
<213>  Artificial Sequence

<220>
<223>  Cell-penetrating peptide

<400>  220

Arg Ile Arg Arg Ala Cys Gly Pro Pro Arg Val Arg Val
1               5                   10

<210>  221
<211>  15
<212>  PRT
<213>  Artificial Sequence

<220>
<223>  Cell-penetrating peptide

<400>  221

Met Val Lys Ser Lys Ile Gly Ser Trp Ile Leu Val Leu Phe Val
1               5                   10                  15

<210>  222
<211>  10
<212>  PRT
<213>  Artificial Sequence

<220>
<223>  Cell-penetrating peptide

<400>  222

Ser Asp Val Gly Leu Cys Lys Lys Arg Pro
1               5                   10

<210>  223
<211>  18
<212>  PRT
<213>  Artificial Sequence

<220>
<223>  Cell-penetrating peptide

<400>  223

Asn Ala Ala Thr Ala Thr Arg Gly Arg Ser Ala Ala Ser Arg Pro Thr
1               5                   10                  15

Gln Arg

<210>  224
<211>  16
<212>  PRT
<213>  Artificial Sequence

<220>
<223>  Cell-penetrating peptide

<400>  224

Pro Arg Ala Pro Ala Arg Ser Ala Ser Arg Pro Arg Arg Pro Val Gln
1               5                   10                  15

<210>  225
<211>  12
<212>  PRT
<213>  Artificial Sequence

<220>
<223>  Cell-penetrating peptide

<400>  225

Asp Pro Lys Gly Asp Pro Lys Gly Val Thr Val Thr
1               5                   10

<210>  226
<211>  14
<212>  PRT
<213>  Artificial Sequence

<220>
<223>  Cell-penetrating peptide

<400>  226

Val Thr Val Thr Val Thr Gly Lys Gly Asp Pro Lys Pro Asp
1               5                   10

<210>  227

<211>  9
<212>  PRT
<213>  Artificial Sequence

<220>
<223>  Cell-penetrating peptide

<400>  227

Lys Leu Ala Leu Lys Leu Ala Leu Lys
1               5


<210>  228
<211>  9
<212>  PRT
<213>  Artificial Sequence

<220>
<223>  Cell-penetrating peptide

<400>  228

Ala Leu Lys Ala Ala Leu Lys Leu Ala
1               5


<210>  229
<211>  12
<212>  PRT
<213>  Artificial Sequence

<220>
<223>  Cell-penetrating peptide

<400>  229

Gly Trp Thr Leu Asn Ser Ala Gly Tyr Leu Leu Gly
1               5                   10


<210>  230
<211>  15
<212>  PRT
<213>  Artificial Sequence

<220>
<223>  Cell-penetrating peptide

<400>  230

Lys Ile Asn Leu Lys Ala Leu Ala Ala Leu Ala Lys Lys Ile Leu
1               5                   10                  15


<210>  231
<211>  13
<212>  PRT
<213>  Artificial Sequence

<220>
<223>  Cell-penetrating peptide

<400>  231

Arg Leu Ser Gly Met Asn Glu Val Leu Ser Phe Arg Trp
1               5                   10


<210>  232
<211>  15
<212>  PRT
<213>  Artificial Sequence

<220>
<223>  Cell-penetrating peptide

<400>  232

Ser Asp Leu Trp Glu Met Met Met Val Ser Leu Ala Cys Gln Tyr
1               5                   10                  15


<210>  233
<211>  10
<212>  PRT
<213>  Artificial Sequence

<220>
<223>  Cell-penetrating peptide

<400>  233

Pro Ile Glu Val Cys Met Tyr Arg Glu Pro
1               5                   10


<210>  234
<211>  36
<212>  DNA
<213>  Artificial Sequence

<220>
<223>  Primer

<400>  234
gaaggagata tacatatgag ctcccagatt cgtcag                                36


<210>  235
<211>  36
<212>  DNA
<213>  Artificial Sequence

<220>
<223>  Primer

<400>  235
ctcgaattcg gatccttagt cgtgcttgag agtgag                                36

## Description

### TECHNICAL FIELD

The present invention relates to a method for producing organic compound-encapsulating ferritin and the like.

### BACKGROUND ART

Ferritin is a spherical protein that is ubiquitously present in organisms from animals and plants to microorganism and has an internal cavity formed by a plurality of monomers. In animals such as humans, it is known that two kinds of monomers of H and L chains are present as ferritin, and that ferritin is a multimer that comprises 24 monomers (in many cases, a mixture of H chain monomers and L chain monomers) and has a cage form with an outer diameter of 12 nm with an internal cavity with an inner diameter of 7 nm. It is known that ferritin is deeply involved in homeostasis of an iron element in the living organisms and cells, and holds iron inside the internal cavity thereof for playing physiological functions such as transporting and storing iron.

Ferritin is contemplated also for application as a DDS carrier that encapsulates a reagent in the internal cavity thereof and utilized also in production of electronic devices. Several methods of encapsulating an organic compound in ferritin have been reported in connection with such applications of ferritin. Specifically, as such methods, (1) a method for controlling disassembly and reassembly of ferritin by changing pH of a solution, (2) a method for modifying ferritin with a modifying reagent, and refolding, and (3) a method by pressurizing have been reported.

For example, in Non Patent Literature 1, it is reported that approximately 28 doxorubicin molecules can be encapsulated in ferritin by disassembling ferritin under an acidic condition at pH 2 and then reassembling ferritin by changing the pH of the solution into 7-9.

In Non Patent Literature 2, it is reported that approximately 30-90 doxorubicin molecules can be encapsulated in ferritin by modifying ferritin to improve stability of ferritin as well as changing the pH of the solution into pH 2 by addition of HCl to adjust the disassembly and reassembly of ferritin.

In Non Patent Literature 3, it is reported that approximately 33 doxorubicin molecules can be encapsulated in ferritin while a natural folding structure of ferritin is restored by denaturing ferritin with a modifying reagent such as urea at the beginning, then mixing the modified ferritin with a reagent, and then removing the modifying reagent in a solution.

In Patent Literature 1, it is reported that approximately 10-20 doxorubicin molecules can be encapsulated in ferritin by leaving a mixture solution of ferritin and doxorubicin to stand at high pressure (200-800 MPa) for 6-20 hours.

### PRIOR ART REFERENCES

### PATENT LITERATURES

Patent Literature 1: Chinese Patent Application Laid-open No. 106110333

### NON PATENT LITERATURES

Non Patent Literature 1: Journal of Controlled Release 196 (2014) 184-196.

Non Patent Literature 2: Biomacromolecules 2016, 17, 514-522.

Non Patent Literature 3: Proc Natl Acad Sci USA. 2014 111 (41): 14900-5.

### SUMMARY OF INVENTION

### PROBLEM TO BE SOLVED BY THE INVENTION

The conventional methods described above have problems such as low recovery yield of ferritin and low encapsulation efficiency of organic compounds in ferritin as well as burdens in terms of complexity of operation, need for specific apparatus, and requirement of specific modification on surfaces of ferritin for improvement of the encapsulation efficiency.

More specifically, the above methods of (1) and (2) require destruction (disassembly/ denature) of a ferritin structure with use of an acidic buffer or a modifying reagent, and have problems such as lowered recovery yield of ferritin because a part of ferritin remains destroyed without being restored to original state even when a structural recovery process (reassembly / refolding) is provided once the ferritin structure is destroyed. In addition, the methods of (1) and (2) merely involves incorporating an organic compound distributed in a solution (reaction field) and encapsulating it into ferritin, thereby having problems such as a small amount of the organic compound encapsulated in ferritin because of incapability of encapsulating the organic compound at a higher concentration than that in the solution.

In the method of (1), substitution of the buffer is required for need of changing a pH condition. In the method of (2), it is necessary not only to remove the modifying reagent for refolding, but also to specially modify the surface of ferritin for the purpose of improving the encapsulation efficiency. Therefore, the methods of (1) and (2) are complicated.

Furthermore, the above method (3) causes overload as it requires a special equipment capable of achieving very high pressure conditions.

Accordingly, the present invention is aimed at providing an alternative method for producing an organic compound-encapsulating ferritin, which can solve one or more problem as described above.

### SOLUTION TO PROBLEM

Up to now, it is known that a metal atom and a metal compound (e.g., metal oxide) can be encapsulated in ferritin under a condition that does not cause destruction (disassembly / denature) of a ferritin structure because of small sizes of the metal atom and the metal compound.

However, since organic compounds were considered to have relatively large sizes without ferritin permeability to the ferritin structure, it was believed that the organic compound were unable to be encapsulated in ferritin under the condition that does not cause destruction of the ferritin structure. In fact, Non Patent Literature 1 discloses strong acidic condition (pH is approximately 2.5 or less) capable of causing the disassembly of ferritin is employed to disassemble ferritin for the encapsulation of doxorubicin into ferritin.

As a result of intensive studies, the present inventors have found that organic compounds can be encapsulated in ferritin by a simple method of using a buffer having a pH appropriate for an acid dissociation constant (pKa) of an organic compound within a pH range that does not destroy the ferritin structure.

That is, the present invention is as follows.
[1] A method for producing an organic compound-encapsulating ferritin, the method comprising:
   (1) mixing an organic compound with ferritin in a buffer to obtain a mixture of the organic compound and ferritin; and
   (2) incubating the mixture in the buffer,
   wherein the buffer has a pH of 3 or more and less than 13, and
   the buffer has a pH of 3 or more and less than 7 when the organic compound has a pKa less than 7, and has a pH of 6 or more and less than 13 when the organic compound has a pKa of 7 or more.
[2] The method according to [1], wherein the pH of the buffer and the pKa of the organic compound satisfy a relation defined by formula: pH = 2.6 + 0.6 pKa ± 0.4 pKa.
[3] The method according to [1] or [2], wherein a temperature of the incubating is 10°C or more and 60°C or less.
[4] The method according to any one of [1] to [3], wherein a molecular weight of the organic compound is 100 or more and less than 1000.
[5] The method according to any one of [1] to [4], wherein the pKa of the organic compound is 2 or more and 13 or less.
[6] The method according to any of [1] to [5], wherein 10 or more and 200 or less molecules of the organic compound are encapsulated in one molecule of ferritin.
[7] The method according to any of [1] to [6], wherein a total time for the mixing and the incubating is 30 minutes or more.
[8] The method according to any of [1] to [7], wherein a weight ratio of the organic compound to ferritin (organic compound / ferritin) at the mixing is 0.20 or more and 0.36 or less.
[9] The method according to any of [1] to [8], wherein the organic compound has a positively charged portion or a portion capable of being positively charged.
[10] The method according to [9], wherein the positively charged portion is (a) a cationic nitrogen-containing group selected from the group consisting of ammonium group, guanidinium group, imidazolium group, oxazolium group, thiazolium group, oxadiazolium group, triazolium group, pyrrolidinium group, pyridinium group, piperidinium group, pyrazolium group, pyrimidinium group, pyrazinium group and triazinium group or (b) phosphonium group.
[11] The method according to [9] or [10], wherein the portion capable of being positively charged is (a') a nitrogen-containing group selected from the group consisting of amino group, guanidino group, nitro group, amide group, hydrazide group, imide group, azide group and diazo group or (b') phosphino group.
[12] The method according to any of [1] or [11], wherein ferritin is (1) naturally occurring ferritin or (2) ferritin including a genetically modified ferritin monomer with any of following modifications (a) to (c):
   (a) a ferritin monomer with a functional peptide inserted into a flexible linker region between second and third α-helices counted from an N-terminus of the ferritin monomer comprising six α-helices;
   (b) a ferritin monomer with the functional peptide added to the N-terminus; or
   (c) a ferritin monomer with the functional peptide added to the C-terminus.
[13] A buffer comprising an organic compound-encapsulating ferritin,
   wherein the buffer has a pH of 3 or more and less than 7 when an organic compound has a pKa less than 7, and has a pH of 6 or more and less than 12 when the organic compound has a pKa of 7 or more.
[14] The buffer according to [13], wherein the buffer has a pH of 6 or more and 9 or less.
[15] The buffer according to [13] or [14], wherein the organic compound has a pKa of 6 or more and 9 or less.
[16] An organic compound-encapsulating ferritin, wherein
   an organic compound is selected from the group consisting of an anthracycline substance, a histamine H2 receptor antagonist, a piguanide substance, an ATP-sensitive potassium channel opening agent, a β2 adrenergic receptor agonist, an imidazoline substance, a vitamin, or a labeling substance, and
   number of molecules of the organic compound encapsulated into one molecule of ferritin is as follows:
   (1) 40 or more and 200 or less molecules when the organic compound is the anthracycline substance and ferritin is naturally occurring ferritin;
   (2) 100 or more and 200 or less molecules when the organic compound is the anthracycline substance and ferritin is genetically modified ferritin; or
   (3) 10 or more and 200 or less molecules when the organic compound is the histamine H2 receptor antagonist, the piguanide substance, the ATP-sensitive potassium channel opening agent, the β2 adrenergic receptor agonist, the imidazoline substance, the vitamin, or the labeling substance, and ferritin is naturally occurring ferritin or genetically modified ferritin.
[17] The organic compound-encapsulating ferritin according to [16],
   wherein the anthracycline substance is doxorubicin,
   the histamine H2 receptor antagonist is famotidine,
   the piguanide substance is metformin,
   the ATP-sensitive potassium channel opening agent is minoxidil,
   the β2 adrenergic receptor agonist is terbutaline,
   the imidazoline substance is creatinine,
   the vitamin is thiamine, riboflavin or nicotinamide,
   the labeling substance is rhodamine B, uranine or Congo red.
[18] The organic compound-encapsulating ferritin according to [16] or [17], wherein the organic compound is selected from the group consisting of the anthracycline substance, the histamine H2 receptor antagonist, the piguanide substance, the ATP-sensitive potassium channel opening agent and the β2 adrenergic receptor agonist.
[19] The organic compound-encapsulating ferritin according to any of [16] to [18], wherein ferritin is (1) naturally occurring ferritin or (2) ferritin including a genetically modified ferritin monomer with any of following modifications (a) to (c):
   (a) a ferritin monomer with a functional peptide inserted into a flexible linker region between second and third α-helices counted from an N-terminus of the ferritin monomer comprising six α-helices;
   (b) a ferritin monomer with the functional peptide added to the N-terminus; or
   (c) a ferritin monomer with the functional peptide added to the C-terminus.

### EFFECT OF THE INVENTION

According to the method of the present invention, an organic compound can be encapsulated in ferritin by a simple method using a buffer with a pH appropriate for pKa of the organic compound.

The method of the present invention is excellent also in the production efficiency of the organic compound-encapsulating ferritin. First, the method of the present invention does not require destroying (disassembling / denaturing) a ferritin structure, thereby achieving more superior production efficiency than conventional methods that require destroying (disassembling / denaturing) the ferritin structure as well as restoring processes (parts of ferritin remains destroyed without recovery even by providing the restoring process). Next, the method of the present invention enables encapsulating the organic compound into ferritin at a higher concentration than that in a solution by utilizing an electrochemical potential difference between inside and outside of ferritin in such an environment as to maintain the ultramolecular structure of ferritin, thereby encapsulating more organic compounds into ferritin efficiently than the conventional method (method of incorporating the solution into the internal cavity of ferritin while the ferritin structure is restored) that has a difficulty in encapsulating the organic compound into ferritin at a higher concentration than that in the solution.

The method of the present invention is particularly simple because of no need for the buffer substitution, removal of the modifying reagent, or the special apparatus, unlike the conventional method.

### BRIEF DESCRIPTION OF DRAWINGS

FIG. 1 is a graph representing an incorporation efficiency (wt%) of a reagent into ferritin in different reaction pHs.
FIG. 2 is a graph representing the incorporation efficiency (wt%) of the reagent into ferritin at different temperatures.
FIG. 3 is a graph representing a time variance of the incorporation efficiency (wt%) of the reagent into ferritin.
FIG. 4 is a plot representing a relation between the incorporation efficiency (wt%) of the reagent and concentration ratios of the reagent and ferritin (protein) in a reaction solution.
FIG. 5 is a profile representing overlap of column elution peaks of ferritin and the reagent (that is, encapsulation of the reagent into ferritin) after a reaction for the encapsulation of the reagent into ferritin.
FIG. 6 is a graph that demonstrates presence of a composite (aqueous dispersive nanoparticle) of reagent-encapsulating ferritin with use of Zetasizer Nano ZS (Malvern Panalytical Ltd.).
FIG. 7 is a graph that demonstrates that a surface charge of ferritin without doxorubicin being encapsulated therein is equivalent to that of the reagent-encapsulating ferritin (therefore, the composite is not formed by adsorbing doxorubicin on the ferritin surface).
FIG. 8 is a graph representing pH stability of the reagent-encapsulating ferritin.
FIG. 9 is a graph representing a relation between the incorporation efficiency of the reagent and a standing time after ferritin is disassembled at a conventional disassembly-reassembly process.
FIG. 10 is a profile representing comparison of a one-step process of the present invention with the conventional disassembly-reassembly process in terms of a ferritin recovery yield at each process.
FIG. 11 is a graph representing correlation between pKa for different small molecule agents and pHs (optimum pHs) of buffers used for reactions achieving the largest amounts of the reagents incorporated in ferritin. An approximate linear line refers to the middle line (pH = 2.6 + 0.6 pKa) in FIG. 11. The pKa of each small molecule agent and the optimum pH is within a range defined by formula: pH = 2.6 + 0.6 pKa ± 0.4 pKa (intercept: 2.6; slope: 0.6; coefficient of variation: 0.4).
FIG. 12 is a graph representing ratios of amounts of incorporated reagents at the one-step process of the present invention to those at the conventional disassembly-reassembly process.
FIG. 13 is a graph representing results of measurement of ferritin sizes at different pHs by a dynamic light scattering (DLS) method with use of Zetasizer Nano ZS.
FIG. 14 is a graph representing results of stability evaluation for uranine-encapsulating ferritin in plasma.
FIG. 15 is a graph representing measurement results of incorporation of uranine-encapsulating ferritin into a transferrin receptor TfR presenting cell (SKBR-3 cell) by fluorescence activated cell sorting (FACS).
FIG. 16 is a graph representing measurement results of incorporation of the uranine-encapsulating ferritin into the transferrin receptor TfR presenting cell (SKBR-3 cell) with two-photon excitation fluorescence microscope.

### EMBODIMENTS FOR CARRYING OUT THE INVENTION

The present invention provides a method for producing an organic compound-encapsulating ferritin. The method comprises:
(1) mixing an organic compound with ferritin in a buffer to obtain a mixture of the organic compound and ferritin; and
(2) incubating the mixture in the buffer, in which

   the buffer has a pH of 3 or more and less than 13, and the buffer has a pH of 3 or more and less than 7 when a pKa of the organic compound is less than 7, and has a pH of 6 or more and less than 13 when the pKa of the organic compound is 7 or more.

Ferritin (24 multimeric protein) is universally present in various higher organisms such as mammals. It is known that ferritin monomers constituting ferritin have six α-helices highly conserved among various higher organisms and that two monomers of H and L chains are present as the ferritin monomers of the higher organisms.

In the present invention, ferritin monomers of mammals can be used as the ferritin monomers constituting ferritin. Examples of mammals include primates (e.g., humans, monkeys, chimpanzees), rodents (e.g., mice, rats, hamsters, guinea pigs, rabbits), and livestock and working mammals (e.g., cattle, pigs, sheep, goats and horses). It is possible to use H chain, L chain or a mixture thereof as the ferritin monomer. It is possible to use either a naturally occurring ferritin monomer or a genetically modified ferritin monomer capable of forming 24-mer as the ferritin monomer.

In one embodiment, the genetically modified ferritin monomer may be modified in a flexible linker region between α-helices among six α-helices constituting the ferritin monomer. Examples of such a genetically modified ferritin monomer include a ferritin monomer with a functional peptide inserted into a flexible linker region between first and second, second and third, third and fourth, fourth and fifth, or fifth and sixth (preferably second and third) α-helices counted from N-terminus of the ferritin monomer comprising six α-helices (Examples of this application, and US Patent Application Publication No. 2016/0060307; Jae Og Jeon et al., ACS Nano (2013), 7 (9), 7462-7471; Sooji Kim et al., Biomacromolecules (2016), 17 (3), 1150-1159; Young Ji Kang et al., Biomacromolecules (2012), 13 (12), 4057-4064). For example, Table A lists first to sixth α-helices of six α-helices counted from N-terminus of a human ferritin H chain (SEQ ID NO: 1) and a human ferritin L chain (SEQ ID NO: 2). The flexible linker region of the human ferritin H chain (SEQ ID NO: 1) refers to the flexible linker region between first and second (region including 43rd to 49th amino acid residues), the flexible linker region between second and third (region including 78th to 96th amino acid residues), the flexible linker region between third and fourth (region including 125th to 127th amino acid residues), the flexible linker region between fourth and fifth (position at 138th amino acid residue), or the flexible linker region between fifth and sixth (region including 160th to 164th amino acid residues). The flexible linker region of the human ferritin L chain (SEQ ID NO: 2) refers to the flexible linker region between first and second (region including 38th to 45th amino acid residues), the flexible linker region between second and third (region including 74th to 92nd amino acid residues), the flexible linker region between third and fourth (region including 121st to 123rd amino acid residues), the flexible linker region between fourth and fifth (position at 134th amino acid residue), or the flexible linker region between fifth and sixth (region including 155th to 159th amino acid residues).

### [TABLE A]

**Table A. Positions of α-helices in ferritin**

| α-helices | Positions of amino acid residues in amino acid sequences | |
|---|---|---|
| | Human ferritin | |
| | H chain (SEQ ID NO: 1) | L chain (SEQ ID NO: 2) |
| First | 15-42 | 11-37 |
| Second | 50-77 | 46-73 |
| Third | 97-124 | 93-120 |
| Fourth | 128-137 | 124-133 |
| Fifth | 139-159 | 135-154 |
| Sixth | 165-174 | 160-170 |

| | | |
|---|---|---|
| Classification of α-helices is based on Int J Mol Sci. 2011; 12 (8): 5406-5421. | | |

In another embodiment, the genetically modified ferritin monomer may be modified in the N-terminus region and/or C-terminus region thereof. The N-terminus region of the ferritin monomer is exposed on a surface of the multimer, while the C-terminus thereof is unable to be exposed on the surface. As such, the peptide moiety added to the N-terminus of the ferritin monomer is exposed on the surface of the multimer, and is thereby capable of interacting with the target material present outside the multimer (e.g., WO2006/126595). On the other hand, the C-terminus of the ferritin monomer can interact with the organic compound inside the ferritin cavity by modifying its amino acid residue (e.g., Y. J. Kang, Biomacromolecules. 2012, vol. 13 (12), 4057). Examples of such a genetically modified ferritin monomer include a ferritin monomer with the functional peptide added to the N-terminus or C-terminus.

Preferably, ferritin is human ferritin from the viewpoint of clinical application to humans. It is possible to use the human ferritin H chain, the human ferritin L chain or a mixture thereof as the ferritin monomer each constituting the human ferritin.

In the present invention, one molecule (or one unit) of ferritin refers to a complex (24-mer) including 24 ferritin monomers, and the ferritin monomer does not correspond to the one molecule (or one unit) of ferritin.

In a specific embodiment, the ferritin H chain may be as follows:
(A1) a protein comprising the amino acid sequence of SEQ ID NO: 1;
(B1) a protein comprising an amino acid sequence including one or several modification(s) of an amino acid residue(s) selected from the group consisting of substitution, deletion, insertion and addition of the amino acid residue(s) in the amino acid sequence of SEQ ID NO: 1, the protein capable of forming a multimer (e.g., 24-mer); or
(C1) a protein comprising an amino acid sequence having 90% or more homology to the amino acid sequence of SEQ ID NO: 1, the protein capable of forming a multimer (e.g., 24-mer).

Preferably, the ferritin L chain may be as follows:
(A2) a protein comprising the amino acid sequence of SEQ ID NO: 2;
(B2) a protein comprising an amino acid sequence including one or several modification(s) of an amino acid residue(s) selected from the group consisting of substitution, deletion, insertion and addition of the amino acid residue(s) in the amino acid sequence of SEQ ID NO: 2, the protein capable of forming a multimer (e.g., 24-mer); or
(C2) a protein comprising an amino acid sequence having 90% or more homology to the amino acid sequence of SEQ ID NO: 2, the protein capable of forming a multimer (e.g., 24-mer).

In the proteins (B1) and (B2), one or several amino acid residue(s) can be modified by one, two, three or four kinds of modifications selected from the group consisting of deletion, substitution, addition and insertion of amino acid residues. The modification of the amino acid residues may be introduced into one region in the amino acid sequence, or may be introduced into a plurality of different regions. The term "one or several" represents the number that is selected not to impair activities of the protein. The number represented by the term "one or several" refers to 1 to 50, for example, preferably 1 to 40, more preferably 1 to 30, still more preferably 1 to 20, and particularly preferably 1 to 10 or 1 to 5 (e.g., 1, 2, 3, 4 or 5).

In the protein (C1) or (C2), the extent of homology to the amino acid sequence of interest is preferably 92% or more, more preferably 95% or more, still more preferably 97% or more, and most preferably 98% or more or 99% or more. The homology % of the protein can be determined by algorithm blastp. More specifically, the homology % of the protein can be determined by using default settings of Scoring Parameters (Matrix: BLOSUM62; Gap Costs: Existence = 11 Extension = 1; Compositional Adjustments: Conditional compositional score matrix adjustment) in the algorithm blastp.

The position of the amino acid residue to which the mutation is introduced in the amino acid sequence is apparent to a person skilled in the art, but may be determined with further reference to the sequence alignment. Specifically, a person skilled in the art can (1) compare a plurality of amino acid sequences, (2) reveal a relatively conserved region and a region that is not relatively conserved, and (3) then predict a region capable of playing important roles for functions and a region incapable of playing important roles for functions from the relatively conserved region and the region that is not relatively conserved, respectively, for recognition of correlations between structures and functions. As such, a person skilled in the art can determine the position to which the mutation should be introduced in the amino acid sequence by utilizing the sequence alignment, as well as the position of the amino acid residue to which the mutation should be introduced in the amino acid sequence by combination use of known secondary and tertiary structure information.

When the amino acid residue is mutated by substitution, the substitution of the amino acid residue may be a conservative substitution. The term "conservative substitution" used in this description refers to replacing a certain amino acid residue with an amino acid residue having an analogous side chain. Families of the amino acid residues with analogous side chains are known in the relevant field. Examples of such families include amino acids with basic side chains (e.g., lysine, arginine and histidine), amino acids with acidic side chains (e.g., aspartic acid and glutamic acid), amino acids with uncharged polar side chains (e.g., glycine, asparagine, glutamine, serine, threonine, tyrosine and cysteine), amino acids with non-polar side chains (e.g., alanine, valine, leucine, isoleucine, proline, phenylalanine, methionine and tryptophan), amino acids with β-branched side chains (e.g., threonine, valine and isoleucine), amino acids with aromatic side chains (e.g., tyrosine, phenylalanine, tryptophan and histidine), amino acids with hydroxy group (e.g., alcoholic and phenolic)-containing side chains (e.g., serine, threonine and tyrosine), and amino acids with sulfur-containing side chains (e.g., cysteine and methionine). Preferably, the conservative substitutions of amino acids may be substitution between aspartic acid and glutamic acid, substitution among arginine, lysine and histidine, substitution between tryptophan and phenylalanine, substitution between phenylalanine and valine, substitution among leucine, isoleucine and alanine, and substitution between glycine and alanine.

In a specific embodiment, ferritin used in the present invention may be (1) naturally occurring ferritin, or (2) ferritin including the genetically modified ferritin monomers with any of the following modifications (a) to (c) :
(a) the ferritin monomer with the functional peptide inserted into the flexible linker region between second and third α-helices counted from the N-terminus of the ferritin monomer comprising six α-helices;
(b) the ferritin monomer with the functional peptide added to the N-terminus; or
(c) the ferritin monomer with the functional peptide added to the C-terminus.

As the functional peptide, a peptide that enables addition of any function to a target protein can be used when fused with the target protein. Examples of such a peptide include a peptide capable of binding to a target material, a protease degrading peptide, a cell-permeable peptide and a stabilizing peptide.

The functional peptide may refer to one peptide with a desired function, or same type or different types of plural (e.g., several such as two, three, four) peptides with desired functions. When the functional peptide refers to the plural peptides as described above, the functional peptides can be inserted in any order and fused with the ferritin monomer. The fusion between the ferritin monomer and the functional peptide can be achieved through an amide bond(s). Such a fusion may be achieved by directly connecting the ferritin monomer with the functional peptide through the amide bond(s), or indirectly by the amide bond(s) mediated by one amino acid residue (e.g., methionine) or a peptide (peptide linker) comprising several (e.g., 2 to 20, preferably 2 to 10, more preferably 2, 3, 4 or 5) amino acid residues. Since various peptide linkers are known, such a peptide linker can be used in the present invention. Preferably, the functional peptide is a peptide comprising 20 or less (preferably 18 or less, more preferably 15 or less, still more preferably 12 or less, and particularly preferably 10 or less) amino acid residues.

When the peptide capable of binding to the target material is used as the functional peptide, examples of the target material include an organic substance and an inorganic substance (e.g., conductor, semiconductor and magnetic substance). More specifically, examples of such a target material include biological organic molecules, metal materials, silicon materials, carbon materials, materials (e.g., nickel, maltose and glutathione) capable of interacting with a tag for protein purification (e.g., histidine tags, maltose-binding protein tags, glutathione-S-transferase), labeling substances (e.g., radioactive substances, fluorescent substances and dyes), polymers (e.g., hydrophobic organic polymers or conductive polymers such as polymethylmethacrylate, polystyrene, polyethylene oxide and poly(L-lactic acid)).

Examples of biological organic molecules include proteins (e.g., oligopeptide or polypeptide), nucleic acids (e.g., DNA, RNA, nucleosides, nucleotides, oligonucleotides or polynucleotides), saccharides (e.g., monosaccharides, oligosaccharides or polysaccharides) and lipids. The biological organic molecule may also be a cell surface antigen (e.g., a cancer antigen, a heart disease marker, a diabetes marker, a neurological disease marker, an immune disease marker, an inflammatory marker, a hormone, an infectious disease marker). The biological organic molecule may also be a disease antigen (e.g., a cancer antigen, a heart disease marker, a diabetes marker, a neurological disease marker, an immune disease marker, an inflammatory marker, a hormone, an infectious disease marker). Various peptides have been reported as peptides capable of binding to such biological organic molecules. Several peptides have been reported as follows: for example, peptides capable of binding to protein (see, e.g., F. Danhier et al., Mol. Pharmaceutics, 2012, vol. 9, No. 11, p. 2961; C-H. Wu et al., Sci. Transl. Med., 2015, Vol. 7, No. 290, 290ra91; L. Vannucci et al., Int. J. Nanomedicine. 2012, Vol. 7, p. 1489; J. Cutrera et al., Mol. Ther. 2011, Vol 19 (8), p. 1468; R. Liu et al., Adv. Drug Deliv. Rev. 2017, Vol. 110-111, p. 13); peptides capable of binding to nucleic acid (see, e.g., R. Tan et al., Proc. Natl. Acad. Sci. USA, 1995, vol. 92, p. 5282; R. Tan et al., Cell, 1993, vol. 73, p 1031; R. Talanian et al., Biochemistry. 1992, Vol. 31, p. 6871); peptides capable of binding to saccharide (see, e.g., K. Oldenburg et al., Proc. Natl. Acad. Sci. USA, 1992, vol. 89, No. 12, p. 5393-5397; K. Yamamoto et al., J. Biochem., 1992, vol. 111, p. 436; A. Baimiev et al., Mol. Biol. (Moscow), 2005, vol. 39, No. 1, p. 90); and peptides capable of binding to lipid (see, e.g., O. Kruse et al., B Z. Naturforsch., 1995, Vol. 50c, p. 380; O. Silva et al., Sci. Rep., 2016, Vol. 6, 27128; A. Filoteo et al., J. Biol. Chem., 1992, vol. 267, No. 17, p. 11800).

Preferably, the peptide capable of binding to biological organic molecule may be the peptide capable of binding to protein. Examples of the peptide capable of binding to protein include RGD-containing peptides disclosed in Danhier et al., Mol. Pharmaceutics, 2012, vol. 9, No. 11, p. 2961, and modified sequence thereof (e.g., RGD (SEQ ID NO: 19), ACDCRGDCFCG (SEQ ID NO: 20), CDCRGDCFC (SEQ ID NO: 21), GRGDS (SEQ ID NO: 22), ASDRGDFSG (SEQ ID NO: 23)), and other integrin recognition sequences (e.g., EILDV (SEQ ID NO: 24) and REDV (SEQ ID NO: 25)), peptides disclosed in L. Vannucci et al., Int. J. Nanomedicine. 2012, vol. 7, p. 1489 (e.g., SYSMEHFRWGKP (SEQ ID NO: 26)), peptides disclosed in J. Cutrera et al., Mol. Ther. 2011, vol. 19, No. 8, p. 1468 (e.g., VNTANST (SEQ ID NO: 27)), peptides disclosed in R. Liu et al., Adv. Drug Deliv. Rev. 2017, vol. 110-111, p. 13 (e.g., DHLASLWWGTEL (SEQ ID NO: 28) and NYSKPTDRQYHF (SEQ ID NO: 29), IPLPPPSRPFFK (SEQ ID NO: 30), LMNPNNHPRTPR (SEQ ID NO: 31), CHHNLTHAC (SEQ ID NO: 32), CLHHYHGSC (SEQ ID NO: 33), CHHALTHAC (SEQ ID NO: 34), SPRPRHTLRLSL (SEQ ID NO: 35), TMGFTAPRFPHY (SEQ ID NO: 36), NGYEIEWYSWVTHGMY (SEQ ID NO: 37), FRSFESCLAKSH (SEQ ID NO: 38), YHWYGYTPQNVI (SEQ ID NO: 39), QHYNIVNTQSRV (SEQ ID NO: 40), QRHKPRE (SEQ ID NO: 41), HSQAAVP (SEQ ID NO: 42), AGNWTPI (SEQ ID NO: 43), PLLQATL (SEQ ID NO: 44), LSLITRL (SEQ ID NO: 45), CRGDCL (SEQ ID NO: 46), CRRETAWAC (SEQ ID NO: 47), RTDLDSLRTYTL (SEQ ID NO: 48), CTTHWGFTLC (SEQ ID NO: 49), APSPMIW (SEQ ID NO: 50), LQNAPRS (SEQ ID NO: 51), SWTLYTPSGQSK (SEQ ID NO: 52), SWELYYPLRANL (SEQ ID NO: 53), WQPDTAHHWATL (SEQ ID NO: 54), CSDSWHYWC (SEQ ID NO: 55), WHWLPNLRHYAS (SEQ ID NO: 56), WHTEILKSYPHE (SEQ ID NO: 57), LPAFFVTNQTQD (SEQ ID NO: 58), YNTNHVPLSPKY (SEQ ID NO: 59), YSAYPDSVPMMS (SEQ ID NO: 60), TNYLFSPNGPIA (SEQ ID NO: 61), CLSYYPSYC (SEQ ID NO: 62), CVGVLPSQDAIGIC (SEQ ID NO: 63), CEWKFDPGLGQARC (SEQ ID NO: 64), CDYMTDGRAASKIC (SEQ ID NO: 65), KCCYSL (SEQ ID NO: 66), MARSGL (SEQ ID NO: 14), MARAKE (SEQ ID NO: 67), MSRTMS (SEQ ID NO: 68), WTGWCLNPEESTWGFCTGSF (SEQ ID NO: 69), MCGVCLSAQRWT (SEQ ID NO: 70), SGLWWLGVDILG (SEQ ID NO: 71), NPGTCKDKWIECLLNG (SEQ ID NO: 72), ANTPCGPYTHDCPVKR (SEQ ID NO: 73), IVWHRWYAWSPASRI (SEQ ID NO: 74), CGLIIQKNEC (SEQ ID NO: 75), MQLPLAT (SEQ ID NO: 76), CRALLRGAPFHLAEC (SEQ ID NO: 77), IELLQAR (SEQ ID NO: 78), TLTYTWS (SEQ ID NO: 79), CVAYCIEHHCWTC (SEQ ID NO: 80), THENWPA (SEQ ID NO: 81), WHPWSYLWTQQA (SEQ ID NO: 82), VLWLKNR (SEQ ID NO: 83), CTVRTSADC (SEQ ID NO: 84), AAAPLAQPHMWA (SEQ ID NO: 85), SHSLLSS (SEQ ID NO: 86), ALWPPNLHAWVP (SEQ ID NO: 87), LTVSPWY (SEQ ID NO: 88), SSMDIVLRAPLM (SEQ ID NO: 89), FPMFNHWEQWPP (SEQ ID NO: 90), SYPIPDT (SEQ ID NO: 91), HTSDQTN (SEQ ID NO: 92), CLFMRLAWC (SEQ ID NO: 93), DMPGTVLP (SEQ ID NO: 94), DWRGDSMDS (SEQ ID NO: 95), VPTDTDYS (SEQ ID NO: 96), VEEGGYIAA (SEQ ID NO: 97), VTWTPQAWFQWV (SEQ ID NO: 98), AQYLNPS (SEQ ID NO: 99), CSSRTMHHC (SEQ ID NO: 100), CPLDIDFYC (SEQ ID NO: 101), CPIEDRPMC (SEQ ID NO: 102), RGDLATLRQLAQEDGVVG (SEQ ID NO: 103), SPRGDLAVLGHK (SEQ ID NO: 104), SPRGDLAVLGHKY (SEQ ID NO: 105), CQQSNRGDRKRC (SEQ ID NO: 106), CMGNKCRSAKRP (SEQ ID NO: 107), CGEMGWVRC (SEQ ID NO: 108), GFRFGALHEYNS (SEQ ID NO: 109), CTLPHLKMC (SEQ ID NO: 110), ASGALSPSRLDT (SEQ ID NO: 111), SWDIAWPPLKVP (SEQ ID NO: 112), CTVALPGGYVRVC (SEQ ID NO: 113), ETAPLSTMLSPY (SEQ ID NO: 114), GIRLRG (SEQ ID NO: 115), CPGPEGAGC (SEQ ID NO: 116), CGRRAGGSC (SEQ ID NO: 117), CRGRRST (SEQ ID NO: 118), CNGRCVSGCAGRC (SEQ ID NO: 119), CGNKRTRGC (SEQ ID NO: 120), HVGGSSV (SEQ ID NO: 121), RGDGSSV (SEQ ID NO: 122), SWKLPPS (SEQ ID NO: 123), CRGDKRGPDC (SEQ ID NO: 124), GGKRPAR (SEQ ID NO: 125), RIGRPLR (SEQ ID NO: 126), CGFYWLRSC (SEQ ID NO: 127), RPARPAR (SEQ ID NO: 128), TLTYTWS (SEQ ID NO: 129), SSQPFWS (SEQ ID NO: 130), YRCTLNSPFFWEDMTHEC (SEQ ID NO: 131), KTLLPTP (SEQ ID NO: 132), KELCELDSLLRI (SEQ ID NO: 133), IRELYSYDDDFG (SEQ ID NO: 134), NVVRQ (SEQ ID NO: 135), VECYLIRDNLCIY (SEQ ID NO: 136), CGGRRLGGC (SEQ ID NO: 137), WFCSWYGGDTCVQ (SEQ ID NO: 138), NQQLIEEIIQILHKIFEIL (SEQ ID NO: 139), KMVIYWKAG (SEQ ID NO: 140), LNIVSVNGRH (SEQ ID NO: 141), QMARIPKRLARH (SEQ ID NO: 142) and QDGRMGF (SEQ ID NO: 143)), and mutant peptides thereof (e.g., mutation such as conservative substitutions of one, two, three, four or five amino acid residues), and peptides with one or more such amino acid sequences.

Preferably, the peptide capable of binding to biological organic molecule may be the peptide capable of binding to nucleic acid. Examples of the peptide capable of binding to nucleic acid include peptides disclosed in R. Tan et al., Proc. Natl. Acad. Sci. USA, 1995, vol. 92, p. 5282 and partial peptides thereof (e.g., TRQARR (SEQ ID NO: 17), TRQARRN (SEQ ID NO: 144), TRQARRNRRRRWRERQR (SEQ ID NO: 145), TRRQRTRRARRNR (SEQ ID NO: 146), NAKTRRHERRRKLAIER (SEQ ID NO: 147), MDAQTRRRERRAEKQAQWKAA (SEQ ID NO: 148), and RKKRRQRRR (SEQ ID NO: 149)), peptides disclosed in R. Tan et al., Cell, 1993, vol. 73, p. 1031 (e.g., TRQARRNRRRRWRERQR (SEQ ID NO: 150)), peptides disclosed in Talanian et al., Biochemistry. 1992, vol. 31, p. 6871 (e.g., KRARNTEAARRSRARK (SEQ ID NO: 151)), and mutant peptides thereof (e.g., mutation such as conservative substitutions of one, two, three, four or five amino acid residues), and peptides with one or more such amino acid sequences.

Preferably, the peptide capable of binding to biological organic molecule may be the peptide capable of binding to saccharide. Examples of the peptide capable of binding to saccharide include peptides disclosed in K. Oldenburg et al., Proc. Natl. Acad. Sci. USA, 1992, vol. 89, No12, p. 5393-5397 (e.g., DVFYPYPYASGS (SEQ ID NO: 152) and RVWYPYGSYLTASGS (SEQ ID NO: 153)), peptides disclosed in K. Yamamoto et al., J. Biochem., 1992, vol. 111, p. 436 (e.g., DTWPNTEWS (SEQ ID NO: 154), DSYHNIW (SEQ ID NO: 155), DTYFGKAYNPW (SEQ ID NO: 156) and DTIGSPVNFW (SEQ ID NO: 157)), peptides disclosed in A. Baimiev et al., Mol. Biol. (Moscow), 2005, vol. 39, No. 1, p. 90 (TYCNPGWDPRDR (SEQ ID NO: 158) and TFYNEEWDLVIKDEH (SEQ ID NO: 159)), and mutant peptides thereof (e.g., mutation such as conservative substitutions of one, two, three, four or five amino acid residues), and peptides with one or more such amino acid sequences.

Preferably, the peptide capable of binding to biological organic molecule may be the peptide capable of binding to lipid. Examples of the peptide capable of binding to lipid include peptides disclosed in O. Kruse et al., Z. Naturforsch., 1995, vol. 50c, p. 380 (e.g., MTLILELVVI (SEQ ID NO: 160), MTSILEREQR (SEQ ID NO: 161) and MTTILQQRES (SEQ ID NO: 162)), peptides disclosed in O. Silva et al., Sci. Rep., 2016, Vol. 6, 27128 (e.g., VFQFLGKIIHHVGNFVHGFSHVF (SEQ ID NO: 163)), peptides disclosed in A. Filoteo et al., J. Biol. Chem., 1992, vol. 267 (17), p. 11800, (e.g., KKAVKVPKKEKSVLQGKLTRLAVQI (SEQ ID NO: 164)), and mutant peptides thereof (e.g., mutation such as conservative substitutions of one, two, three, four or five amino acid residues), and peptides with one or more such amino acid sequences.

Examples of the metal material include metals and metal compounds. Examples of the metals include titanium, gold, chromium, zinc, lead, manganese, calcium, copper, calcium, germanium, aluminum, gallium, cadmium, iron, cobalt, silver, platinum, palladium, hafnium, and tellurium. Examples of the metal compounds include oxides, sulfides, carbonates, arsenides, chlorides, fluorides, iodides and intermetallic compounds of such metals. Various peptides capable of binding to such metal materials have been reported (e.g., WO2005/010031; WO2012/086647; K. Sano et al., Langmuir, 2004, vol. 21, p. 3090; S. Brown, Nat. Biotechnol., 1997, Vol. 15, p. 269; K. Kjaergaard et al., Appl. Environ. Microbiol., 2000, vol. 66. p. 10; Umetsu et al., Adv. Mater., 17, 2571-2575 (2005); M. B. Dickerson et al., Chem. Commun., 2004, Vol. 15. p. 1776; C. E. Flynn et al., J. Mater. Chem., 2003, vol. 13. p. 2414). In the present invention, such various peptides can be used.

Preferably, the peptide capable of binding to the metal material may refer to peptides capable of binding to titanium materials such as titanium or titanium compounds (e.g., titanium oxide), and peptides capable of binding to gold materials such as gold or gold compounds. Examples of the peptide capable of binding to the titanium material include peptides that are described in Examples and disclosed in WO2006/126595 (e.g., RKLPDA (SEQ ID NO: 165), peptides disclosed in M. J. Pender et al., Nano Lett., 2006, Vol. 6, No. 1, p. 40-44 (e.g., SSKKSGSYSGSKGSKRRIL (SEQ ID NO: 166)), peptides disclosed in I. Inoue et al., J. Biosci. Bioeng., 2006, vol. 122, No. 5, p. 528 (e.g., AYPQKFNNNFMS (SEQ ID NO: 167)), peptides disclosed in WO2006/126595 (e.g., RKLPDAPGMHTW (SEQ ID NO: 168) and RALPDA (SEQ ID NO: 169)), and mutant peptides thereof (e.g., mutation such as conservative substitutions of one, two, three, four or five amino acid residues), and peptides with one or more such amino acid sequences. Examples of the peptide capable of binding to the gold material include peptides that are described in Examples and disclosed in S. Brown, Nat. Biotechnol. 1997, vol. 15, p. 269 (e.g., MHGKTQATSGTIQS (SEQ ID NO: 170)), peptides disclosed in J. Kim et al., Acta Biomater., 2010, Vol. 6, No. 7, p. 2681 (e.g., TGTSVLIATPYV (SEQ ID NO: 171) and TGTSVLIATPGV (SEQ ID NO: 172)), peptides disclosed in K. Nam et. al., Science, 2006, vol. 312, No. 5775, p. 885 (e.g., LKAHLPPSRLPS (SEQ ID NO: 173)), and mutant peptides thereof (e.g., mutation such as conservative substitutions of one, two, three, four or five amino acid residues), and peptides with one or more such amino acid sequences.

Examples of the silicon material include silicon or silicon compounds. Examples of the silicon compound include silicon oxides (e.g., silicon monoxide (SiO), silicon dioxide (SiO₂)), silicon carbide (SiC), silane (SiH₄) and silicone rubber. Various peptides have been reported as peptides capable of binding to such a silicon material (For example, WO2006/126595; WO2006/126595; M. J. Pender et al., Nano Lett., 2006, vol. 6, No. 1, p. 40-44). Therefore, such a variety of peptides can be used in the present invention.

Preferably, the peptide capable of binding to the silicon material may be the peptide capable of binding to silicon or silicon compound (e.g., silicon oxide). Examples of such a peptide include peptides disclosed in WO2006/126595 (e.g., RKLPDA (SEQ ID NO: 165)), peptides disclosed in M. J. Pender et al., Nano Lett., 2006, vol. 6, No. 1, p. 40-44 (e.g., SSKKSGSYSGSKGSKRRIL (SEQ ID NO: 174)), peptides disclosed in WO2006/126595 (e.g., MSPHPHPRHHHT (SEQ ID NO: 175), TGRRRRLSCRLL (SEQ ID NO: 176) and KPSHHHHHTGAN (SEQ ID NO: 177)), and mutant peptides thereof (e.g., mutation such as conservative substitutions of one, two, three, four or five amino acid residues), and peptides with one or more such amino acid sequences.

Examples of the carbon material include carbon nanomaterials (e.g., carbon nanotube (CNT), carbon nanohorn (CNH)), fullerene (C60), graphene sheet and graphite. Various peptides have been reported as peptides capable of binding to such a carbon material (For example, Japanese Patent Application Laid-open No. 2004-121154; Japanese Patent Application Laid-open No. 2004-121154; and M. J. Pender et al., Nano Lett., 2006, vol. 6, No. 1, p. 40-44). Therefore, such a variety of peptides can be used in the present invention.

Preferably, the peptide capable of binding to the carbon material may be a peptide capable of binding to a carbon nanomaterial such as a carbon nanotube (CNT) or a carbon nanohorn (CNH). Examples of such peptides include peptides that are described below in Examples and disclosed in Japanese Patent Application Laid-open No. 2004-121154 (e.g., DYFSSPYYEQLF (SEQ ID NO: 178)), peptides disclosed in M. J. Pender et al., Nano Lett., 2006, vol. 6, No. 1, p. 40-44 (HSSYWYAFNNKT (SEQ ID NO: 179)), peptides disclosed in Japanese Patent Application Laid-open No. 2004-121154 (e.g., YDPFHII (SEQ ID NO: 180)), and mutant peptides thereof (e.g., mutation such as conservative substitutions of one, two, three, four or five amino acid residues), and peptides with one or more such amino acid sequences.

When a protease-degrading peptide is used as the functional peptide, examples of the protease include cysteine protease such as caspase and cathepsin (D. McIIwain1 et al., Cold Spring Harb Perspect Biol., 2013, vol. 5, a008656; V. Stoka et al., IUBMB Life. 2005, vol. 57, No. 4-5p. 347), collagenase (G. Lee et al., Eur J Pharm Biopharm., 2007, vol. 67, No. 3, p. 646), thrombin and Xa factor (R. Jenny et al., Protein Expr. Purif., 2003, vol. 31, p. 1; H. Xu et al., J. Virol., 2010, vol. 84, No. 2, p. 1076), and a virus-derived protease (C. Byrd et al., Drug Dev. Res., 2006, vol. 67, p. 501).

Examples of the protease-degrading peptide include peptides disclosed in E. Lee et al., Adv. Funct. Mater., 2015, vol. 25, p. 1279 (e.g., GRRGKGG (SEQ ID NO: 181)), G. Lee et al., Eur J Pharm Biopharm., 2007, vol. 67, No. 3, p. 646 (e.g., GPLGV (SEQ ID NO: 182) and GPLGVRG (SEQ ID NO: 183)), peptides disclosed in Y. Kang et al., Biomacromolecules, 2012, vol. 13, No. 12, p. 4057 (e.g., GGLVPRGSGAS (SEQ ID NO: 184)), peptides disclosed in R. Talanian et al., J. Biol. Chem., 1997, vol. 272, p. 9677 (e.g., YEVDGW (SEQ ID NO: 185), LEVDGW (SEQ ID NO: 186), VDQMDGW (SEQ ID NO: 187), VDVADGW (SEQ ID NO: 188), VQVDGW (SEQ ID NO: 189) and VDQVDGW (SEQ ID NO: 190)), peptides disclosed in Jenny et al., Protein Expr. Purif., 2003, vol. 31, p. 1 (e.g., ELSLSRLRDSA (SEQ ID NO: 191), ELSLSRLR (SEQ ID NO: 192), DNYTRLRK (SEQ ID NO: 193), YTRLRKQM (SEQ ID NO: 194), APSGRVSM (SEQ ID NO: 195), VSMIKNLQ (SEQ ID NO: 196), RIRPKLKW (SEQ ID NO: 197), NFFWKTFT (SEQ ID NO: 198), KMYPRGNH (SEQ ID NO: 199), QTYPRTNT (SEQ ID NO: 200), GVYARVTA (SEQ ID NO: 201), SGLSRIVN (SEQ ID NO: 202), NSRVA (SEQ ID NO: 203), QVRLG (SEQ ID NO: 204), MKSRNL (SEQ ID NO: 205), RCKPVN (SEQ ID NO: 206) and SSKYPN (SEQ ID NO: 207)), peptides disclosed in H. Xu et al., J. Virol., 2010, vol. 84, No. 2, p. 1076 (e.g., LVPRGS (SEQ ID NO: 208)), and mutant peptides thereof (e.g., mutation such as conservative substitutions of one, two, three, four or five amino acid residues), and peptides with one or more such amino acid sequences.

When a stabilizing peptide is used as the functional peptide, examples of the stabilizing peptide include peptide disclosed in X. Meng et al., Nanoscale, 2011, vol. 3, No. 3, p. 977 (e.g., CCALNN (SEQ ID NO: 209)), peptides disclosed in E. Falvo et al., Biomacromolecules, 2016, vol. 17, No. 2, p. 514 (e.g., PAS (SEQ ID NO: 210)), and mutant peptides thereof (e.g., mutation such as conservative substitutions of one, two, three, four or five amino acid residues), and peptides with one or more such amino acid sequences.

When a cell-permeable peptide is used as the functional peptide, examples of the cell-permeable peptide include peptides disclosed in Z. Guo et al., Biomed. Rep., 2016, vol. 4, No. 5, p. 528 (e.g., GRKKRRQRRRPPQ (SEQ ID NO: 211), RQIKIWFQNRRMKWKK (SEQ ID NO: 212), CGYGPKKKRKVGG (SEQ ID NO: 213), RRRRRRRR (SEQ ID NO: 214), KKKKKKKK (SEQ ID NO: 215), GLAFLGFLGAAGSTM (SEQ ID NO: 216), GAWSQPKKKRKV (SEQ ID NO: 217), LLIILRRRIRKQAHAHSK (SEQ ID NO: 218), MVRRFLVTL (SEQ ID NO: 219), RIRRACGPPRVRV (SEQ ID NO: 220), MVKSKIGSWILVLFV (SEQ ID NO: 221), SDVGLCKKRP (SEQ ID NO: 222), NAATATRGRSAASRPTQR (SEQ ID NO: 223), PRAPARSASRPRRPVQ (SEQ ID NO: 224), DPKGDPKGVTVT (SEQ ID NO: 225), VTVTVTGKGDPKPD (SEQ ID NO: 226), KLALKLALK (SEQ ID NO: 227), ALKAALKLA (SEQ ID NO: 228), GWTLNSAGYLLG (SEQ ID NO: 229), KINLKALAALAKKIL (SEQ ID NO: 230), RLSGMNEVLSFRW (SEQ ID NO: 231), SDLWEMMMVSLACQY (SEQ ID NO: 232) and PIEVCMYREP (SEQ ID NO: 233)), and mutant peptides thereof (e.g., mutation such as conservative substitutions of one, two, three, four or five amino acid residues), and peptides with one or more such amino acid sequences.

The functional peptide is preferably the peptide capable of binding to the target material. A preferred example of the peptide capable of binding to the target material is a peptide capable of binding to an organic substance. The peptide capable of binding to an organic substance is preferably the peptide capable of binding to biological organic molecule, and more preferably the peptide capable of binding to protein.

Ferritin can be obtained by utilizing a host cell that contains polynucleotides encoding ferritin and allows production of ferritin therein. Examples of such a host cell include cells derived from animals, insects, fishes or plants, and microorganisms. The animals are preferably mammals or avian species (e.g., chicken), and more preferably mammals. Examples of the mammals include primates (e.g., humans, monkeys, and chimpanzees), rodents (e.g., mice, rats, hamsters, guinea pigs, and rabbits) and livestock and working mammals (e.g., cattle, pigs, sheep, goats, and horses).

In a preferred embodiment, from the viewpoint of clinical application, the host cell may refer to human cells or cells (e.g., Chinese hamster ovary (CHO) cells, and human fetal kidney-derived HEK 293 cells) used for production of a human protein.

In another preferred embodiment, from the viewpoint of mass production of ferritin and the like, the host cell may be a microorganism. Examples of the microorganism include bacteria and fungi. As the bacteria, any bacteria used as the host cells can be used. Examples of the bacterium include bacteria belonging to Bacillus (e.g., Bacillus subtilis), Corynebacterium (e.g., Corynebacterium glutamicum), Escherichia (e.g., Escherichia coli) and Pantoea (e.g., Pantoea ananatis). As the fungi, any fungi used as the host cells can be used. Examples of the fungus include fungi belonging to Saccharomyces (e.g., Saccharomyces cerevisiae) and Schizosaccharomyces (e.g., Schizosaccharomyces pombe). Alternatively, filamentous fungi may be used as the microorganism. Examples of the filamentous fungi include fungi belonging to Acremonium/Talaromyces, Trichoderma, Aspergillus, Neurospora, Fusarium, Chrysosporium, Humicola, Emericella and Hypocrea.

Any organic compound can be used as the organic compound. Examples of such organic compounds include low molecular weight compounds, saccharide compounds, peptide compounds, nucleic acid compounds (e.g., DNA, RNA and artificial nucleic acids), but low molecular weight compounds are preferred.

The term "low molecular weight compound" refers to an organic compound having a molecular weight of 100 to 1000. The molecular weight of the organic compound that refers to the low molecular weight compound may be 150 or more, 200 or more, 250 or more, or 300 or more. The molecular weight may also be 950 or less, 900 or less, 850 or less, or 800 or less. More specifically, the molecular weight may be 150 to 950, 200 to 900, 250 to 850, or 300 to 800.

Examples of the low molecular weight compound include medicines, vitamins, labeling substances, amino acids, oligopeptides, nucleosides, nucleotides, oligonucleotides, monosaccharides, lipids, fatty acids, and metabolites thereof.

In a specific embodiment, the medicine used in the present invention may be an anthracycline substance, a histamine H2 receptor antagonist, a piguanide substance, an ATP-sensitive potassium channel opening agent, a β2 adrenergic receptor agonist, an imidazoline substance, a vitamin, or a labeling substance.

Examples of the anthracycline substance include doxorubicin, daunorubicin, epirubicin, idarubicin, valrubicin and mitoxantrone. Among them, doxorubicin is preferred.

Examples of the histamine H2 receptor antagonist include famotidine, cimetidine, ranitidine, nizatidine, roxatidine acetate, and lafutidine. Among them, famotidine is preferred.

Examples of the piguanide substance include metformin, buformin, phenformin, proguanil, chloroproguanil, chlorhexidine and alexidine. Among them, metformin is preferred.

Examples of the ATP-sensitive potassium channel opening agent include minoxidil, nicorandil, pinacidil, diazoxide, cromakalim, levcromakalim and lemakalim. Among them, minoxidil is preferred.

Examples of the β2 adrenergic receptor agonist include terbutaline, salbutamol, levosalbutamol, pirbuterol, procaterol, metaproterenol, fenoterol, bitolterol, salmeterol, formoterol, vilanterol, bambuterol, clenbuterol and indacaterol. Among them, terbutaline is preferred.

Examples of the imidazoline substance include creatinine, oxymetazoline, clonidine, cibenzoline, tizanidine, tetrahydrozoline, naphazoline, phentolamine, brimonidine and moxonidine. Among them, creatinine is preferred.

Examples of the vitamin include vitamin B1 (e.g., thiamine), vitamin B2 (e.g., riboflavin), vitamin B3 (e.g., niacin and nicotinamide), vitamin B5 (e.g., pantothenic acid), vitamin B6 (e.g., pyridoxal, pyridoxamine and pyridoxine), vitamin B7 (e.g., biotin), vitamin B9 (e.g., folate), vitamin B12 (e.g., cyanocobalamin and hydroxocobalamin), vitamin C (e.g., ascorbic acid), vitamin A (e.g., retinol, β-carotene, α-carotene and β-cryptoxanthin), vitamin D (e.g., ergocalciferol, cholecalciferol), vitamin E (e.g., tocopherol and tocotrienol), vitamin K (e.g., phylloquinone and menaquinone). Among them, vitamin B1, vitamin B2 and vitamin B3 are preferred.

Examples of the labeling substance include rhodamine (e.g., rhodamine B, 6G, 6GP, 3GO and 123), fluorescent substances such as uranine, pigments and dyes such as Congo red, and luminescent materials. Among them, rhodamine B, uranine, or Congo red is preferred.

Examples of the amino acid include α-amino acids (e.g., alanine, asparagine, cysteine, glutamine, isoleucine, leucine, methionine, phenylalanine, proline, serine, threonine, tryptophan, tyrosine, valine, aspartic acid, glutamic acid, arginine, histidine, lysine and glycine), β-amino acids (e.g., β-alanine and pantothenic acid), γ-amino acids (e.g., γ-aminobutyric acid). The amino acid may have L form or D form.

The pKa of the organic compound may be 2 or more and 13 or less. When the pKa of the organic compound is 2 or more and 13 or less, the organic compound can be encapsulated more efficiently in ferritin by using the buffer with an appropriate pH corresponding to the pKa. More specifically, when the pKa of the organic compound is 2 or more and less than 7 (preferably 3 or more and less than 6), the buffer preferably has a pH of 3 or more and less than 7 (preferably 3 or more and less than 6). When the pKa of the organic compound is 7 or more and 13 or less, the buffer preferably has a pH of 6 or more and less than 13 (preferably 6 or more and less than 12, more preferably 6 or more and less than 11, and still more preferably 6 or more and less than 10).

The pKa of the organic compound can be determined by a neutralization titration method (Kagaku Binran Kisohen I and II (Handbook of Chemistry, basic I and II) (Ver. 4) edited by The Chemical Society of Japan, Maruzen Publishing, 1993). In the neutralization titration method, 0.1 mol/L of is prepared for each molecule, then 0.1 mol/L of sodium hydroxide aqueous solution or hydrochloric acid solution is titrated to the solution at 25°C, and then pH is measured for the determination of pKa. When the organic compound has a single pKa value, the value is employed as the pKa of the organic compound. When the organic compound has plural (for example, 2 to 6, preferably 2 to 5, more preferably 2 to 4, still more preferably 2 or 3) pKa values, an average value thereof is employed as the pKa of the organic compound.

Preferably, the organic compound may have a positively charged portion and/or a potentially positively charged portion.

In one embodiment, the organic compound has the positively charged portion. The positively charged portion refers to a positively charged group in a solid state. Examples of the positively charged portion include a cationic nitrogen-containing group and a cationic phosphorus-containing group.

The cationic nitrogen-containing group refers to a group having a positively charged nitrogen atom. Examples of the cationic nitrogen-containing group include an ammonium group (e.g., primary, secondary, tertiary or quaternary), guanidinium group, imidazolium group, an oxazolium group, thiazolium group, oxadiazolium group, triazolium group, pyrrolidinium group, pyridinium group, piperidinium group, pyrazolium group, pyrimidinium group, pyrazinium group and triazinium group.

The cationic phosphorus-containing group refers to a group having a positively charged phosphorus atom. Examples of the cationic phosphorus-containing group include a phosphonium group (e.g., primary, secondary, tertiary or quaternary).

In another embodiment, the organic compound has the potentially positively charged portion. The potentially positively charged portion refers to a group that is not positively charged in a solid state but functions as an acceptor of a hydrogen atom so as to be capable of being positively charged depending on the pH of the aqueous solution when dissolved in the aqueous solution. Examples of the potentially positively charged portion include potentially positively charged nitrogen-containing groups and potentially positively charged phosphorus-containing groups. Examples of the potentially positively charged nitrogen-containing group include an amino group (e.g., primary, secondary or tertiary), guanidino group, nitro group, amide group, hydrazide group, imide group, azide group and diazo group. Examples of the potentially positively charged phosphorus-containing group include a phosphino group (e.g., primary, secondary or tertiary).

The organic compound may have a negatively charged portion (e.g., an oxide group in an amine oxide) or a potentially negatively charged portion (e.g., a sulfuric acid group) in addition to the positively charged portion or the potentially positively charged portion. When the organic compound has the negatively charged portion or the potentially negatively charged portion in addition to the positively charged portion or the potentially positively charged portion, it is preferable that total number of the positively charged portion(s) and/or the potentially positively charged portion(s) is larger than the total number of the negatively charged portion and/or the potentially negatively charged portion (that is, it is preferable that the organic compound is positively charged as a whole, or can be easily positively charged as a whole depending on a desired pH condition of the solution).

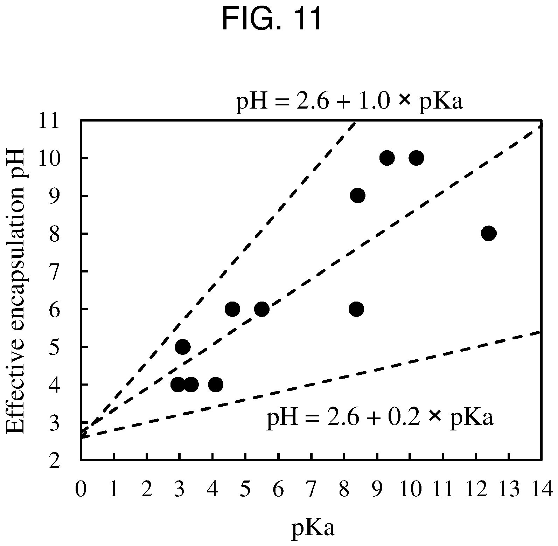

In a specific embodiment, the pKa of the organic compound and pH may be within a range defined by a correlation formula: pH = 2.6 + 0.6 pKa ± 0.4 pKa (FIG. 11). A coefficient of variation is preferably 0.3.

In another specific embodiment, the organic compound may have a pKa of 6 or more and 9 or less. In this case, the organic compound-encapsulating ferritin can be stably stored after the production of the organic compound-encapsulating ferritin by using the buffer (e.g., pH is 6 or more and 9 or less) used in the production method of the organic compound-encapsulating ferritin as it is. As such, the organic compound with the pKa of 6 or more and 9 or less can be suitably used in the present invention.

In the method of the present invention, a mixture of the organic compound and ferritin is obtained by mixing the organic compound with ferritin in the buffer at the beginning. The mixing can be carried out in any method. For example, the mixing can be carried out by dissolving the organic compound in a ferritin-containing buffer, or mixing an organic compound-containing buffer with the ferritin-containing buffer. The weight ratio of the organic compound to ferritin (organic compound / ferritin) in the mixing is not particularly limited as long as the organic compound can be encapsulated in ferritin, but may be, for example, 0.05 to 0.45. From the viewpoint of efficiently encapsulating the organic compound in ferritin, such a weight ratio may be 0.20 or more and 0.36 or less, for example.

The buffer used in the present invention has a pH of 3 or more and less than 13. With such a range of pH, it is possible to prevent the ferritin structure from being destructed (disassembled and denatured). The pH of the buffer may be 4, 5, 6 or 7, depending on factors such as pKa of the organic compound. The pH of the buffer may be also less than 13, less than 12, less than 11, less than 10, less than 9, less than 8, or less than 7, depending on factors such as pKa of the organic compound. A value that is measured by a glass electrode method at 25°C can be employed as the pH of the buffer.

The buffer can be selected depending on the aimed pH, as appropriate. Examples of the buffer that can be preferably used when the aimed pH is 3 or more and less than 7 include phosphate buffer, acetate buffer, citrate buffer, citrate phosphate buffer, borate buffer, tartrate buffer, phthalate buffer, glycine hydrochloride buffer, MES-NaOH buffer, MOPS buffer, HEPES-NaOH buffer, PIPES-NaOH buffer and bis Tris-HCl buffer. Examples of the buffer that can be preferably used when the aimed pH is 6 or more and less than 13 include Tris-HCl buffer, sodium carbonate buffer, MOPS buffer, HEPES-NaOH buffer, PIPES-NaOH buffer, glycine-NaOH buffer, CAPS-NaOH buffer, and potassium chloride-sodium hydroxide buffer.

In a specific embodiment, the pH of the buffer may be 6 or more and 9 or less. In this case, the organic compound-encapsulating ferritin can be stably stored after the production of the organic compound-encapsulating ferritin by using the buffer used in the production method of the organic compound-encapsulating ferritin as it is. As such, the buffer with the pH of 6 or more and 9 or less can be preferably used in the present invention.

In the method of the present invention, the mixture is then incubated in the buffer. The incubation can be carried out in any method. For example, the incubation is performed by leaving to stand or stirring the mixture in the buffer. The temperature of the incubation is not particularly limited as long as the organic compound can be encapsulated in ferritin, and is 10°C or more and 60°C or less, for example. The incubation temperature may be 15°C or more and 20°C or less. The incubation temperature may be 55°C or less, or 50°C or less as well.

Total time of the mixing and the incubation is not particularly limited as long as the organic compound can be encapsulated in ferritin, and may be, for example, 5 minutes or more, 10 minutes or more, 15 minutes or more, or 20 or more. Preferably, from the viewpoint of sufficiently encapsulating the organic compound in ferritin, the total time of mixing and incubation may be 30 minutes or more.

In the present invention, the number of organic compounds encapsulated in one molecule of ferritin is not particularly limited, and 10 or more and 200 or less molecules of the organic compound can be encapsulated in one molecule of ferritin. The number of the organic compounds encapsulated in one molecule of ferritin is preferably 20 or more molecules, more preferably 30 or more molecules, still more preferably 40 or more molecules, still further more preferably 50 or more molecules, particularly preferably 60 or more molecules, 70 or more molecules, 80 or more molecules, 90 or more molecules, 100 or more molecules, 110 or more molecules, 120 or more molecules, 130 or more molecules, 140 or more molecules, or 150 or more molecules. As well, the number of the organic compounds encapsulated in one molecule of ferritin is preferably 190 or less molecules, more preferably 180 or less molecules, still more preferably 170 or less molecules, still further more preferably 160 or less molecules, particularly preferably 150 or less molecules, 140 or less molecules, 130 or less molecules, 120 or less molecules, 110 or less molecules, 100 or less molecules, 90 or less molecules, 80 or less molecules, 70 or less molecules, 60 or less molecules, 50 or less molecules, 40 or less molecules, 30 or less molecules, 20 or less molecules, or 15 or less molecules.

In a specific embodiment, when the organic compound is the anthracycline substance, the number of anthracycline substance(s) encapsulated in one molecule of ferritin can be varied depending on ferritin that is either (a) the naturally occurring ferritin including the naturally occurring ferritin monomers or (b) the genetically modified ferritin including the genetically modified ferritin monomers.

For example, when the organic compound is the anthracycline substance and ferritin is the naturally occurring ferritin, the number of anthracycline substances encapsulated in one molecule of the naturally occurring ferritin may be 40 or more molecules and 200 or less molecules. The number of anthracycline substances encapsulated in one molecule of the naturally occurring ferritin may be preferably 50 or more molecules, more preferably 60 or more molecules, still more preferably 70 or more molecules, still further more preferably 80 or more molecules, particularly preferably 90 or more molecules. As well, the number of anthracycline substances encapsulated in one molecule of the naturally occurring ferritin may be preferably 170 or less molecules, more preferably 140 or less molecules.

When the organic compound is the anthracycline substance and ferritin is the genetically modified ferritin, the number of anthracycline substances encapsulated in one molecule of the genetically modified ferritin may be 100 or more molecules and 200 or less molecules. The number of anthracycline substances encapsulated in one molecule of the genetically modified ferritin may be preferably 110 or more molecules, more preferably 120 or more molecules, still more preferably 130 or more molecules, still further more preferably 140 or more molecules, particularly preferably 150 or more molecules. As well, the number of anthracycline substances encapsulated in one molecule of the genetically modified ferritin may be preferably 190 or less molecules, more preferably 180 or less molecules.

Alternatively, when the organic compound is the anthracycline substance, the number of anthracycline substances encapsulated in one molecule of ferritin can be defined, irrespective of the kinds of ferritin (a) the naturally occurring ferritin and (b) the genetically modified ferritin. In this case, the number of anthracycline substances encapsulated in one molecule of the ferritin may be 100 or more molecules and 200 or less molecules. The number of anthracycline substances encapsulated in one molecule of ferritin may be preferably 110 or more molecules, more preferably 120 or more molecules, still more preferably 130 or more molecules, still further more preferably 140 or more molecules, particularly preferably 150 or more molecules. As well, the number of anthracycline substances encapsulated in one molecule of ferritin may be preferably 190 or less molecules, more preferably 180 or less molecules.

In a specific embodiment, when the organic compound is the histamine H2 receptor antagonist, the number of the histamine H2 receptor antagonist encapsulated in one molecule of ferritin may be 10 or more molecules and 200 or less molecules. The number of the histamine H2 receptor antagonist encapsulated in one molecule of ferritin may be preferably 20 or more molecules, more preferably 30 or more molecules. As well, the number of histamine H2 receptor antagonist encapsulated in one molecule of ferritin may be preferably 150 or less molecules, more preferably 100 or less molecules, and still more preferably 50 or less molecules.

In a specific embodiment, when the organic compound is the piguanide substance, the number of the piguanide substances encapsulated in one molecule of ferritin may be 10 or more molecules and 200 or less molecules. The number of the piguanide substances encapsulated in one molecule of ferritin may be preferably 20 or more molecules, more preferably 30 or more molecules, still more preferably 40 or more molecules, still further more preferably 50 or more molecules, particularly preferably 60 or more molecules, or 70 or more molecules. As well, the number of the piguanide substances encapsulated in one molecule of ferritin may be preferably 150 or less molecules, more preferably 100 or less molecules.

In a specific embodiment, when the organic compound is the ATP-sensitive potassium channel opening agent, the number of the ATP-sensitive potassium channel opening agents encapsulated in one molecule of ferritin may be 10 or more molecules and 200 or less molecules. The number of the ATP-sensitive potassium channel opening agents encapsulated in one molecule of ferritin may be preferably 20 or more molecules, more preferably 30 or more molecules, still more preferably 40 or more molecules, still further more preferably 50 or more molecules, particularly preferably 60 or more molecules. As well, the number of the ATP-sensitive potassium channel opening agents encapsulated in one molecule of ferritin may be preferably 150 or less molecules, more preferably 100 or less molecules.

In a specific embodiment, when the organic compound is the β2 adrenergic receptor agonist, the number of the β2 adrenergic receptor agonist encapsulated in one molecule of ferritin may be 10 or more molecules and 200 or less molecules. The number of the β2 adrenergic receptor agonist encapsulated in one molecule of ferritin may be preferably 20 or more molecules, more preferably 30 or more molecules, still more preferably 40 or more molecules, still further more preferably 50 or more molecules, particularly preferably 60 or more molecules, 70 or more molecules, 80 or more molecules, 90 or more molecules, 100 or more molecules, 110 or more molecules, or 120 or more molecules. As well, the number of the β2 adrenergic receptor agonist encapsulated in one molecule of ferritin may be preferably 150 or less molecules.

In a specific embodiment, when the organic compound is the imidazoline substance, the number of the imidazoline substances encapsulated in one molecule of ferritin may be 10 or more molecules and 200 or less molecules. The number of the imidazoline substances encapsulated in one molecule of ferritin may be preferably 15 or more molecules, more preferably 20 or more molecules. As well, the number of the imidazoline substances encapsulated in one molecule of ferritin may be preferably 150 or less molecules, more preferably 100 or less molecules, and still more preferably 50 or less molecules.

In a specific embodiment, when the organic compound is vitamin, the number of vitamin encapsulated in one molecule of ferritin may be 10 or more molecules and 200 or less molecules. The number of vitamin encapsulated in one molecule of ferritin may be preferably 150 or less molecules, more preferably 100 or less molecules, still more preferably 50 or less molecules, still further more preferably 30 or less molecules, and particularly preferably 20 or less molecules.

In a specific embodiment, when the organic compound is the labeling substance, the number of the labeling substances encapsulated in one molecule of ferritin may be 10 or more molecules and 200 or less molecules. The number of the labeling substances encapsulated in one molecule of ferritin may be preferably 150 or less molecules, more preferably 100 or less molecules, still more preferably 50 or less molecules, still further more preferably 30 or less molecules, and particularly preferably 20 or less molecules.

The encapsulation of the organic compound into ferritin can be confirmed by measurement of absorption peaks of ferritin and the organic compound.

The measurement of absorption peaks of ferritin and the organic compound is described. First, ferritin in the buffer after the incubation is purified with a gel filtration column. Then, the absorption peak (absorbance at 280 nm) of ferritin and the absorption peak (e.g., absorbance at 480 nm of doxorubicin) of the organic compound are simultaneously measured for an eluted solution after the purification. When both of the absorption peaks are overlapped with each other at an elution position, it can be determined that the organic compound is encapsulated in ferritin. According to the method of the present invention, it is confirmed that the organic compound is not adsorbed on the surface of ferritin but encapsulate in the internal cavity of ferritin reproducibly, and therefore the organic compound can be considered to be encapsulated in ferritin by the peak overlap of absorption peaks at the elusion position.

Other methods may be employed for combination use, for the purpose of confirming the encapsulation of the organic compound into ferritin. Examples of such other methods include measurement of surface charge and/or size of ferritin.

The measurement of surface charge of ferritin is described. When the surface charge (zeta potential) of ferritin in the buffer after the incubation is equivalent to the surface charge of ferritin without the organic compound being encapsulated therein, it can be determined that the organic compound is not adsorbed on the surface of ferritin for forming the composite (that is, the organic compound is encapsulated in ferritin). The surface charge can be measured by an electrophoretic light scattering method. Preferably, the measurement by the electrophoretic light scattering method can be carried out by using Zetasizer Nano ZS (Malvern Panalytical Ltd.). The pKa of the organic compound preferably used for the measurement of the surface charge of ferritin is 1-4 (preferably 1-3) and 6-14 (preferably 7-14), for example. In the present invention, it is also preferable to use the organic compound with such a pKa value.

The measurement of the size of ferritin is described. In this measurement, the size of ferritin in the buffer after the incubation may be further confirmed to be comparable to the size (outside diameter of approximately 12 nm) of ferritin without the organic compound being encapsulated therein. The comparability in size can be confirmed by a dynamic light scattering method. Preferably, Zetasizer Nano ZS (Malvern Panalytical Ltd.) can be employed for the measurement by the dynamic light scattering method.

The number of molecules of the organic compound encapsulated into ferritin can be determined as follows: (1) separating the organic compound from ferritin in the buffer after the incubation (e.g., use of desalting column); (2) disassembling ferritin (e.g., pH adjustment to 2.3) and then releasing the organic compound from ferritin into the solution; (3) determining the concentration (weight / volume) of the organic compound in the solution using a calibration curve based on the absorbance; (4) determining the concentration (weight / volume) of ferritin in the solution using a standard protein quantification method (e.g., use of protein assay CBB solution with bovine albumin as a standard); (5) determining an efficiency of incorporating the organic compound into ferritin (weight of the organic compound with respect to the sum of weights of the organic compound and ferritin); (6) determining the concentration (mol / volume) of the organic compound in the solution from the concentration (weight / volume) of the organic compound in the solution using molecular weight of the organic compound; (7) determining the concentration (mol / volume) of ferritin in the solution from the concentration (weight / volume) of ferritin in the solution using molecular weight of ferritin; and (8) determining the number of the organic compound incorporated into ferritin (the number of mols of the organic compound per mole of ferritin).

The present invention also provides a specific organic compound-encapsulating ferritin. In the organic compound-encapsulating ferritin of the present invention, the organic compound is selected from the group consisting of the anthracycline substance, the histamine H2 receptor antagonist, the piguanide substance, the ATP-sensitive potassium channel opening agent, the β2 adrenergic receptor agonist, the imidazoline substance, vitamin, and the labeling substance, and
the number of molecules of the organic compound encapsulated into one molecule of ferritin is as follows:
(1) 40 or more and 200 or less molecules, when the organic compound is the anthracycline substance and ferritin is the naturally occurring ferritin;
(2) 100 or more and 200 or less molecules, when the organic compound is the anthracycline substance and ferritin is the genetically modified ferritin; or
(3) 10 or more and 200 or less molecules, when the organic compound is the histamine H2 receptor antagonist, the piguanide substance, the ATP-sensitive potassium channel opening agent, the β2 adrenergic receptor agonist, the imidazoline substance, the vitamin or the labeling, and when ferritin is the naturally occurring ferritin or the genetically modified ferritin.

Preferably, in the organic compound-encapsulating ferritin of the present invention, the organic compound may be selected from the group consisting of the anthracycline substance, the histamine H2 receptor antagonist, the piguanide substance, the ATP-sensitive potassium channel opening agent, and the β2 adrenergic receptor agonist.

Details of components such as the specific organic compound, ferritin, and the number of encapsulated molecules in the organic compound-encapsulating ferritin of the present invention are described above.

The present invention further provides a buffer containing the organic compound-encapsulating ferritin. Details of components such as the organic compound, ferritin, the buffer, and the number of encapsulated molecules in the buffer of the present invention are described above.

### EXAMPLES

Next, the present invention is described in detail with reference to Examples, but the present invention is not limited to the following Examples. All of reagents (small molecules) used below are organic compounds. A value measured by glass electrode method at 25°C was employed as pH of a buffer.

### <Example 1: Production of ferritin>

Total synthesis was carried out for a DNA encoding a human-derived ferritin H chain (FTH (SEQ ID NO: 1)) monomer. PCR was carried out using the synthesized DNA as a template as well as the following primers: 5'-GAAGGAGATATACATATGACGACCGCGTCCACCTCG-3' (SEQ ID NO: 3) and 5'-CTCGAATTCGGATCCTTAGCTTTCATTATCACTGTC-3' (SEQ ID NO: 4). PCR was carried out using pET20 (Merck KGaA) as a template as well as the following primers: 5'-TTTCATATGTATATCTCCTTCTTAAAGTTAAAC-3' (SEQ ID NO: 5) and 5'-TTTGGATCCGAATTCGAGCTCCGTCG-3' (SEQ ID NO: 6). The resulting PCR products were purified using Wizard DNA Clean-Up System (Promega Corporation), and then subjected to In-Fusion enzyme treatment at 50°C for 15 minutes using In-Fusion HD Cloning Kit (Takara Bio Inc.) to construct an expression plasmid (pET20-FTH) carrying a gene encoding FTH monomer.

Subsequently, Escherichia coli BL21 (DE3) into which the constructed pET20-FTH was introduced was cultured in 100 mL of an LB medium (including 10 g/L of Bactotyptone, 5 g/L of Bacto-yeast extract, 5 g/L NaCl and 100 mg/L of ampicillin) at 37°C for 24 hours using flasks. The resulting bacterial cells were sonicated for cell disruption, and then the resulting supernatant was heated at 60°C for 20 minutes. The supernatant obtained after the heating was injected into a HiPerp Q HP column (GE Healthcare Inc.) equilibrated with 50 mM Tris-HCl buffer (pH 8.0). Then, ferritin (referred to as FTH ferritin, hereinafter) formed of the FTH monomers was separated and purified by applying a concentration gradient of the salt from 0 mM to 500 mM NaCl in 50 mM Tris-HCl buffer (pH 8.0). The solvent of the solution containing the protein was replaced with 10 mM Tris-HCl buffer (pH 8.0) by centrifugal ultrafiltration using Vivaspin 20-100K (GE Healthcare Inc.). The resulting solution was injected into a HiPrep 26/60 Sephacryl S-300 HR column (GE Healthcare Inc.) equilibrated with 10 mM Tris-HCl buffer (pH 8.0) to separate and purify FTH ferritin by size. The solution containing FTH ferritin was concentrated by centrifugal ultrafiltration using Vivaspin 20-100K (GE Healthcare Inc.), and then concentration of the contained protein was determined using a protein assay CBB solution (Nacalai Tesque, Inc.) as well as bovine albumin as a standard. As a result, 1 mL solution containing 5 mg/mL of FTH ferritin was obtained.

### <Example 2: Investigation of pH condition>

pH condition was studied for establishment of a method (one-step process) for incorporating the small molecule into FTH ferritin without undergoing a disassembly-reassembly process.

FTH ferritin and Doxorubicin hydrochloride (DOX, CAS No. 25316-40-9, molecular weight 579.98) were mixed with 0.1 mL of 50 mM buffer (pH 3 to 9) to achieve final concentrations of 1 mg/mL and 0.1 mg/mL respectively, and then the resulting solution was left to stand at 25°C for 60 minutes. In this reaction, an acetate buffer was used for pH 3, 4, 5 and 6, while Tris-HCl buffer was used for pH 7, 8 and 9. After the standing, 0.5 mL of water was added to the resulting solution. After the resulting solution was centrifuged (at 15,000 rpm for one minute), the supernatant was injected into a desalting column PD-10 (Sephadex G-25 filled product, GE Healthcare Inc.) equilibrated with 10 mM Tris-HCl buffer (pH 8) to separate the protein from a reagent that was not encapsulated. Whole amount (3.5 mL) of the resulting solution was concentrated to 0.1 mL by centrifugal ultrafiltration using Vivaspin 20-100K (GE Healthcare Inc.), and then absorbances at 280 nm and 480 nm were measured using spectrophotometer (DU-800, Beckman Coulter Inc.). On the basis of the obtained absorbances, concentrations of DOX and FTH ferritin were determined with reference to a calibration curve that was obtained by absorbances at 280 nm and 480 nm measured for different concentrations of DOX and FTH ferritin. Incorporation efficiency of the reagent (weight of DOX with respect to total weight of DOX and FTH ferritin) was determined based on the concentrations.

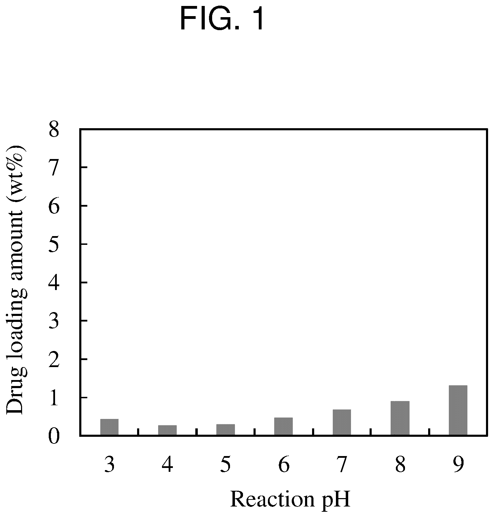

As a result, the incorporation efficiency of the reagent was 0.5% (wt) or less at pH 6 or less, suggesting that DOX was slightly incorporated (FIG. 1). Meanwhile, at pH 7 or more, the incorporation efficiency of the reagent was increased with pH, and the incorporation efficiency of the reagent was 1% (wt) or more at pH 9. The above results suggested the possibility of incorporating DOX into FTH ferritin at pH 7 or more.

### <Example 3: Investigation of temperature condition>

Temperature condition was studied for establishment of the method (one-step process) for incorporating the small molecule into FTH ferritin without undergoing a disassembly-reassembly process.

FTH ferritin and Doxorubicin hydrochloride (DOX, CAS No. 25316-40-9, molecular weight 579.98) were mixed with 0.1 mL of 50 mM Tris-HCl buffer (pH 9) to achieve final concentrations of 1 mg/mL and 0.1 mg/mL respectively, and then the resulting solution was left to stand for 60 minutes at different temperatures ranging from 10°C to 60°C. After the standing, 0.5 mL of water was added to the resulting solution. After the resulting solution was centrifuged (at 15,000 rpm for one minute), the supernatant was injected into the desalting column PD-10 (Sephadex G-25 filled product, GE Healthcare Inc.) equilibrated with 10 mM Tris-HCl buffer (pH 8) to separate the protein from a reagent that was not encapsulated. Whole amount (3.5 mL) of the resulting solution was concentrated to 0.1 mL by centrifugal ultrafiltration using Vivaspin 20-100K (GE Healthcare Inc.), and then absorbances at 280 nm and 480 nm were measured using spectrophotometer (DU-800, Beckman Coulter Inc.). On the basis of the obtained absorbances, concentrations of DOX and FTH ferritin were determined with reference to a calibration curve that was obtained by absorbances at 280 nm and 480 nm measured for different concentrations of DOX and FTH ferritin. Incorporation efficiency of the reagent (weight of DOX with respect to total weight of DOX and FTH ferritin) was determined based on the concentrations.

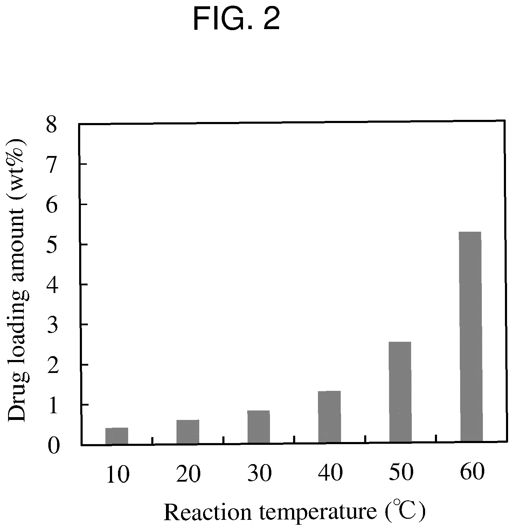

As a result, the incorporation efficiency of the reagent was increased with an increase in the reaction temperature at 20°C or more. The incorporation efficiency of the reagent was 5% (wt) or more at 60°C (FIG. 2). The above results suggested DOX was incorporated into FTH ferritin efficiently at room temperature or more.

### <Example 4: Investigation of reaction time>

Reaction time condition was studied for establishment of the method (one-step process) for incorporating the small molecule into FTH ferritin without undergoing the disassembly-reassembly process.

FTH ferritin and Doxorubicin hydrochloride (DOX, CAS No. 25316-40-9, molecular weight 579.98) were mixed with 0.5 mL of 50 mM Tris-HCl buffer (pH 9) to achieve final concentrations of 1 mg/mL and 0.1 mg/mL respectively, and then the resulting solution was left to stand at 60°C for different times ranging from 5 to 60 minutes. After the standing, 0.5 mL water was added to the resulting solution. After the resulting solution was centrifuged (at 15,000 rpm for one minute), the supernatant was injected into the desalting column PD-10 (Sephadex G-25 filled product, GE Healthcare Inc.) equilibrated with 10 mM Tris-HCl buffer (pH 8) to separate the protein from a reagent that was not encapsulated. Whole amount (3.5 mL) of the resulting solution was concentrated by centrifugal ultrafiltration using Vivaspin 20-100K (GE Healthcare Inc.). Glycine-HCl buffer (pH 2.3) was added to the resulting solution to achieve a final concentration of 100 mM, then a volume was controlled to 0.2 mL. Herein, at pH 2.3, FTH ferritin was disassembled so as to allow DOX incorporated in FTH ferritin to be released into the solution. The absorbance at 480 nm was measured using spectrophotometer (DU-800, Beckman Coulter Inc.) for the resulting solution in order to determine the concentration of DOX. The concentration of protein in the solution was determined using the protein assay CBB solution (Nacalai Tesque, Inc.) as well as bovine albumin as a standard. The incorporation efficiency of the reagent (weight of DOX with respect to total weight of DOX and FTH ferritin) was determined based on the concentrations.

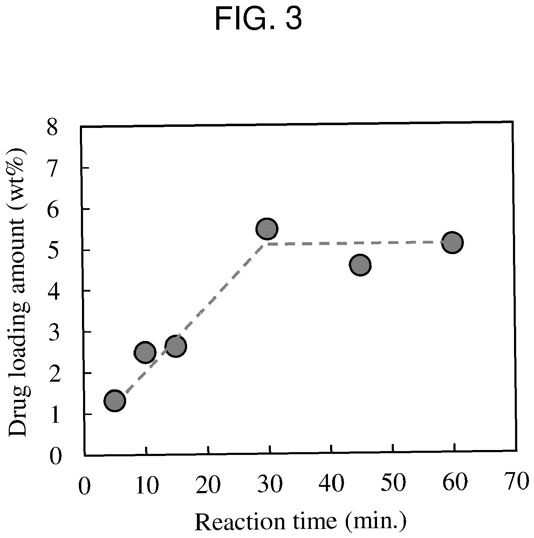

As a result, the amount of incorporated DOX was confirmed to be increased with the standing time until 30 minutes elapsed for the standing. DOX was incorporated at a rate of 1.7 mg-DOX/minute per gram of ferritin (FIG. 3). After 30 minutes elapsed, the incorporation efficiency of the reagent was not substantially changed. At that time, the maximum incorporation efficiency of the reagent was 5% (wt), and the number of molecules of DOX incorporated into one molecule of FTH ferritin was presumed to be 50 in average.

### <Example 5: Investigation of amount ratio of FTH ferritin to DOX in reaction solution>

Amount ratio was studied for establishment of the method (one-step process) for incorporating the small molecule into FTH ferritin without undergoing the disassembly-reassembly process.

Doxorubicin hydrochloride (DOX, CAS No. 25316-40-9, molecular weight 579.98) was mixed with 0.5 mL of 50 mM Tris-HCl buffer (pH 9) that contains FTH ferritin at a final concentration of 1 mg/mL, to achieve different final concentrations ranging from 0.05 mg/L to 0.5 mg/L, and then the resulting solutions were left to stand at 60°C for 60 minutes. After the standing, 0.5 mL water was added to the resulting solution. After the resulting solution was centrifuged (at 15,000 rpm for one minute), the supernatant was injected into the desalting column PD-10 (Sephadex G-25 filled product, GE Healthcare Inc.) equilibrated with 10 mM Tris-HCl buffer (pH 8) to separate the protein from a reagent that was not encapsulated. Whole amount (3.5 mL) of the resulting solution was concentrated by centrifugal ultrafiltration using Vivaspin 20-100K (GE Healthcare Inc.). Glycine-HCl buffer (pH 2.3) was added to the resulting solution to achieve a final concentration of 100 mM, then a volume was controlled to 0.5 mL. The absorbance at 480 nm was measured using spectrophotometer (DU-800, Beckman Coulter Inc.) for the resulting solution in order to determine the concentration of DOX. The concentration of protein in the solution was determined using the protein assay CBB solution (Nacalai Tesque, Inc.) as well as bovine albumin as a standard. The incorporation efficiency of the reagent (weight of DOX with respect to total weight of DOX and FTH ferritin) was determined based on the concentration.

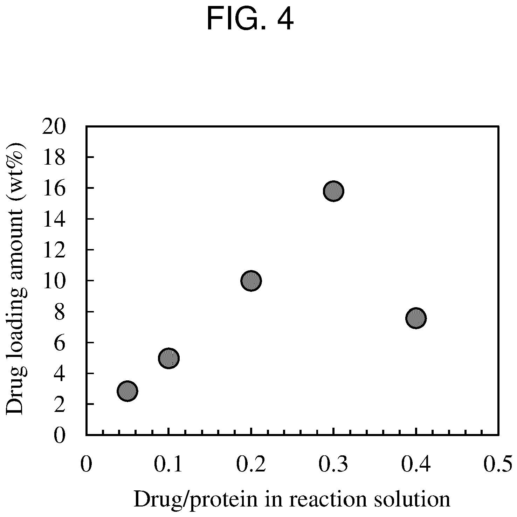

As a result, when a concentration ratio of FTH ferritin to DOX was 10:3 or less, the amount of incorporated DOX was confirmed to be increased with the concentration ratio. The incorporation efficiency of the reagent was 16% (wt) at the maximum (FIG. 4). At that time, the number of molecules of DOX incorporated into one molecule of FTH ferritin was presumed to be 165 in average. On the other hand, when the concentration ratio of FTH ferritin to DOX was 10:4, the incorporation amount was decreased. When the ratio was 10:5, the protein could not be collected possibly due to precipitation of DOX in a high concentration range that causes precipitation of FTH ferritin together with DOX.

According to calculation results based on data that was reported in the prior knowledge (Journal of Controlled Release 196 (2014) 184-196, Proc Natl Acad Sci USA. 2014 111 (41): 14900-5), the incorporation efficiency of the reagent into ferritin is 3% (wt) to 4% (wt) in a conventional method. Meanwhile, the methods in this Example enabled incorporating a larger amount of the reagent, up to 4 to 5-fold or more, than the conventional method. The incorporation efficiency of the reagent is 11% (wt) for Doxil (certification number: 21900AMX00001000) that is commercially available doxorubicin encapsulating liposome. The method in this Example enabled incorporating a larger amount of the reagent, up to 1.4-fold or more, than Doxil.

### <Example 6: Dispersibility of DOX-encapsulating FTH ferritin>

A small molecule-encapsulating FTH ferritin obtained by the one step process was confirmed to actually incorporate a small molecule therein.

DOX was mixed with 1 mL of 50 mM Tris-HCl buffer (pH 9) that contains FTH ferritin at a final concentration of 1 mg/mL, to achieve a final concentration of 0.1 mg/L, and then the resulting solutions were left to stand at 60°C for 60 minutes. After the standing and centrifuging (at 15,000 rpm for one minute), the supernatant was injected into the desalting column PD-10 (Sephadex G-25 filled product, GE Healthcare Inc.) equilibrated with 10 mM Tris-HCl buffer (pH 8) to separate the protein from a reagent that was not encapsulated. Whole amount (3.5 mL) of the resulting solution was concentrated by centrifugal ultrafiltration using Vivaspin 20-100K (GE Healthcare Inc.). The 10 mM Tris-HCl buffer (pH 8) that contains 5 mL of DOX-encapsulating FTH ferritin prepared in the same way, was mixed and then subjected to centrifugal ultrafiltration to achieve 1 mL volume. The resulting solution was injected into the HiPrep 26/60 Sephacryl S-300 HR column (GE Healthcare Inc.) equilibrated with 10 mM Tris-HCl buffer (pH 8.0) in order to measure the absorbances at 280 nm and 480 nm simultaneously while separating and purifying by size.

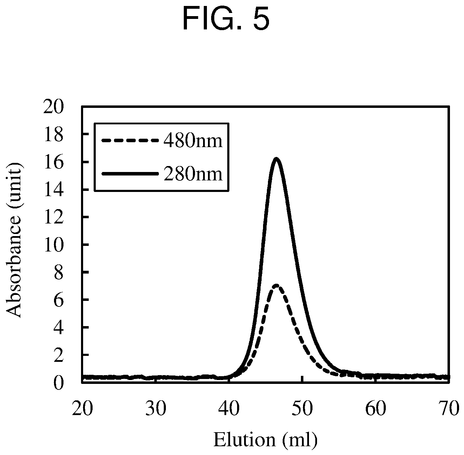

As a result, the absorption peak at 480 nm presumed to derive from DOX was observed at the same elusion position as that of FTH ferritin not containing DOX, demonstrating that DOX and FTH ferritin were securely combined with each other to form a composite inseparable by dilution washing using ultrafiltration or gel-filtration column (FIG. 5).

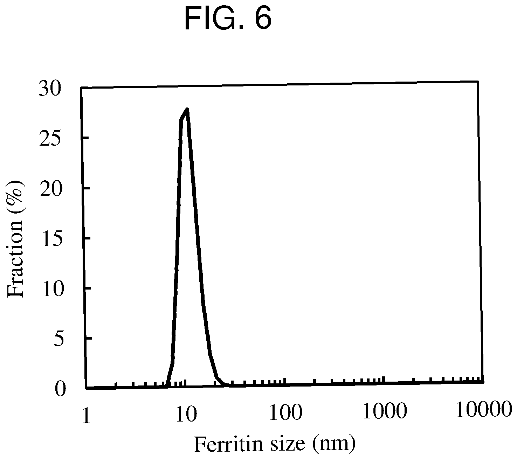

The dispersibility of the DOX-FTH ferritin composite in the solution was measure also with use of Zetasizer Nano ZS (Malvern Panalytical Ltd.), revealing that the DOX-FTH ferritin composite is an aqueous dispersive nanoparticle with a diameter of 12 nm comparable to that of wild-type FTH ferritin (FIG. 6). The measurement was carried out at 25°C using a ZEN0040 cell filled with 50 µL of the 10 mM Tris-HCl buffer (pH 8.0) that contains the DOX-FTH ferritin composite at a concentration of 1 mg/mL as the weight of FTH ferritin as well as the following settings: Material setting (Ri: 1.450, Absorption: 0.001), Dispersant setting (Temperature: 25°C, Viscosity: 0.8872 cP, RI: 1.330, Dielectric constant: 78.5), Mark-Houwink Parameters (A Parameter: 0.428, K Parameter: 7.67 x 10⁻⁵ cm²/s), and Measurement angle of 173°, in Backscatter mode.

### <Example 7: Surface charge of DOX-encapsulating FTH ferritin>

The encapsulation of the small molecule agent using the small molecule agent-encapsulating FTH ferritin obtained by the one-step process was confirmed by evaluation of surface charge.

DOX was mixed with 1 mL of 50 mM Tris-HCl buffer (pH 8) that contains FTH ferritin at a final concentration of 1 mg/mL, to achieve a final concentration of 0.3 mg/L, and then the resulting solution was left to stand at 40°C for 60 minutes. After the standing and the centrifuging (at 15,000 rpm for one minute), the supernatant was injected into the desalting column PD-10 (Sephadex G-25 filled product, GE Healthcare Inc.) equilibrated with 10 mM Tris-HCl buffer (pH 8) to separate the protein from a reagent that was not encapsulated. Whole amount (3.5 mL) of the resulting solution was concentrated by centrifugal ultrafiltration using Vivaspin 20-100K (GE Healthcare Inc.) in order to obtain the composite of DOX and FTH ferritin suspended in 1 mL of water.

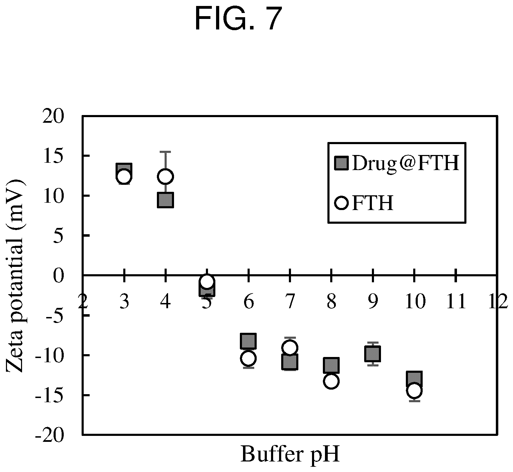

Subsequently, the DOX-FTH ferritin composite was suspended in phosphate buffer (pH 3, 6 or 7), acetate buffer ((pH 4 or 5), Tris-HCl buffer (pH 8 or 9) or sodium carbonate buffer (pH 10)) with a final concentration of 50 mM to achieve a final concentration of 0.1 g (as the amount of FTH ferritin)/L for each. The surface charge at 25°C was measured for this resulting buffer with use of Zetasizer Nano ZS (Malvern Panalytical Ltd.). The measurement was carried out at 25°C using a DTS 1070 cell filled with 750 µL of a sample as well as the following settings: Material setting (RI: 1.450, Absorption: 0.001), Dispersant setting (Temperature: 25°C, Viscosity: 0.8872cP, RI: 1.330, Dielectric constant: 78.5) and Smoluchowski model (F*κ*a value: 1.50). As a result, the DOX-FTH ferritin composite was revealed to have a comparable surface charge to that of FTH ferritin without DOX encapsulated therein, demonstrating that the composite is not formed by adsorbing DOX on the surface of FTH ferritin (FIG. 7).

That is, the DOX-FTH ferritin composite obtained by leaving DOX and FTH ferritin to stand in an aqueous solution at one step, was revealed to have a comparable surface charge to that of FTH ferritin, suggesting a structure in which a basic DOX is not adsorbed on the FTH ferritin surface but encapsulated inside FTH ferritin.

### <Example 8: Evaluation of stability of DOX-encapsulating FTH ferritin>

Stability of the small molecule agent-encapsulating FTH ferritin obtained by the one-step process, was evaluated.

DOX was mixed with 1 mL of 50 mM Tris-HCl buffer (pH 8) that contains FTH ferritin at a final concentration of 1 mg/mL, to achieve a final concentration of 0.3 mg/L, and then the resulting solutions were left to stand at 40°C for 60 minutes. After the standing and centrifuging (at 15,000 rpm for one minute), the supernatant was injected into the desalting column PD-10 (Sephadex G-25 filled product, GE Healthcare Inc.) equilibrated with 10 mM Tris-HCl buffer (pH 8) to separate the protein from a reagent that was not encapsulated. Whole amount (3.5 mL) of the resulting solution was concentrated by centrifugal ultrafiltration using Vivaspin 20-100K (GE Healthcare Inc.), and then the composite of DOX and FTH ferritin suspended in 1 mL of water was obtained. The concentration of protein was determined using the protein assay CBB solution (Nacalai Tesque, Inc.) as well as bovine albumin as a standard.

The DOX-FTH ferritin composite was suspended in acetate buffer (pH 4) with a final concentration of 50 mM, 50 mM phosphate buffer (pH 6), 50 mM Tris-HCl buffer (pH 9) or D-PBS (-) (pH 7.4, FUJIFILM Wako Pure Chemical Corporation) to achieve a final concentration of 1.5 g/L as a protein content concentration. 0.1 mL of each resulting solution was preserved at a cold dark place (4°C). Subsequently, three samples in each condition were collected every several days, and then the DOX-FTH ferritin composite was separated from the buffer using Vivaspin 500-100K (GE Healthcare Inc.). The absorbance at 480 nm was measured using spectrophotometer (DU-800, Beckman Coulter Inc.) for each sample in order to determine the amount of DOX incorporated in the DOX-FTH ferritin composite.

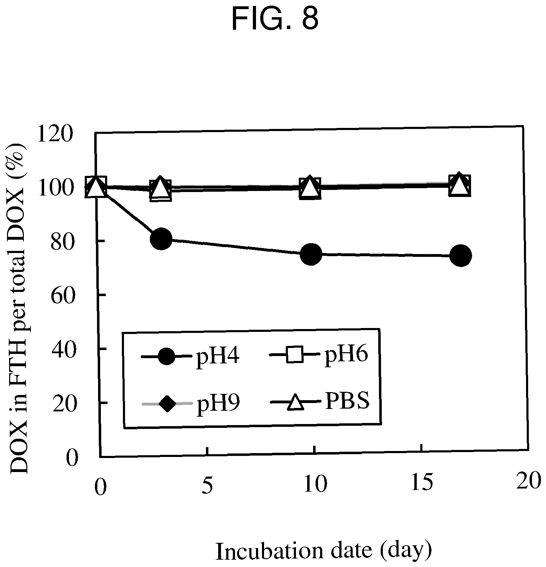

As a result, it was revealed that most of DOX preserved in the buffers at pH 6 to pH 9 was not released out from the DOX-FTH ferritin composite even after allowed to stand for two weeks or more (FIG. 8, average value ± standard deviation). Meanwhile, at pH 4, 30% (wt) of DOX was released right after the standing, and the extent of the release became small afterward. That is, the DOX-FTH ferritin composite was revealed to be stably maintained under appropriate pH conditions.

### <Example 9: Estimation of state of DOX in FTH ferritin>

Ferritin has a cage shape having a diameter of 12 nm, and the diameter of an internal cavity thereof is 7 nm. Ideally, the volume of the internal cavity is 0.18 zL (zL: Zepto little, 10 to the negative power of 21). When one molecule of DOX (molecular weight 579.98) is present in the internal cavity of ferritin, the concentration of DOX in the internal cavity of ferritin is 5.4 g/L. The solubility of DOX in water at room temperature is 60 g/L or more. In Examples, 165 molecules of DOX was incorporated per molecule of FTH ferritin, and the concentration of DOX in the internal cavity of ferritin is 890 g/L in Examples. The concentration is substantially higher than the water solubility of DOX in room temperature, and it is highly possible to cause deposition and formation of nanoparticles in the internal cavity of ferritin. In view of this, it is necessary to disassemble ferritin once to allow DOX to be released in advance for quantification of DOX incorporated in ferritin by means of spectroscopic evaluation.

### <Example 10: Comparison of one-step process with disassembly-reassembly process (1)>

The one-step process was compared with the disassembly-reassembly process in terms of the amount of encapsulated DOX of the DOX-encapsulating FTH ferritin obtained by each process.

In the disassembly-reassembly process, 0.5 mL of 50 mM glycine-HCl buffer (pH 2.3) containing FTH ferritin at a 1 mg/mL final concentration and DOX at a 0.2 mg/L final concentration, was left to stand for 5 to 120 minutes at room temperature. Then, 10 µL of 1M Tris-HCl buffer (pH 9.0) was added for neutralization, and the resulting solution was left to stand for 5 to 120 minutes at room temperature. After the standing, 0.5 mL of water was added to the resulting solution. After the resulting solution was centrifuged (at 15,000 rpm for one minute), the supernatant was injected into the desalting column PD-10 (Sephadex G-25 filled product, GE Healthcare Inc.) equilibrated with 10 mM Tris-HCl buffer (pH 8) to separate the protein from a reagent that was not encapsulated. Whole amount (3.5 mL) of the resulting solution was concentrated to 0.1 mL by centrifugal ultrafiltration using Vivaspin 20-100K (GE Healthcare Inc.), and then the absorbances at 280 nm and 480 nm were measured using spectrophotometer (DU-800, Beckman Coulter Inc.). On the basis of the obtained absorbances, concentrations of DOX and FTH ferritin were determined with reference to a calibration curve that was obtained by the absorbances at 280 nm and 480 nm measured for different concentrations of DOX and FTH ferritin. Incorporation efficiency of the reagent (weight of DOX with respect to total weight of DOX and FTH ferritin) was determined based on the concentrations.

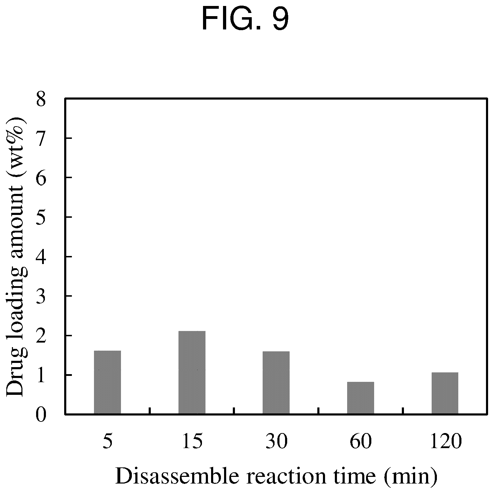

As a result, FTH ferritin allowed to stand for 15 minutes was confirmed to have the highest incorporation efficiency of the reagent of 2% (wt) (FIG. 9). Meanwhile, the DOX-encapsulating FTH ferritin that was obtained in the one-step process using FTH ferritin with a 1 mg/mL final concentration and DOX with a 0.2 mg/L final concentration, was confirmed to exhibit 5-fold increase in the incorporation efficiency of the reagent (FIG. 4). As such, it was revealed that the one-step process enables producing the FTH ferritin with a higher encapsulation efficiency of the reagent.

### <Example 11: Comparison of one-step process with disassembly-reassembly process (2)>

The one-step process was compared with the disassembly-reassembly process in terms of recovery yield of FTH ferritin obtained by each process.

At the one-step process, 0.1 mL of 50 mM Tris-HCl buffer (pH 9) containing FTH ferritin 10 mg/mL was left to stand at 60°C for 60 minutes. Next, 2.9 mL of 10 mM Tris-HCl buffer (pH 8.0) was added, and then the resulting solution was injected into the HiPrep 16/60 Sephacryl S-300 HR column (GE Healthcare Inc.) equilibrated with 10 mM Tris-HCl buffer (pH 8.0) in order to measure the absorbance at 280 nm while separating and purifying by size.

At the disassembly-reassembly process, 0.1 mL of 50 mM glycine-HCl buffer (pH 2.3) containing FTH ferritin 10 mg/mL was left to stand at room temperature for 15 minutes for disassembly of FTH ferritin into monomers. Next, 10 µL of 1 M Tris-HCl buffer (pH 9.0) was added for neutralization, and then the resulting solution was left to stand at room temperature for 15 minutes for reassembly of FTH ferritin. Next, 2.9 mL of 10 mM Tris-HCl buffer (pH 8.0) was added, and then the resulting solution was injected into the HiPrep 16/60 Sephacryl S-300 HR column (GE Healthcare Inc.) equilibrated with 10 mM Tris-HCl buffer (pH 8.0) in order to measure the absorbance at 280 nm while separating and purifying by size.

As a control, an equivalent amount of FTH ferritin without the standing at 60°C and the disassembly-reassembly treatment was also injected into the HiPrep 16/60 Sephacryl S-300 HR column (GE Healthcare Inc.) equilibrated with 10 mM Tris-HCl buffer (pH 8.0) in order to measure the absorbance at 280 nm while separating and purifying by size.

As a result, the recovery yield of FTH ferritin that was obtained by the one-step process with the standing at 60°C for 60 minutes was 97% of that of untreated FTH ferritin. Meanwhile, the recovery yield of FTH ferritin obtained by the disassembly-reassembly process was 72% of that of untreated FTH ferritin, and FTH ferritin monomers not involved in the reassembly was also confirmed in the later half period of elusion.

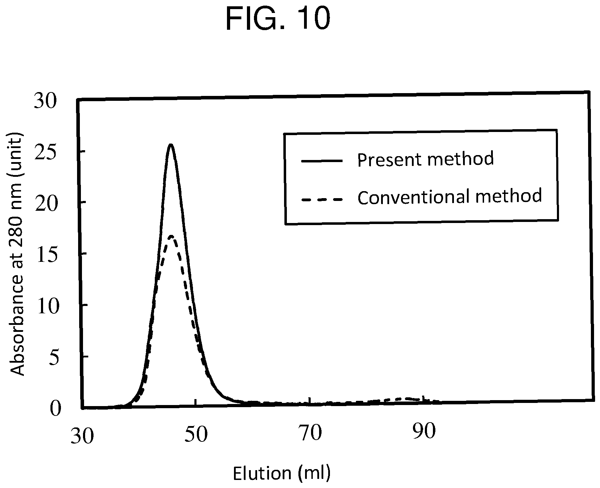

The above results demonstrate that the one-step process without proactively destroying an ultramolecular structure of FTH ferritin achieve a substantially larger amount of FTH ferritin recovered after the treatment, compared to the disassembly-reassembly process, which involves destroying the ultramolecular structure of FTH ferritin once, and thereby is an efficient method (FIG. 10).

### <Example 12: Incorporation of small molecule agent at one-step process (1)>

Various small molecules were incorporated into FTH ferritin at the one-step process.

The small molecule agents used for the incorporation in Examples are listed in Table 1. Each of various small molecule agents was mixed with phosphate buffer (pH 3, 6 or 7), acetate buffer (pH 4 or 5), Tris-HCl buffer (pH 8 or 9) or sodium carbonate buffer (pH 10) at a final concentration of 50 mM containing FTH ferritin at a final concentration of 1 mg/mL so as to achieve final concentrations ranging from 0.2 mM to 5 mM, and then each of resulting solutions was left to stand for 60 minutes at 20°C to 60°C. All of the reactions were performed in 0.1 mL volume. After the standing, the resulting solution was centrifuged (at 15,000 rpm for one minute), the supernatant was injected into the desalting column PD-10 (Sephadex G-25 filled product, GE Healthcare Inc.) equilibrated with 10 mM Tris-HCl buffer (pH 8) to separate the protein from a reagent that was not encapsulated. Whole amount (3.5 mL) of the resulting solution was concentrated to 0.1 mL by centrifugal ultrafiltration using Vivaspin 20-100K (GE Healthcare Inc.).

As a control, some of small molecule agents listed in Table 1 was incorporated into ferritin also at the disassembly-reassembly process. That is, 0.5 mL of 50 mM glycine-HCl buffer (pH 2.3) containing FTH ferritin at a 1 mg/mL final concentration and each of various small molecules at a 0.2 mg/L final concentration was left to stand for 15 minutes at room temperature. Then, 10 µL of 1M Tris-HCl buffer (pH 9.0) was added for neutralization, and the resulting solution was left to stand for 15 minutes at room temperature. After the standing, 0.5 mL of water was added. After the resulting solution was centrifuged (at 15,000 rpm for one minute), the supernatant was injected into the desalting column PD-10 (Sephadex G-25 filled product, GE Healthcare Inc.) equilibrated with 10 mM Tris-HCl buffer (pH 8) to separate the protein from a reagent that was not encapsulated. Whole amount (3.5 mL) of the resulting solution was concentrated to 0.1 mL by centrifugal ultrafiltration using Vivaspin 20-100K (GE Healthcare Inc.).

### [Table 1]

**Table 1.**

| No. | Molecule to be incorporated | Cas No. | Molecular weight | pKa | logP |
|---|---|---|---|---|---|
| 1 | Rhodamine B | 81-88-9 | 479.02 | 3.1 | 1.78 |
| 2 | Congo Red | 573-58-0 | 696.663 | 4.1 | -1.88 |
| 3 | Famotidine | 76824-35-6 | 337.449 | 8.38 | -0.64 |
| 4 | Metformin | 657-24-9 | 129.164 | 12.4 | -1.82 |
| 5 | Minoxidil | 38304-91-5 | 209.25 | 4.61 | 1.24 |
| 6 | Terbutaline | 23031-25-6 | 225.28 | 8.86; 9.76 | 0.9 |
| 7 | Creatinine | 60-27-5 | 113.118 | 2.96 | -1.6 |
| 8 | Nicotinamide | 98-92-0 | 122.12 | 3.35 | -0.37 |
| 9 | Riboflavin | 83-88-5 | 376.36 | 10.2 | -1.46 |
| 10 | Thiamine | 67-03-8 | 337.27 | 5.5 | -4.6 |

The absorbances were measured using spectrophotometer (DU-800, Beckman Coulter Inc.) for these solutions, and the concentration of contained protein was determined using the protein assay CBB solution (Nacalai Tesque, Inc.) as well as bovine albumin as a standard. Absorbances at 280 nm and 480 nm were measured for Rhodamine B and Congo Red. Absorbances at 280 nm and 480 nm were measured for Rhodamine B and Congo Red. Absorbances at 250 nm and 280 nm were measured for Famotidine, Metformin, Minoxidil, Terbutaline, Creatinine, Nicotinamide, Riboflavin and Thiamine. Then, after the absorbances were calibrated based on the absorbances that were derived from the proteins and presumed by the amount of contained protein quantified using the protein assay CBB solution, the concentrations of small molecule agents were determined based on the calibration curves obtained by the absorbances of the molecules.

Tables 2 and 3 list the incorporation conditions (reaction pH, reaction temperature and reaction time), the number of encapsulation (the number of small molecules encapsulated in one cage of the ferritin 24-mer) and the incorporation efficiency (the weight of the small molecule with respect to the total weight of the small molecule and ferritin, wt%). As a result, the one-step process enabled incorporating also the small molecule agents with different physical properties such as molecular weight and pKa simply and easily.

### [Table 2]

**Table 2. Conditions and results on introduction of small molecule agent by one-step process (1)**

| No. | Small molecule | pH | Temp. (°C) | Time (min) | Number of agent incorporated | Incorporation efficiency (wt%) |
|---|---|---|---|---|---|---|
| 1 | Rhodamine B | 4 | 60 | 60 | 4.3 | 0.4 |
| 2 | Rhodamine B | 5 | 60 | 60 | 16.7 | 1.5 |
| 3 | Rhodamine B | 6 | 60 | 60 | 3.5 | 0.3 |
| 4 | Rhodamine B | 7 | 60 | 60 | 2.3 | 0.2 |
| 5 | Rhodamine B | 8 | 60 | 60 | 1.8 | 0.2 |
| 6 | Rhodamine B | 9 | 60 | 60 | 2.5 | 0.2 |
| 7 | Rhodamine B | 10 | 60 | 60 | 6.6 | 0.6 |
| 8 | Rhodamine B | 8 | 20 | 60 | 11.1 | 1.0 |
| 9 | Rhodamine B | 9 | 30 | 60 | 7.6 | 0.7 |
| 10 | Rhodamine B | 9 | 40 | 60 | 3.9 | 0.4 |
| 11 | Rhodamine B | 9 | 50 | 60 | 2.6 | 0.2 |
| 12 | Rhodamine B | 10 | 40 | 60 | 3.2 | 0.3 |
| 13 | Rhodamine B | 10 | 50 | 60 | 2.6 | 0.2 |
| 14 | Congo Red | 3 | 25 | 60 | 0.2 | 0.0 |
| 15 | Congo Red | 4 | 25 | 60 | 3.7 | 0.5 |
| 16 | Congo Red | 5 | 25 | 60 | 1.0 | 0.1 |
| 17 | Congo Red | 6 | 25 | 60 | 0.9 | 0.1 |
| 18 | Congo Red | 7 | 25 | 60 | 0.7 | 0.1 |
| 19 | Congo Red | 8 | 25 | 60 | 0.8 | 0.1 |
| 20 | Congo Red | 9 | 25 | 60 | 0.8 | 0.1 |
| 21 | Congo Red | 6 | 10 | 60 | 1.4 | 0.2 |
| 22 | Congo Red | 6 | 20 | 60 | 1.4 | 0.2 |
| 23 | Congo Red | 6 | 30 | 60 | 1.6 | 0.2 |
| 24 | Congo Red | 6 | 40 | 60 | 1.7 | 0.2 |
| 25 | Congo Red | 6 | 50 | 60 | 1.8 | 0.2 |
| 26 | Congo Red | 6 | 60 | 60 | 2.8 | 0.4 |
| 27 | Famotidine | 4 | 40 | 60 | 0.0 | 0.0 |
| 28 | Famotidine | 6 | 40 | 60 | 15.1 | 1.0 |
| 29 | Famotidine | 8 | 40 | 60 | 8.4 | 0.6 |
| 30 | Famotidine | 10 | 40 | 60 | 5.1 | 0.3 |

### [Table 3]

**Table 3. Conditions and results on introduction of small molecule agent by one-step process (2)**

| No. | Small molecule | pH | Temp. (°C) | Time (min) | Number of agent incorporated | Incorporation efficiency (wt%) |
|---|---|---|---|---|---|---|
| 31 | Famotidine | 6 | 20 | 60 | 36.9 | 2.4 |
| 32 | Famotidine | 6 | 60 | 60 | 23.4 | 1.5 |
| 33 | Metformin | 4 | 40 | 60 | 0.0 | 0.0 |
| 34 | Metformin | 6 | 40 | 60 | 0.2 | 0.0 |
| 35 | Metformin | 8 | 40 | 60 | 2.9 | 0.1 |
| 36 | Metformin | 10 | 40 | 60 | 1.8 | 0.0 |
| 37 | Metformin | 8 | 20 | 60 | 78.0 | 1.9 |
| 38 | Metformin | 8 | 60 | 60 | 53.5 | 1.3 |
| 39 | Minoxidil | 4 | 40 | 60 | 8.3 | 0.3 |
| 40 | Minoxidil | 6 | 40 | 60 | 20.0 | 0.8 |
| 41 | Minoxidil | 8 | 40 | 60 | 12.0 | 0.5 |
| 42 | Minoxidil | 10 | 40 | 60 | 4.3 | 0.2 |
| 43 | Minoxidil | 6 | 20 | 60 | 65.6 | 2.6 |
| 44 | Minoxidil | 6 | 60 | 60 | 12.0 | 0.5 |
| 45 | Terbutaline | 4 | 40 | 60 | 0.0 | 0.0 |
| 46 | Terbutaline | 6 | 40 | 60 | 19.5 | 0.9 |
| 47 | Terbutaline | 8 | 40 | 60 | 21.0 | 0.9 |
| 48 | Terbutaline | 10 | 40 | 60 | 27.1 | 1.2 |
| 49 | Terbutaline | 10 | 20 | 60 | 32.8 | 1.4 |
| 50 | Terbutaline | 10 | 60 | 60 | 126.8 | 5.3 |
| 51 | Creatinine | 4 | 20 | 60 | 15.8 | 0.4 |
| 52 | Creatinine | 4 | 60 | 60 | 24.8 | 0.5 |
| 53 | Nicotinamide | 4 | 40 | 60 | 15.6 | 0.4 |
| 54 | Nicotinamide | 6 | 40 | 60 | 6.8 | 0.2 |
| 55 | Nicotinamide | 8 | 40 | 60 | 13.9 | 0.3 |
| 56 | Nicotinamide | 10 | 40 | 60 | 7.6 | 0.2 |
| 57 | Riboflavin | 4 | 40 | 60 | 6.4 | 0.5 |
| 58 | Riboflavin | 6 | 40 | 60 | 6.5 | 0.5 |
| 59 | Riboflavin | 8 | 40 | 60 | 6.4 | 0.5 |
| 60 | Riboflavin | 10 | 40 | 60 | 7.7 | 0.6 |
| 61 | Thiamine | 4 | 40 | 60 | 11.6 | 0.8 |
| 62 | Thiamine | 6 | 40 | 60 | 13.9 | 0.9 |
| 63 | Thiamine | 8 | 40 | 60 | 13.7 | 0.9 |
| 64 | Thiamine | 10 | 40 | 60 | 13.6 | 0.9 |

Tables 4 and 5 list the relation between the pKa of each small molecule agent and the pH of the buffer used in the reaction with the largest amount of incorporation within ferritin. The correlation is indicated in FIG. 11. The correlation coefficient was 0.84, indicating significantly positive correlation (t-test, p < 1%). It was suggested that the efficient incorporation within ferritin could be achieved for molecules with pKa lower than neutral pH (pH 7) under acidic conditions (pH 6 or less) and molecules with pKa higher than neutral pH under weak acidic to basic conditions (pH 6 or more).

### [Table 4]

**Table 4. Relationship between organic compound and its characteristic (1)**

| Organic compound | pKa | Optimal pH in reaction |
|---|---|---|
| 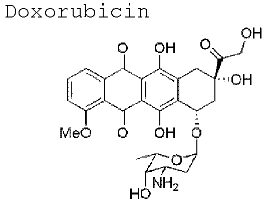 | 7.34 | 9 |
| | 8.46 | |
| | 9.46 | |
| | (average 8.42) | |
| 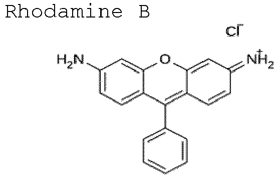 | 3.1 | 5 |
| 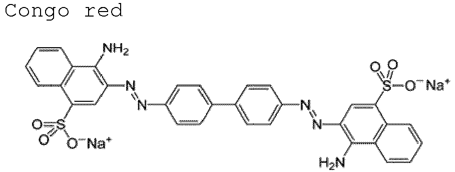 | 4.1 | 4 |
| 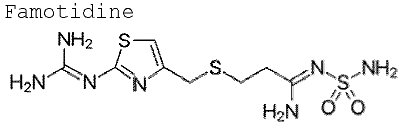 | 8.38 | 6 |
| 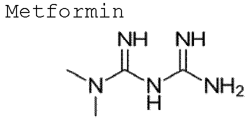 | 12.4 | 8 |
| 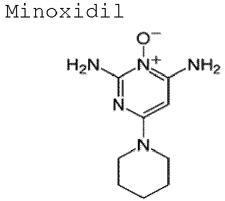 | 4.61 | 6 |

### [Table 5]

**Table 5. Relationship between organic compound and its characteristic (2)**

| Organic compound | pKa | Optimal pH in reaction |
|---|---|---|
| 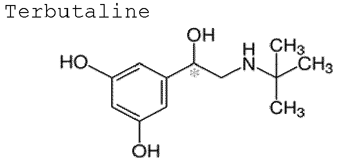 | 8.86 | 10 |
| | 9.76 | |
| | (average 9.31) | |
| 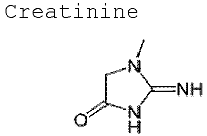 | 2.96 | 4 |
| 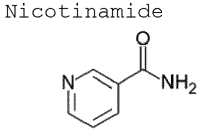 | 3.35 | 4 |
| 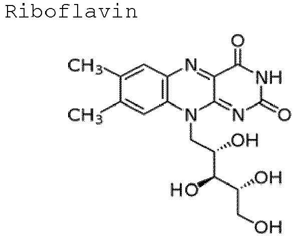 | 10.2 | 10 |
| 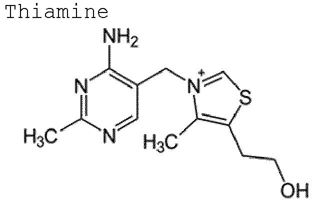 | 5.5 | 6 |

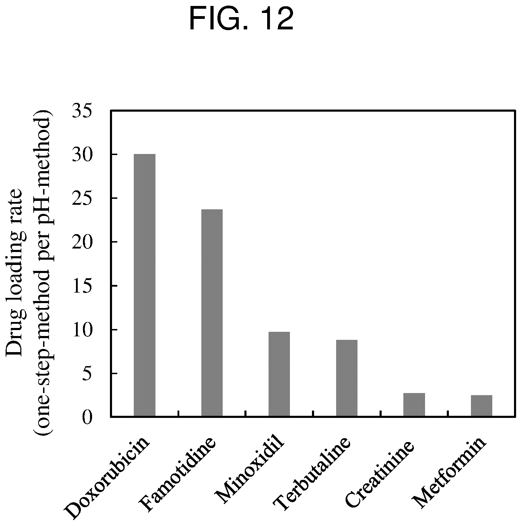

Next, the amount of the small molecule agent incorporated into ferritin at the one-step process was compared with the amount of the small molecule agent incorporated into ferritin at the disassembly-reassembly process. FIG. 12 represents ratios of amounts of incorporated reagents at the one-step process to those at the disassembly-reassembly process. The comparison of the incorporation amount of DOX was calculated according to the method in Examples 5 and 10. For all small molecule agents, the one-step process was confirmed to enable incorporating larger amounts of reagents into ferritin, in view of 2.5-fold difference even in the case of Metformin causing the smallest difference in the incorporation amount as well as 30-fold difference in the case of DOX achieving the largest difference in the incorporation amount. The disassembly-reassembly process involves the small molecule in the reaction solution for the encapsulation accompanied by the reassemblyof ferritin, thereby making it difficult to achieve a higher concentration for the encapsulation than that of small molecules in the reaction solution. In addition, the monomer structure of ferritin is partially modified when ferritin is disassembled into the monomers under the acidic condition, potentially causing risks such as leakage of the encapsulated reagent even after the reassembly.

Meanwhile, the one-step process enables incorporating the reagent into ferritin under the environment capable of maintaining the ultrastructure of ferritin, and is almost free from the leakage because of reduced damage on ferritin itself even after the reaction. Since various reagents were incorporated while the ultramolecular structure of ferritin is maintained, the reagent is presumed to be incorporated into ferritin through 3-fold channels similarly to metal ions. (T Douglas and DR Ripoll Protein Sci, 7, 1083-1091. (1998)), MA Carrondo EMBO J. 22, 1959-1968. (2003)). The possible reason is that ferritin is considered to have such a tight structure as to be incapable of passing therethrough even metal ions except the channels and therefore organic molecules with larger exclusion volumes are incapable of passing therethrough. Similarity to incorporation of metal ions resulting from an electrostatic gradient that is caused by negatively charged 3-fold channels in the neutral buffer, organic molecules are considered to be also actively incorporated into ferritin and then accumulated inside negatively charged ferritin and thereby enable encapsulating at a concentration of the small molecule in the reaction solution or higher. On the assumption that the negatively charged surface of ferritin allows through poles thereof incorporating inward due to the electrostatic gradient, organic molecules that have many amino groups and are easily positively charged are considered to be easily incorporated.

The above results demonstrate that the one-step process enables incorporating more reagents than conventional disassembly-reassembly process.

### <Example 13: Construction of DOX-encapsulating mutant FTH Ferritin (1) >

Total synthesis was carried out for a DNA encoding a human-derived ferritin H chain (FTH-BC-TBP) monomer in which a titanium recognizing peptide (minTBP1 : RKLPDA (SEQ ID NO: 7)) was inserted for fusion at a flexible linker region between second and third α-helices counted from an N-terminus of a ferritin monomer comprising six α-helices. PCR was carried out using the synthesized DNA as a template as well as the following primers: 5'-GAAGGAGATATACATATGACGACCGCGTCCACCTCG-3' (SEQ ID NO: 8) and 5'-CTCGAATTCGGATCCTTAGCTTTCATTATCACTGTC-3' (SEQ ID NO: 9). PCR was carried out using pET20 (Merck KGaA) as a template as well as the following primers: 5'-TTTCATATGTATATCTCCTTCTTAAAGTTAAAC-3' (SEQ ID NO: 10) and 5'-TTTGGATCCGAATTCGAGCTCCGTCG-3' (SEQ ID NO: 11). The resulting PCR products were purified using Wizard DNA Clean-Up System (Promega Corporation), and then subjected to In-Fusion enzyme treatment at 50°C for 15 minutes using In-Fusion HD Cloning Kit (Takara Bio Inc.) to construct an expression plasmid (pET20-FTH-BC-TBP) carrying a gene encoding FTH-BC-TBP monomer. The ferritin constructed from the FTH-BC-TBP monomer is referred to as FTH-BC-TBP ferritin, as appropriate.

PCR was carried out using pET20-FTH carrying a DNA encoding a human-derived ferritin H chain monomer as a template as well as the following primers: 5'-TTTGGATCCGAATTCGAGCTCCGTCG-3' (SEQ ID NO: 12) and 5'-TTTGGATCCTTAACAGCTTTCATTATCACTG-3' (SEQ ID NO: 13). The resulting PCR products were purified using Wizard DNA Clean-Up System (Promega Corporation), and then treated with restriction enzymes, DpnI and BamHI (Takara Bio Inc.) for self-ligation in order to construct an expression plasmid (pET20-FTHc) carrying a gene encoding FTHc monomer with a cysteine added to the C-terminus. Ferritin including FTHc monomers is referred to as FTHc ferritin, as appropriate.

Subsequently, Escherichia coli BL21 (DE3) into which the vector pET20-FTH-BC-TBP or pET20-FTHc carrying the constructed mutate ferritin monomer (FTH-BC-TBP monomer or FTHc monomer) was introduced, was cultured in 100 mL of an LB medium (including 10 g/L of Bacto-typtone, 5 g/L of Bacto-yeast extract, 5 g/L NaCl and 100 mg/L of ampicillin) at 37°C for 24 hours using flasks. The resulting bacterial cells were sonicated for cell disruption, and then the resulting supernatant was heated at 60°C for 20 minutes. The supernatant obtained after the heating was injected into a HiPerp Q HP column (GE Healthcare Inc.) equilibrated with 50 mM Tris-HCl buffer (pH 8.0). Then, the aimed protein was separated and purified by applying a concentration gradient of the salt from 0 mM to 500 mM NaCl in 50 mM Tris-HCl buffer (pH 8.0). The solvent of the solution containing the protein was replaced with 10 mM Tris-HCl buffer (pH 8.0) by centrifugal ultrafiltration using Vivaspin 20-100K (GE Healthcare Inc.). The resulting solution was injected into a HiPrep 26/60 Sephacryl S-300 HR column (GE Healthcare Inc.) equilibrated with 10 mM Tris-HCl buffer (pH 8.0) to separate and purify each mutate ferritin by size. The solution containing each mutate ferritin was concentrated by centrifugal ultrafiltration using Vivaspin 20-100K (GE Healthcare Inc.), and then concentration of the contained protein was determined using a protein assay CBB solution (Nacalai Tesque, Inc.) as well as bovine albumin as a standard. As a result, 1 mL solution containing 0.2 mg/mL of FTH-BC-TBP ferritin and 1 mL solution containing 5 mg/mL of FTHc ferritin, were obtained.

DOX was mixed with 1 mL of 50 mM Tris-HCl buffer (pH 8) that contains each mutate ferritin at a final concentration of 1 mg/mL, to achieve a final concentration of 0.3 mg/L, and then the resulting solutions were left to stand at 50°C for 60 minutes. After the standing and centrifuging (at 15,000 rpm for one minute), the supernatant was injected into the desalting column PD-10 (Sephadex G-25 filled product, GE Healthcare Inc.) equilibrated with 10 mM Tris-HCl buffer (pH 8) to separate the protein from a reagent that was not encapsulated. Whole amount (3.5 mL) of the resulting solution was concentrated by centrifugal ultrafiltration using Vivaspin 20-100K (GE Healthcare Inc.) to obtain the composite of DOX and each mutate ferritin suspended in 1 mL of water. The concentration of contained protein was determined using the protein assay CBB solution (Nacalai Tesque, Inc.) as well as bovine albumin as a standard. The DOX concentration was determined based on analysis of the absorbance at 480 nm.

As a result, DOX could be incorporated into both of the mutant ferritins. At that time, the incorporation efficiency of the reagent was 6.5% (wt) for FTH-DE-TBP ferritin and 8.6% (wt) for FTHc ferritin, revealing that it is possible to incorporate the reagent using the one-step process also for the functionalized ferritin with peptide inserted.

### <Example 14: Construction of DOX-encapsulating mutant FTH (2) >

The incorporation of the reagent into ferritin was carried out using the one-step process for ferritin to which a peptide aptamer is presented.

First, PCR was carried out using pET20-FTH carrying a DNA encoding a human-derived ferritin H chain monomer as a template as well as the following primers: 5'-TTTCATATGGCACGTAGTGGTTTAACGACCGCGTCCACCTCG-3'(SEQ ID NO: 15) and 5'-TTTCATATGTATATCTCCTTCTTAAAGTTAAAC-3'(SEQ ID NO: 16) in order to obtain a DNA encoding the human-derived ferritin H chain (HER2a-FTH) monomer in which a cancer recognition peptide (HER2a: MARSGL (SEQ ID NO: 14); M Houimel et al., Int. J. Cancer 92, 748-755. (2001)) is inserted and fused at the N-terminus of the ferritin monomer. The resulting PCR products were purified using Wizard DNA Clean-Up System (Promega Corporation), and then treated with restriction enzymes, DpnI and NdeI (Takara Bio Inc.) for self-ligation in order to construct an expression plasmid (pET20-HER2a-FTH) carrying a gene encoding HER2a-FTH. Ferritin constructed from the HER2a-FTH monomers is referred to as HER2a-FTH ferritin, as appropriate.

PCR was carried out using pET20-FTH carrying a DNA encoding a human-derived ferritin H chain monomer as a template as well as the following primers: 5'-TTTGGATCCTTATCTGCGTGCTTGACGTGTGCTTTCATTATCACTG-3' (SEQ ID NO: 18) and 5'-TTTGGATCCTTAACAGCTTTCATTATCACTG-3' (SEQ ID NO: 13), in order to obtain a DNA encoding the human-derived ferritin H chain (FTH-U2AF) in which an RNA recognition peptide (U2AF: TRQARR (SEQ ID NO: 17) R Tan and AD Frankel, Proc Natl Acad Sci USA 92, 5282-5286. (1995))) is inserted and fused at the C-terminus of the ferritin monomer. The resulting PCR products were purified using Wizard DNA Clean-Up System (Promega Corporation), and then treated with restriction enzymes, DpnI and NdeI (Takara Bio Inc.) for self-ligation in order to construct an expression plasmid (pET20-FTH-U2AF) carrying a gene encoding FTH-U2AF monomer. Ferritin constructed from the FTH-U2AF monomers is referred to as FTH-U2AF ferritin, as appropriate.

Subsequently, Escherichia coli BL21 (DE3) into which pET20 carrying the constructed mutate ferritin monomer (HER2a-FTH monomer or FTH-U2AF monomer) was introduced, was cultured in 100 mL of an LB medium (including 10 g/L of Bacto-typtone, 5 g/L of Bacto-yeast extract, 5 g/L NaCl and 100 mg/L of ampicillin) at 37°C for 24 hours using flasks. The resulting bacterial cells were sonicated for cell disruption, and then the resulting supernatant was heated at 60°C for 20 minutes. The supernatant obtained after the heating was injected into a HiPerp Q HP column (GE Healthcare Inc.) equilibrated with 50 mM Tris-HCl buffer (pH 8.0). Then, the aimed protein was separated and purified by applying a concentration gradient of the salt from 0 mM to 500 mM NaCl in 50 mM Tris-HCl buffer (pH 8.0). The solvent of the solution containing the protein was replaced with 10 mM Tris-HCl buffer (pH 8.0) by centrifugal ultrafiltration using Vivaspin 20-100K (GE Healthcare Inc.). The resulting solution was injected into a HiPrep 26/60 Sephacryl S-300 HR column (GE Healthcare Inc.) equilibrated with 10 mM Tris-HCl buffer (pH 8.0) to separate and purify each mutate ferritin by size. The solution containing each mutate ferritin was concentrated by centrifugal ultrafiltration using Vivaspin 20-100K (GE Healthcare Inc.), and then concentration of the contained protein was determined using a protein assay CBB solution (Nacalai Tesque, Inc.) as well as bovine albumin as a standard. As a result, 1 mL solution containing 0.8 mg/mL of HER2a-FTH ferritin and 1 mL solution containing 1.1 mg/mL of FTH-U2AF ferritin, were obtained.

DOX was mixed with 1 mL of 50 mM Tris-HCl buffer (pH 9) that contains each mutate ferritin at a final concentration of 1 mg/mL, to achieve a final concentration of 0.3 mg/L, and then the resulting solutions were left to stand at 50°C for 60 minutes. After the standing and centrifuging (at 15,000 rpm for one minute), the supernatant was injected into the desalting column PD-10 (Sephadex G-25 filled product, GE Healthcare Inc.) equilibrated with 10 mM Tris-HCl buffer (pH 8) to separate the protein from a reagent that was not encapsulated. Whole amount (3.5 mL) of the resulting solution was concentrated by centrifugal ultrafiltration using Vivaspin 20-100K (GE Healthcare Inc.) to obtain the composite of DOX and each mutate ferritin suspended in 1 mL of water. The concentration of contained protein was determined using the protein assay CBB solution (Nacalai Tesque, Inc.) as well as bovine albumin as a standard. The DOX concentration was determined based on analysis of the absorbance at 480 nm.

As a result, DOX could be incorporated into both mutant ferritins. At that time, the incorporation efficiency of the reagent was 14.6% (wt) for HER2a-FTH ferritin and 17.9% (wt) for FTH-U2AF ferritin, revealing that it is possible to incorporate the reagent using the one-step process also for the functionalized ferritin with peptide inserted.

### <Example 15: pH-dependence of FTH ferritin structure>

In order to investigate a pH-dependent change of the cage structure of ferritin, FTH ferritin was suspended in each of solutions with different pHs to achieve a final concentration of 1 mg/mL, and then the resulting solutions were left to stand at 25°C for 30 minutes. Then, the size of ferritin was measured by the dynamic light scattering (DLS) method using Zetasizer Nano ZS for each pH. The measurement was carried out at 25°C. The used solutions were 0.1 N HCl (pH 1.7), 20 mM phosphoric acid (pH 2.6, pH 3.2, pH 5.6, pH 6.7 and pH 7.7), 20 mM acetic acid (pH 4.2, pH 4.9 and pH 5.3), 1 mM NaOH (pH 11.1), 10 mM NaOH (pH 12.3) and 100 mM NaOH (pH 12.8). pH adjustment was carried out for the solutions ranging from pH 2.0 to pH 2.4 by adding HCl to 10 mM Tris-HCl solution.

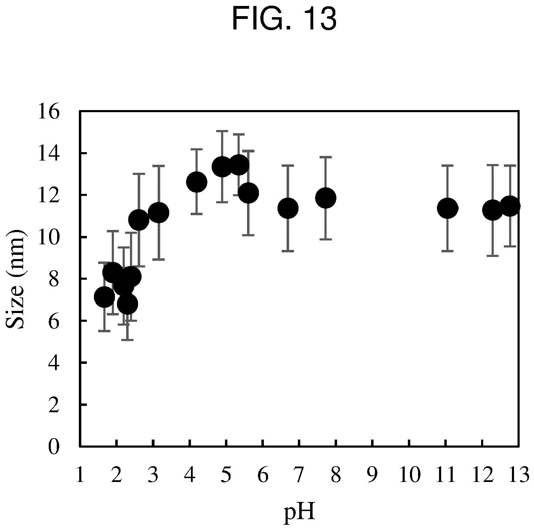

As a result, an average particle diameter determined by DLS was 8 nm or less in a range from pH 1.7 to pH 2.4, suggesting that ferritin is disassembled to break the cage structure (FIG. 13). Meanwhile, the average particle diameter was 11 nm or more in a range from pH 2.6 to pH 12.8, revealing that the cage structure is maintained. Namely, a one-pot method in the Example enables incorporating the reagent into ferritin on the condition that the cage structure is maintained.

### <Example 16: Construction of DOX-encapsulating FTL ferritin>

The incorporation of the reagent into L chain reagent was carried out using the one-step process. Total synthesis was carried out for a DNA encoding a human-derived ferritin L chain (FTL (SEQ ID NO: 2)) monomer. PCR was carried out using the synthesized DNA as a template as well as the following primers: 5'-GAAGGAGATATACATATGAGCTCCCAGATTCGTCAG-3' (SEQ ID NO: 234) and 5'-CTCGAATTCGGATCCTTAGTCGTGCTTGAGAGTGAG-3' (SEQ ID NO: 235). PCR was carried out using pET20 (Merck KGaA) as a template as well as the following primers: 5'-TTTCATATGTATATCTCCTTCTTAAAGTTAAAC-3' (SEQ ID NO: 5) and 5'-TTTGGATCCGAATTCGAGCTCCGTCG-3' (SEQ ID NO: 6). The resulting PCR products were purified using Wizard DNA Clean-Up System (Promega Corporation), and then subjected to In-Fusion enzyme treatment at 50°C for 15 minutes using In-Fusion HD Cloning Kit (Takara Bio Inc.) to construct an expression plasmid (pET20-FTL) carrying a gene encoding FTL monomer.

Subsequently, Escherichia coli BL21 (DE3) into which the constructed pET20-FTL was introduced, was cultured in 100 mL of an LB medium (including 10 g/L of Bacto-typtone, 5 g/L of Bacto-yeast extract, 5 g/L NaCl and 100 mg/L of ampicillin) at 37°C for 24 hours using flasks. The resulting bacterial cells were sonicated for cell disruption, and then the resulting supernatant was heated at 60°C for 20 minutes. The supernatant obtained after the heating was injected into a HiPerp Q HP column (GE Healthcare Inc.) equilibrated with 50 mM Tris-HCl buffer (pH 8.0). Then, ferritin (referred to as FTL ferritin, hereinafter) constructed from the FTL monomer was separated and purified by applying a concentration gradient of the salt from 0 mM to 500 mM NaCl in 50 mM Tris-HCl buffer (pH 8.0). The solvent of the solution containing FTL ferritin was replaced with 10 mM Tris-HCl buffer (pH 8.0) by centrifugal ultrafiltration using Vivaspin 20-100K (GE Healthcare Inc.). The resulting solution was injected into a HiPrep 26/60 Sephacryl S-300 HR column (GE Healthcare Inc.) equilibrated with 10 mM Tris-HCl buffer (pH 8.0) to separate and purify FTL ferritin by size. The solution containing FTL ferritin was concentrated by centrifugal ultrafiltration using Vivaspin 20-100K (GE Healthcare Inc.), and then concentration of the contained protein was determined using a protein assay CBB solution (Nacalai Tesque, Inc.) as well as bovine albumin as a standard. As a result, 1 mL solution containing 3 mg/mL of FTL ferritin was obtained.

DOX was mixed with 1 mL of 50 mM Tris-HCl buffer (pH 8) that contains each mutate FTL ferritin at a final concentration of 1 mg/mL, to achieve a final concentration of 0.3 mg/L, and then the resulting solutions were left to stand at 50°C for 60 minutes. After the standing and centrifuging (at 15,000 rpm for one minute), the supernatant was injected into the desalting column PD-10 (Sephadex G-25 filled product, GE Healthcare Inc.) equilibrated with 10 mM Tris-HCl buffer (pH 8) to separate the protein from a reagent that was not encapsulated. Whole amount (3.5 mL) of the resulting solution was concentrated by centrifugal ultrafiltration using Vivaspin 20-100K (GE Healthcare Inc.) to obtain the composite of DOX and FTL ferritin suspended in 1 mL of water. The concentration of contained protein was determined using the protein assay CBB solution (Nacalai Tesque, Inc.) as well as bovine albumin as a standard. The DOX concentration was determined based on analysis of the absorbance at 480 nm.

As a result, DOX could be incorporated into FTL ferritin. At that time, the incorporation efficiency of the reagent was 7.0 % (wt), revealing that it is possible to incorporate the reagent using the one-step process also for FTL ferritin.

### <Example 17: Incorporation of small molecule agent at one-step process (2) >

Uranine (Cas no. 518-47-8, molecular weight 376, pKa = 2.2, 4.4 and 6.7) having a negatively charged functional group was incorporated into FTH by one-step process.

Uranine was mixed with phosphate buffer (pH 3 or 7) or acetate buffer (pH 5) having a 50 mM final concentration that contains FTH ferritin at a final concentration of 3 mg/mL, to achieve a final concentration of 300 mM, and then the resulting solutions were left to stand at 30°C or 60°C for 60 minutes. All reactions were carried out in 1 mL volume. After the standing and centrifuging (at 15,000 rpm for one minute), the supernatant was injected into the desalting column PD-10 (Sephadex G-25 filled product, GE Healthcare Inc.) equilibrated with 10 mM Tris-HCl buffer (pH 8) to separate the protein from a reagent that was not encapsulated, for obtaining 3.5 mL of sample solution. The absorbance at 430 nm was measured using spectrophotometer (DU-800, Beckman Coulter Inc.) for the resulting solution in order to determine the concentration of uranine. The concentration of contained protein was determined using the protein assay CBB solution (Nacalai Tesque, Inc.) as well as bovine albumin as a standard.

The results are listed in Table 6. An optimum value of the reaction pH presumed from 4.4 that is an average value of uranine pKa in this Example, was approximately pH 5. Actually, the incorporation into ferritin was achieved efficiently at pH 3 and pH 5 at 60°C.

### [Table 6]

**Table 6. Incorporation of uranine by one-step process**

| No. | Reaction temp. | pH | Uranine (µM) | FTH ferritin (µM) | Uranine /FTH ferritin |
|---|---|---|---|---|---|
| 1 | 30°C | 3 | 71.0 | 0.7 | 99.2 |
| 2 | 30°C | 5 | 135.6 | 0.7 | 183.6 |
| 3 | 30°C | 7 | 68.5 | 0.8 | 90.0 |
| 4 | 60°C | 3 | 198.9 | 0.7 | 267.5 |
| 5 | 60°C | 5 | 201.4 | 0.8 | 265.3 |
| 6 | 60°C | 7 | 93.4 | 0.7 | 137.7 |

As a control, uranine was incorporated into ferritin in the disassembly-reassembly process. That is, 0.5 mL of 50 mM glycine-HCl buffer (pH 2.3) that contains FTH ferritin at a 5 mg/mL final concentration and uranine at each of various final concentrations ranging from 1 mM to 300 mM, was left to stand for 15 minutes at room temperature. Then, 10 µL of 1 M Tris-HCl buffer (pH 9.0) was added for neutralization, and the resulting solution was left to stand for 15 minutes at room temperature. After the standing, 0.5 mL of water was added. After the resulting solution was centrifuged (at 15,000 rpm for one minute), the supernatant was injected into the desalting column PD-10 (Sephadex G-25 filled product, GE Healthcare Inc.) equilibrated with 10 mM Tris-HCl buffer (pH 8) to separate the protein from a reagent that was not encapsulated. Whole amount (3.5 mL) of the resulting solution was concentrated to 0.1 mL by centrifugal ultrafiltration using Vivaspin 20-100K (GE Healthcare Inc.).

As a result, the most efficient encapsulation was confirmed in the case of 100 mM of uranine contained in the reaction solution (Table 7). From the comparison of the one-step process with the disassembly-reassembly process in terms of the amount of encapsulating uranine of the uranine-encapsulating FTH ferritin obtained by each process, the sample produced by the one-step process encapsulates a 2.7-fold larger amount of uranine suggesting that the one-step process achieves more efficient encapsulation of the reagent.

### [Table 7]

**Table 7. Incorporation of uranine by disassembly and reassembly process**

| No. | Concentration of uranine in reaction (mM) | Uranine (µM) | FTH ferritin (µM) | Uranine / FTH ferritin |
|---|---|---|---|---|
| 1 | 1 | 864.5 | 23.6 | 36.7 |
| 2 | 5 | 1186.3 | 25.1 | 47.2 |
| 3 | 10 | 1382.1 | 26.3 | 52.6 |
| 4 | 50 | 1764.2 | 24.3 | 72.5 |
| 5 | 100 | 2106.1 | 21.0 | 100.2 |
| 6 | 300 | 2405.1 | 26.5 | 90.8 |

### <Example 18: Stability of dye-encapsulating ferritin constructed at one-step process>

The stability in plasma was evaluated for the uranine-encapsulating FTH ferritin produced at the one-step process. First, the uranine-encapsulating FTH ferritin at a 1 mg/mL final concentration in 40 µL of human plasma (standard human plasma for blood coagulation test, GCH-100 A, SIEMENS, available from Sysmex Corporation, Lot503264B), was left to stand at 37°C while shielded from light. Three samples were collected soon after the beginning of the reaction, 24 hours or 48 hours after the standing, and then refrigerated for storage immediately. Each of the obtained samples was 5-fold diluted with water, and then subjected to spectroscopic measurement at 430 nm for quantification of uranine in FTH ferritin as well as gel filtration column analysis for separation of FTH ferritin from plasma protein. The condition of the gel filtration column analysis is set to 0.8 mL/min flow rate with Superdex 200 increase (GE Healthcare Inc.) and PBS (pH 7.4).

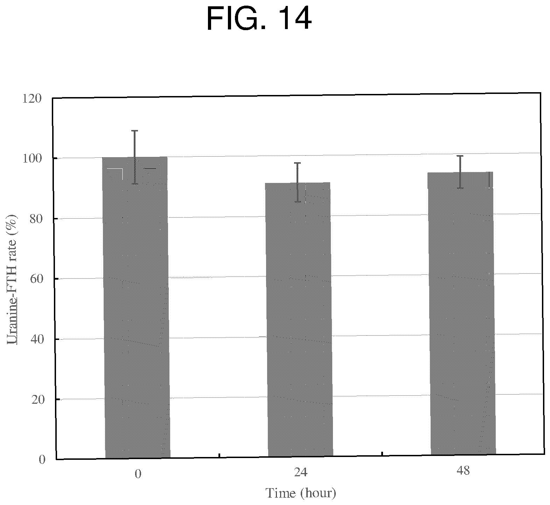

As a result, 94 % of the encapsulated uranine was encapsulated into FTH ferritin even after 48 hours, revealing that the uranine-encapsulating FTH ferritin is stable in plasma (FIG. 14).

### <Example 19: Cell incorporation of dye-encapsulating ferritin produced by one-step process>

It is known that ferritin is transported into cells depending on transferrin receptor (TfR). For evaluation of intracellular transportability of the reagent-encapsulating ferritin produced by the one-step process, intracellular incorporation activity of the uranine-encapsulating FTH ferritin was evaluated using uranine as a model compound.

Uranine was mixed with 2 mL of acetate buffer (pH 5) having a 50 mM final concentration that contains FTH ferritin at a final concentration of 3 mg/mL, to achieve a final concentration of 100 mM, and then the resulting solution was left to stand at 40°C for 60 minutes. After the standing and centrifuging (at 15,000 rpm for one minute), the supernatant was injected into the desalting column PD-10 (Sephadex G-25 filled product, GE Healthcare Inc.) equilibrated with 10 mM Tris-HCl buffer (pH 8) to separate the protein from a reagent that was not encapsulated. 3.5 mL of the resulting sample solution was injected into a HiPrep 16/60 Sephacryl S-300 HR equilibrated with PBS to elude the uranine-encapsulating FTH ferritin at a flow rate of 0.5 mL/min for purification with PBS. After the purification, the absorbance at 430 nm was measured using spectrophotometer (DU-800, Beckman Coulter Inc.) for uranine-encapsulating FTH ferritin concentrated by centrifugal ultrafiltration using Vivaspin 20-100K (GE Healthcare Inc.), for the purpose of determining concentration of uranine as well as the concentration of the contained protein using a protein assay CBB solution (Nacalai Tesque, Inc.) and bovine albumin as a standard. As a result, 1 mL of the uranine-encapsulating FTH ferritin aqueous solution containing 569 µM uranine solution and 6.7 µM FTH ferritin, was obtained.

Human breast cancer derived SKBR-3 cell (20,000 cell/well, 96-well plate) was added to a 100 µL medium produced by adding the uranine-encapsulating FTH ferritin to an evaluation medium (Opti-MEM™ (Thermo Fisher Scientific Inc.) + 1% non-essential amino acid solution (Thermo Fisher Scientific Inc.) + 1% penicillin-streptomycin (Nacalai Tesque, Inc.)) at each of different final concentrations ranging from 0 nM (uranine concentration 0 µM) to 800 nM (uranine concentration 67.9 µM), and then cultured at 37°C. The transferrin receptor TfR was expressed in the SKBR-3 cell. After the cell culture for 24 hours for each, the resulting solution was washed two times with 100 µL of phosphate buffer saline solution and left to stand in 50 µL of Trypsin-EDTA (Sigma-Aldrich, Inc.) at 37°C for 10 minutes. After 100 µL of Opti-MEM™ medium was added, the cells were transferred to a plate for fluorescence-activated cell sorting (FACS), and then subjected to centrifugal separation at 400 x g for 5 minutes. The cells for each were suspended in a FACS buffer for (Attune™ Focusing Fluid, Thermo Fisher Scientific Inc.), and analyzed by FACS (Attune NxT, Thermo Fisher Scientific Inc.).

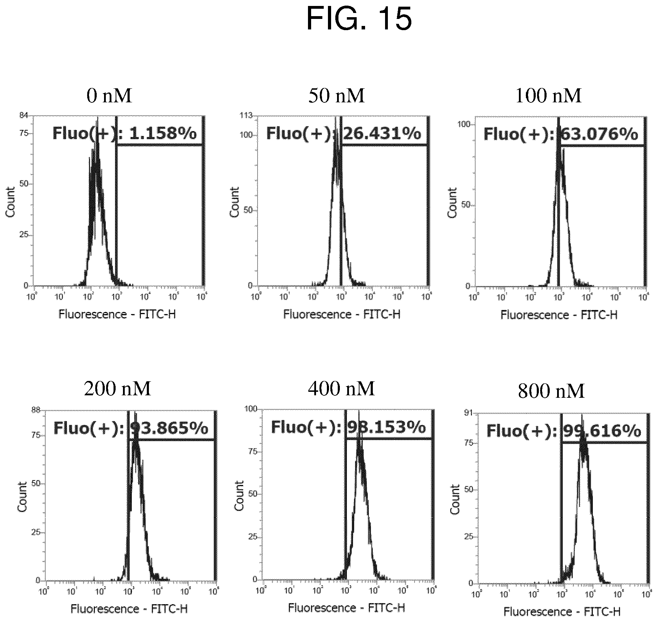

As a result, the fluorescence intensity was confirmed to vary depending on the concentration of the uranine-encapsulating FTH ferritin, suggesting increase in the amount of incorporation into cells dependent on the concentration (FIG. 15).

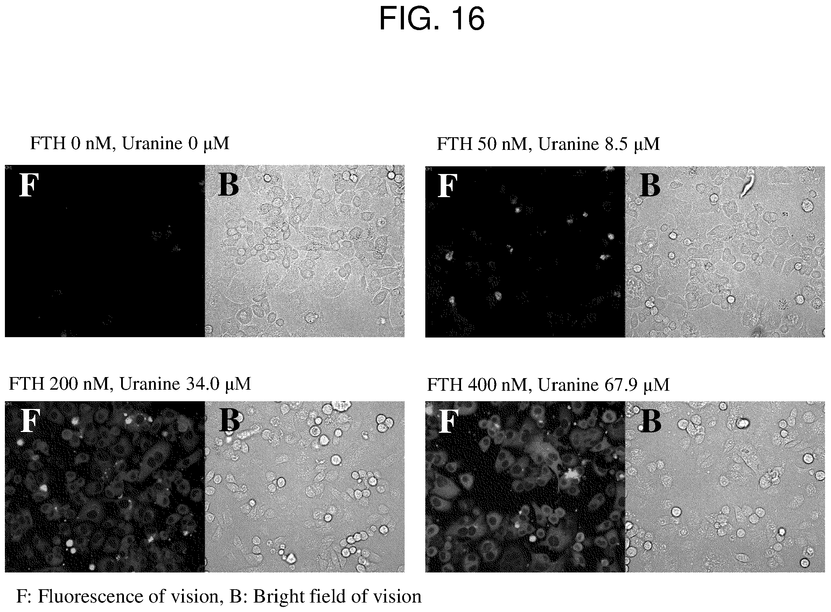

The cells prepared by the same condition were observed with two-photon excitation fluorescence microscope (CQ-1, Yokogawa Electric Corporation), making it possible to confirm the concentration dependent incorporation of the uranine-encapsulating FTH ferritin (FIG. 16).

The above results suggested that the reagent encapsulating ferritin has the capability to transport into cells similarly to naturally occurring ferritin.

### INDUSTRIAL APPLICABILITY

The methods of the present invention are promising for new drug delivery systems (DDS), for example, as well as production of an organic compound-encapsulating ferritin to be utilized in manufacturing of electronic devices. For example, when the ferritin monomer in the fusion protein constituting the multimer of the present invention is the human ferritin monomer, the multimer of the present invention is useful as DDS. The multimer of the present invention achieves superior safety in clinical applications in view of the human ferritin monomer not exhibiting antigenicity or immunogenicity against human.

Buffers of the present invention are useful for production of the organic compound-encapsulating ferritin, for example.

The organic compound-encapsulating ferritin is useful as new drug delivery systems (DDS), for example.

## Claims

1. A method for producing an organic compound-encapsulating ferritin, the method comprising:
(1) mixing an organic compound with ferritin in a buffer to obtain a mixture of the organic compound and ferritin; and
(2) incubating the mixture in the buffer,
wherein the buffer has a pH of 3 or more and less than 13, and
the buffer has a pH of 3 or more and less than 7 when the organic compound has a pKa less than 7, and has a pH of 6 or more and less than 13 when the organic compound has a pKa of 7 or more.

2. The method according to claim 1, wherein the pH of the buffer and the pKa of the organic compound satisfy a relation defined by formula: pH = 2.6 + 0.6 pKa ± 0.4 pKa.

3. The method according to claim 1 or 2, wherein a temperature of the incubating is 10°C or more and 60°C or less.

4. The method according to any one of claims 1 to 3, wherein a molecular weight of the organic compound is 100 or more and less than 1000.

5. The method according to any one of claims 1 to 4, wherein the pKa of the organic compound is 2 or more and 13 or less.

6. The method according to any one of claims 1 to 5, wherein 10 or more and 200 or less molecules of the organic compound are encapsulated in one molecule of ferritin.

7. The method according to any one of claims 1 to 6, wherein a total time for the mixing and the incubating is 30 minutes or more.

8. The method according to any one of claims 1 to 7, wherein a weight ratio of the organic compound to ferritin (organic compound / ferritin) at the mixing is 0.20 or more and 0.36 or less.

9. The method according to any one of claims 1 to 8, wherein the organic compound has a positively charged portion or a portion capable of being positively charged.

10. The method according to claim 9, wherein the positively charged portion is (a) a cationic nitrogen-containing group selected from the group consisting of ammonium group, guanidinium group, imidazolium group, oxazolium group, thiazolium group, oxadiazolium group, triazolium group, pyrrolidinium group, pyridinium group, piperidinium group, pyrazolium group, pyrimidinium group, pyrazinium group and triazinium group or (b) phosphonium group.

11. The method according to claim 9 or 10, wherein the portion capable of being positively charged is (a') a nitrogen-containing group selected from the group consisting of amino group, guanidino group, nitro group, amide group, hydrazide group, imide group, azide group and diazo group or (b') phosphino group.

12. The method according to any one of claims 1 to 11, wherein ferritin is (1) naturally occurring ferritin or (2) ferritin including a genetically modified ferritin monomer with any of following modifications (a) to (c):
(a) a ferritin monomer with a functional peptide inserted into a flexible linker region between second and third α-helicescounted from an N-terminus of the ferritin monomer comprising six α-helices ;
(b) a ferritin monomer with the functional peptide added to the N-terminus; or
(c) a ferritin monomer with the functional peptide added to the C-terminus.

13. A buffer comprising an organic compound-encapsulating ferritin,
wherein the buffer has a pH of 3 or more and less than 7 when an organic compound has a pKa less than 7, and has a pH of 6 or more and less than 12 when the organic compound has a pKa of 7 or more.

14. The buffer according to claim 13, wherein the buffer has a pH of 6 or more and 9 or less.

15. The buffer according to claim 13 or 14, wherein the organic compound has a pKa of 6 or more and 9 or less.

16. An organic compound-encapsulating ferritin, wherein
an organic compound is selected from the group consisting of an anthracycline substance, a histamine H2 receptor antagonist, a piguanide substance, an ATP-sensitive potassium channel opening agent, a β2 adrenergic receptor agonist, an imidazoline substance, a vitamin, or a labeling substance, and
number of molecules of the organic compound encapsulated into one molecule of ferritin is as follows:
(1) 40 or more and 200 or less molecules when the organic compound is the anthracycline substance and ferritin is naturally occurring ferritin;
(2) 100 or more and 200 or less molecules when the organic compound is the anthracycline substance and ferritin is genetically modified ferritin; or
(3) 10 or more and 200 or less molecules when the organic compound is the histamine H2 receptor antagonist, the piguanide substance, the ATP-sensitive potassium channel opening agent, the β2 adrenergic receptor agonist, the imidazoline substance, the vitamin, or the labeling substance, and ferritin is naturally occurring ferritin or genetically modified ferritin.

17. The organic compound-encapsulating ferritin according to claim 16,
wherein the anthracycline substance is doxorubicin,
the histamine H2 receptor antagonist is famotidine,
the piguanide substance is metformin,
the ATP-sensitive potassium channel opening agent is minoxidil,
the β2 adrenergic receptor agonist is terbutaline,
the imidazoline substance is creatinine,
the vitamin is thiamine, riboflavin or nicotinamide,
the labeling substance is rhodamine B, uranine or Congo red.

18. The organic compound-encapsulating ferritin according to claim 16 or 17, wherein the organic compound is selected from the group consisting of the anthracycline substance, the histamine H2 receptor antagonist, the piguanide substance, the ATP-sensitive potassium channel opening agent and the β2 adrenergic receptor agonist.

19. The organic compound-encapsulating ferritin according to any one of claims 16 to 18, wherein ferritin is (1) naturally occurring ferritin or (2) ferritin including a genetically modified ferritin monomer with any of following modifications (a) to (c):
(a) a ferritin monomer with a functional peptide inserted into a flexible linker region between second and third α-helices counted from an N-terminus of the ferritin monomer comprising six α-helices ;
(b) a ferritin monomer with the functional peptide added to the N-terminus; or
(c) a ferritin monomer with the functional peptide added to the C-terminus.
